(19) Europäisches Patentamt
European Patent Office
Office européen des brevets

(11) EP 3 381 474 A1

(12) EUROPEAN PATENT APPLICATION

(43) Date of publication:
**03.10.2018 Bulletin 2018/40**

(51) Int Cl.:
***A61K 47/68*** *(2017.01)* ***A61P 35/00*** *(2006.01)*
***A61P 31/18*** *(2006.01)*

(21) Application number: **17163065.0**

(22) Date of filing: **27.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Alfasigma S.p.A.**
**40133 Bologna (IT)**

(72) Inventors:
- **VESCI, Loredana**
**00125 Roma (IT)**
- **DE SANTIS, Rita**
**00040 Pomezia (RM) (IT)**
- **MILAZZO, Ferdinando Maria**
**00139 Roma (IT)**
- **GIANNINI, Giuseppe**
**00071 Pomezia (RM) (IT)**
- **TADDEI, Maurizio**
**53035 Monteriggioni, Siena (IT)**
- **FALTONI, Valentina**
**52048 Monte San Savino (AR) (IT)**
- **PETRICCI, Elena**
**53100 Siena (IT)**

(74) Representative: **Hiebl, Inge Elisabeth**
**Kraus & Weisert**
**Patentanwälte PartGmbB**
**Thomas-Wimmer-Ring 15**
**80539 München (DE)**

# (54) HISTONE DEACETYLASE INHIBITORS-BASED ANTIBODY DRUG CONJUGATES (ADCS) AND USE IN THERAPY

(57) The present invention relates to novel Histone Deacetylase Inhibitors (HDACi)-based antibody drug conjugates with antibodies directed against a histone deacetylase inhibitor or against a receptor internalized by tumor cells to release a histone deacetylase inhibitor, particularly with antibodies directed to ErbB1, ErbB2 and ErbB3 receptors, pharmaceutical compositions comprising said antibodies as well as to their use in the treatment of cancer or tumor and other diseases where a modulation of one or more histone deacetylase isoforms can be effective for therapeutic interventions.

EP 3 381 474 A1

## Description

### Technical Field

The present invention is directed to novel Histone Deacetylase Inhibitors (HDACi)-based antibody drug conjugates (ADCs) useful for the treatment of proliferative diseases. The invention particularly relates to an antibody-drug-conjugate comprising an antibody directed to ErbB1, ErbB2, ErbB3 receptors or related molecular targets. The invention further provides new HDAC inhibitor drugs comprised by the antibody-drug-conjugates. Moreover, the invention relates to ADCs pharmaceutical compositions and their use in the treatment of cancer or tumors and other diseases where a modulation of one or more histone deacetylase isoforms can be effective for therapeutic interventions.

### Background of the Invention

Medical research is focused on personalized medicine for the treatment of cancer, neurological disorders, inflammatory diseases and viral infections. Today, the knowledge about the links between genetic variations and human diseases allows a greater understanding of their etiology.

Epigenetic aberrations can contribute to the onset and progression of the above mentioned human diseases via the gain or loss of function of epigenetic regulatory proteins *(*Berdasco, 2013 Hum Genet 132: 359-83), because over 1,750 proteins in human cells can be post-translationally modified at lysine residues via acetylation and deacetylation *(*Choudhary 2009 Science 325: 834-40*).* Deacetylating enzymes are considered as valuable targets to treat aberrant deacetylation related to cancer but also various other diseases such neurological disorders, inflammation, viral infections and cardiovascular disorders *(*Minucci 2006 Nature Rev Cancer 6: 38-51*;* Glozak 2007 Oncogene 26: 5420-32*;* Zhang 2015 Med Res Rev 35: 63-84*;* Dinarello 2010 Mol Med 17: 333-52*).*

To date, few HDACi have been approved by the FDA: vorinostat (Zolinza®; Merck) for the treatment of refractory cutaneous T-cell lymphoma (CTCL) *(*Duvic 2007 Blood 109: 31-39); romidepsin (Istodax®; Celgene) for the treatment of CTCL and peripheral T-cell lymphoma (PTCL) *(*VanderMolen 2011 J Antibiot (Tokyo) 64: 525-531*)* and belinostat (Beleodaq®; Spectrum Pharmaceuticals) for the treatment of PTCL *(*West 2014 J Clin Invest 124: 30-39*).* In early 2015 oral panobinostat (Farydak®, Novartis) has been approved by the FDA, as combination therapy with bortezomib and dexamethasone in patients with recurrent multiple myeloma *(*Garnock-Jones KP (2015) Drugs. 75: 695-704*).*

HDAC inhibitors (HDACi) are mostly studied as anticancer agents, but there is a growing body of literature ascribing HDAC enzymes to play a crucial role in other diseases such as neurological disorders, inflammatory processes and viral infections *(*Dinarello 2010 Cell 140: 935-950*;* Gray 2011 Epigenomics 3: 431-450*).*

Recently, within a new class of thiol-based potent pan-HDACi *(*Giannini 2014 J Med Chem 57: 8358-77) the present inventors focused on the potent HDAC inhibitor, ST7612AA1 for the construction of new ADCs to target a broad spectrum of human solid and haematologic malignancies.

ST7612AA1 exhibits the peculiarity to inhibit the growth of several tumors such as Ras-mutant colon carcinoma, a subset of strongly proliferating dedifferentiated colon cancer, associated with reduced patient survival; non small cell lung tumors with wild type EGFR (and mutant KRAS) and T790 EGFR mutation; ovarian with low levels of PTEN and overexpression of ErbB1 and ErbB2 or ovarian cancer without PTEN; triple-negative breast cancer (TNBC) defined by the absence of estrogen receptor, progesterone receptor and ErbB2; acute myeloid leukemia, diffuse large B cell lymphoma. Moreover, ST7612AA1 showed to modulate some transcripts involved in immune response and in key pathogenetic pathways, such NF-κB pathway and epithelial-mesenchymal transition (EMT), thus suggesting a relevant implication not only in cancer but also in the inflammatory diseases *(*Vesci 2015 OncoTarget 20: 5735-48*).*

The action of ST7612AA1 is exerted against both nuclear and cytoplasmatic HDAC isoforms of tumor cells, leading to increased transcription of e-cadherin, keratins and other typical epithelial markers and, concomitantly, down-regulation of vimentin and other genes associated to the mesenchymal phenotype. These data suggest that the treatment with ST7612AA1 might cause a "cadherin switch" and reversion of the epithelial-mesenchymal-transition (EMT) process favoring cell differentiation. The ability of cells to transdifferentiate and dedifferentiate plays a key role in invasion and metastasis by the EMT process and differentiation may be used as an additional prognostic and predictive indicator of therapeutic effectiveness. Through the inhibition of HDAC6, ST7612AA1 was also able to target non-histone HDAC substrates like, for example, TP53, *alpha*-tubulin or the heat shock protein 90 (HSP90) involved in DNA damage signaling, transcription factor binding, molecular homeostasis and DNA repair processes.

ST7612AA1 proved to be able to induce HIV reactivation being potentially useful for new therapies aiming at the eradication of the viral reservoirs *(*Badia 2015 Antiviral Res 123: 62-9*).*

To improve tumor specificity and reduce toxicity, recently antibody-drug conjugates have become a clinically validated cancer therapeutic modality. While considerable strides have been made in treating hematological tumors, challenges remain in the more difficult-to-treat solid cancers.

Antibody-drug conjugates (ADC) are a rapidly growing class of cancer drugs that combine the targeting properties of mAbs with the antitumor effects of potent cytotoxic drugs *(*Leal M 2014 Ann NY Acad Sci 1321: 41-54*).*

Currently, microtubule inhibitors are clinically validated ADC payloads. Both Kadcyla (Trastuzumab emtansine; Genentech) and Adcetris (brentuximab vedotin; Seattle Genetics) are FDA-approved ADC therapeutics, and more than 40 other ADCs have advanced to the clinic *(*Okeley 2014 Hematol Oncol Clin North Am 28: 13-25*;* Baron 2015 J Oncol Pharm Pract 21: 132-42*).*

The payloads currently utilized in ADCs are highly potent cytotoxic drugs, exerting their effects on critical cellular processes required for survival. Highly potent microtubule inhibitors, such as maytansine derivatives (DM1/DM4) or auristatins (MMAE/MMAF), dominate the current ADC landscape. These typically induce apoptosis in cells undergoing mitosis by causing cell cycle arrest at G2/M. More recent works show that microtubule inhibitors may also disrupt non-dividing cells in interphase. These findings provide explanation of how the microtubule inhibitors are also cytotoxic to slowly replicating or non-dividing tumor cells thus exhibiting significant toxicity. Other classes of cytotoxic drugs used in ADCs include enediynes (calicheamicin), duocarmycin derivatives, pyrrolobenzodiazepines (PBDs) and indolinobenzodiazepines, all of which target the minor groove of DNA, and quinoline alkaloids (SN-38), which inhibit topoisomerase I. Thus, the majority of payloads currently utilized in ADCs are highly potent, often cytotoxic in the picomolar range, which is thought to be a requirement for ADC strategy since only a very small amount (<1%) of the ADC injected dose localizes to the tumors (Bornstein 2015 AAPS Journal 17: 525-34*;* Casi and Neri 2015 J Med Chem 58: 8751-61*).*

The majority of ADC toxicity is thought to be derived from the payload release due to linker instability. Rapidly dividing normal cells such as cells lining the digestive tract, cells in the hair follicles and myeloid cells are at risk of toxicity from released microtubule inhibitors resulting in gastrointestinal symptoms, hair loss and myelosuppression. Some key toxicities are found with different payloads. In particular, MMAE induces peripheral neuropathy and neutropenia; MMAF is associated with thrombocytopenia and ocular toxicities; DM1 causes gastrointestinal effects as well as thrombocytopenia and neutropenia, depending on the linker and consequent metabolites; ocular toxicity is the most common adverse event with DM4-conjugated ADCs; calicheamicin causes thrombocytopenia and hepatic dysfunction; and early indications from SN-38 conjugated drugs suggests neutropenia as a frequent toxicity.

A possible strategy to minimize toxicity of next generation ADCs is the selection of low toxicity payloads.

The present invention surprisingly demonstrates that antibody-drug-conjugates made of an anti-tumor antibody, conjugated to an HDACi, a drug with low toxicity, can exert excellent efficacy in vivo. Moreover, the use of an HDACi for ADC construction, represents a first in class example in the field.

The antibody-drug-conjugate is further to have a good stability in blood and body fluids and excellent anti-cancer activity while having low toxicity compared to antibody-drug-conjugates of the prior art.

It is therefore an object of the present invention to provide an antibody-drug-conjugate comprising a safe anti-cancer drug conjugated to an antibody. Another object of the present invention is to provide a method for preparing said antibody-drug-conjugate.

A further object of the present invention is to provide a pharmaceutical composition comprising said antibody-drug-conjugate.

Yet another object of the present invention is to provide said antibody-drug-conjugate for use in a method of treating cancer or tumor and other diseases where a modulation of one or more histone deacetylase isoforms can be effective for therapeutic interventions.

In particular, other diseases like metabolic disorders, autism or inflammation-associated diseases such as lung injury, autoimmune disease, asthma, and type-2 diabetes that display aberrant gene expression and epigenetic regulation during their occurrence can be involved *(*Samanta 2017 Biochim Biophys Acta 1863: 518-28*;* Akhtar 2013 Plos One 8:e67813*;* Mei 2014 Neuron 83: 27-49). Epigenetic modifiers targeting DNA methylation and histone deacetylation enzymes could be a source to treat the pathogenesis of these diseases.

## Summary of the Invention

The present invention relates to an antibody-drug-conjugate of formula (I)

$$D\text{-}(CU)_m\text{-}(S1)_n\text{-}L\text{-}(S2)_o\text{-}(CG)_p\text{-}Ab \qquad \text{(Formula I)}$$

or a pharmaceutically acceptable salt thereof, wherein

D is a histone deacetylase inhibitor drug,

CU is a connecting unit,

S1 and S2 are spacers, which may be the same or different,

L is a linker, which can be cleavable or not cleavable,

CG is a connecting group,

Ab is an antibody or an antigen binding fragment thereof, and

m, n, o and p represent integers of 0 or 1.

In particular, the invention relates to antibody-drug-conjugates, wherein warhead antibody-drug conjugate (ADC) is selected from thiol-based histone deacetylase (HDAC) inhibitors such as ST7464AA1 and ST7660AA1, a thiol vorinostat analogue, having the following formulas:

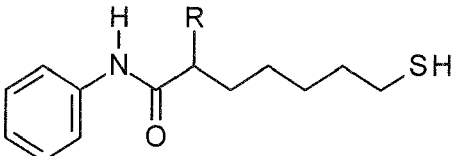

where R =

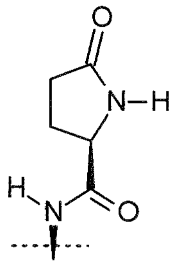

; H (tiol vorinostat analogue)

(ST7660AA1)

(ST7464AA1)

and the payload comprising D-$(CU)_m$-$(S1)_n$-L-$(S2)_o$-$(CG)_p$- is a compound selected from:

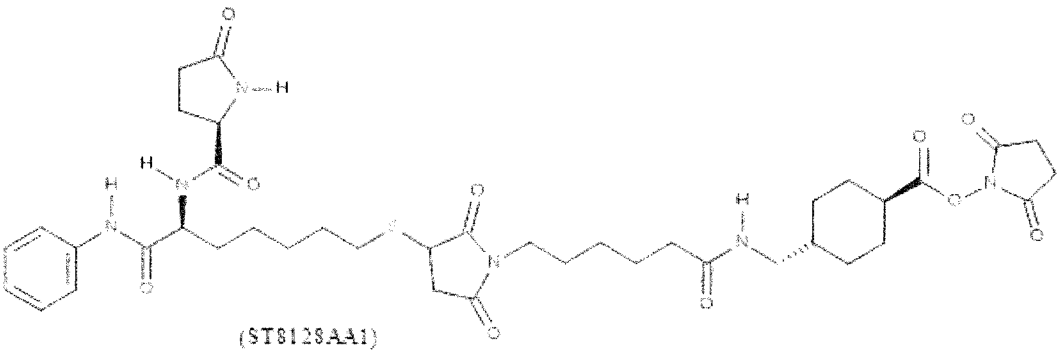

(ST8128AA1)

ST8128AA1

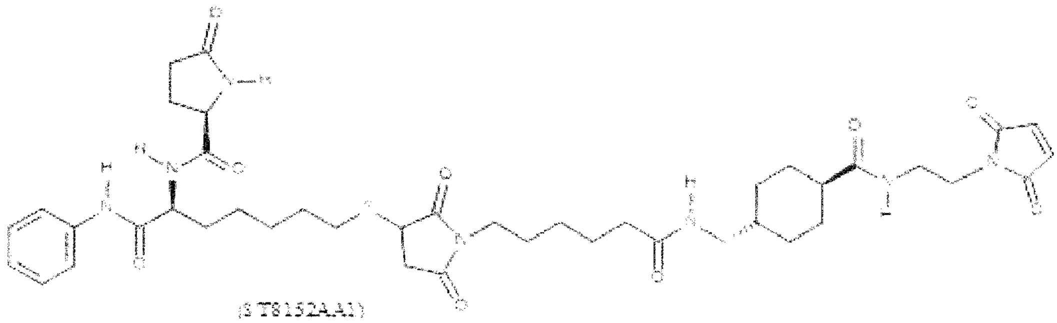

(ST8152AA1)

ST8152AA1

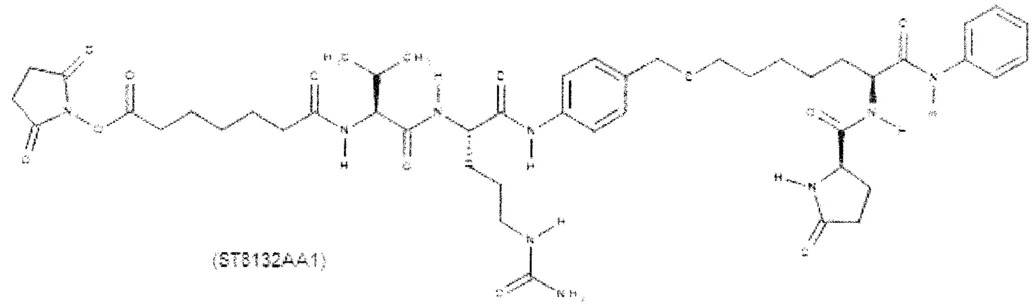

(ST8132AA1)

ST8132AA1

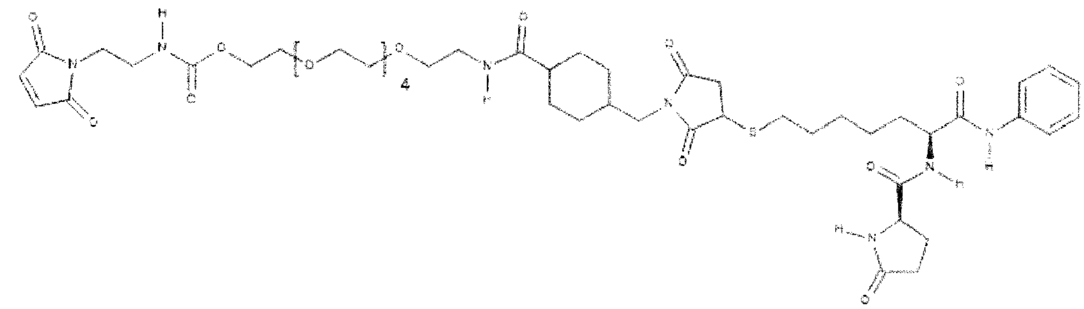

ST8190AA1

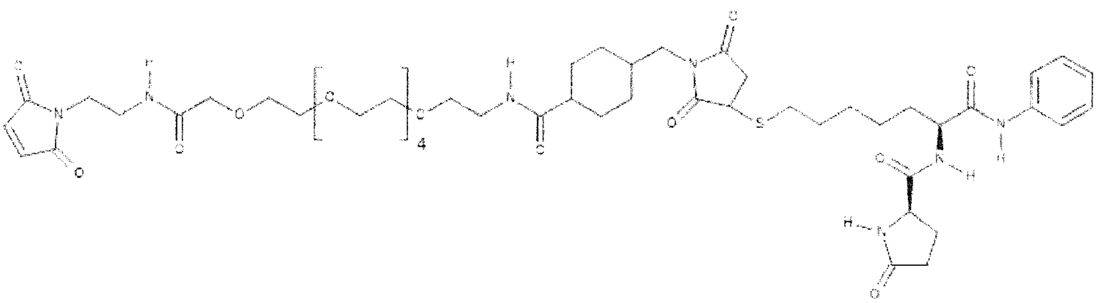

ST8189AA1

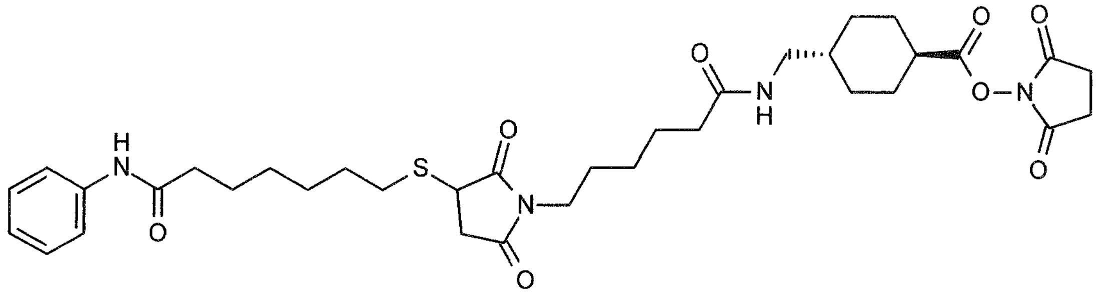

(ST8197AA)

ST8197AA1

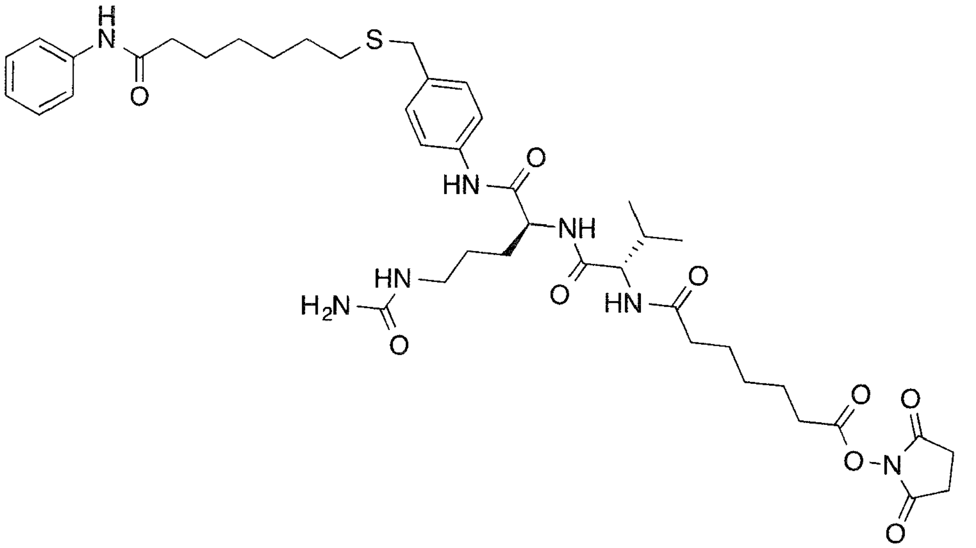

In a further embodiment, the invention relates to antibody-drug-conjugates, wherein warhead antibody-drug conjugate (ADC) is selected from hydroxamic acid-based histone deacetylase inhibitors (HDAC) such as Vorinostat (SAHA) and Panobinostat (LBH589), having the following formulas:

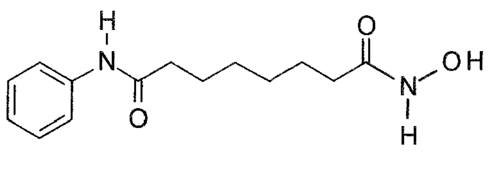

Vorinostat

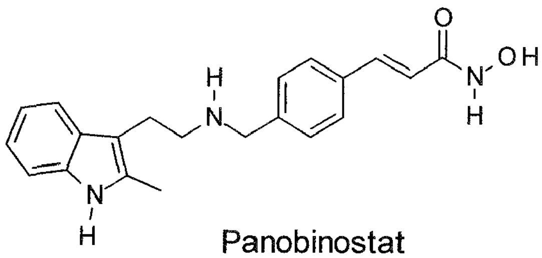

Panobinostat

and the payload comprising D-$(CU)_m$-$(S1)_n$-L-$(S2)_o$-$(CG)_p$- is a compound selected from:

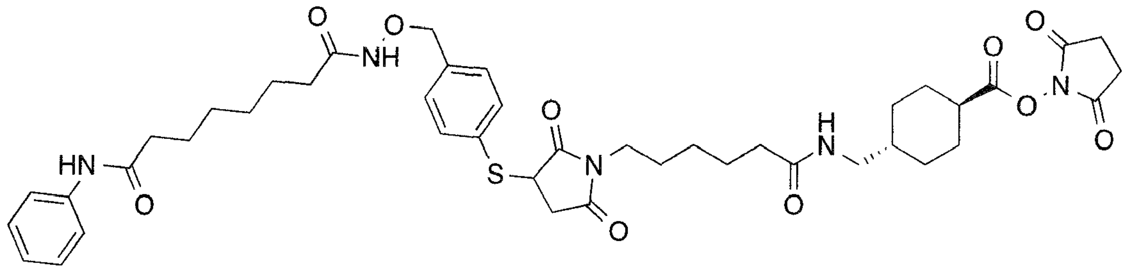

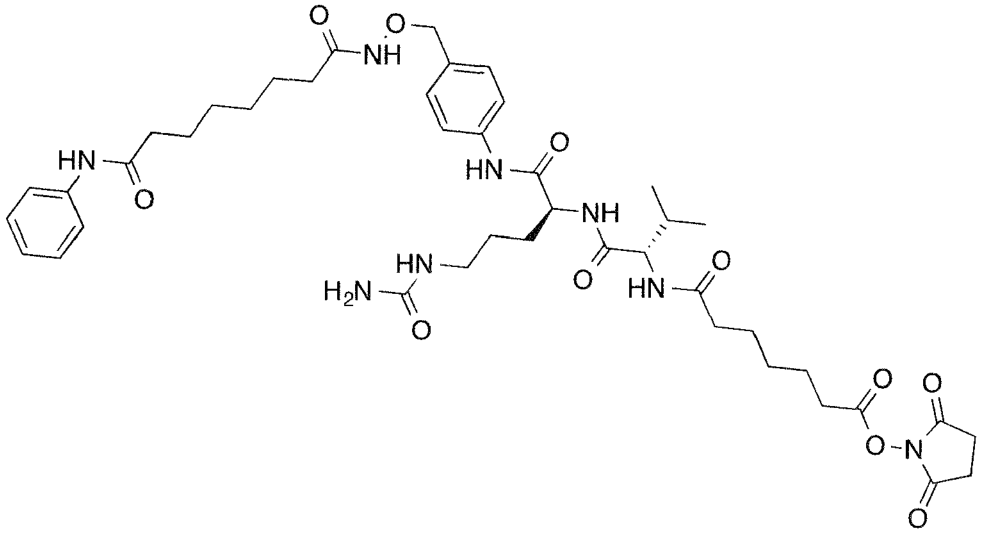

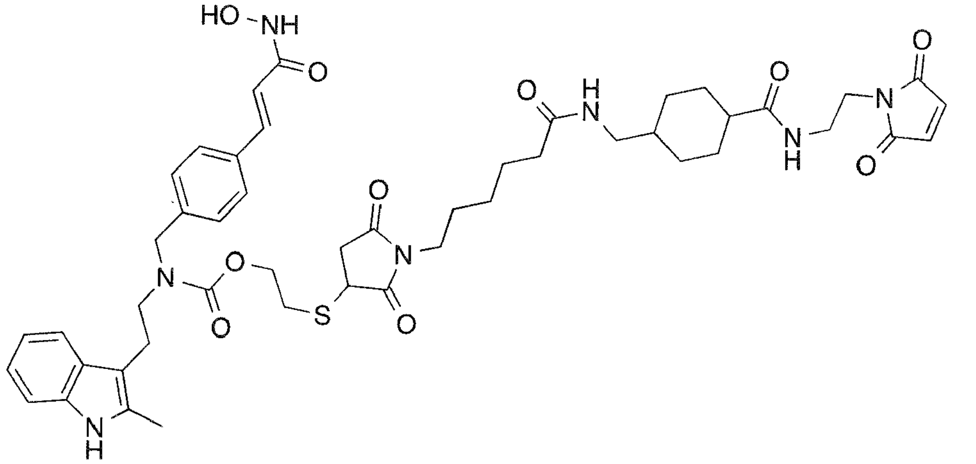

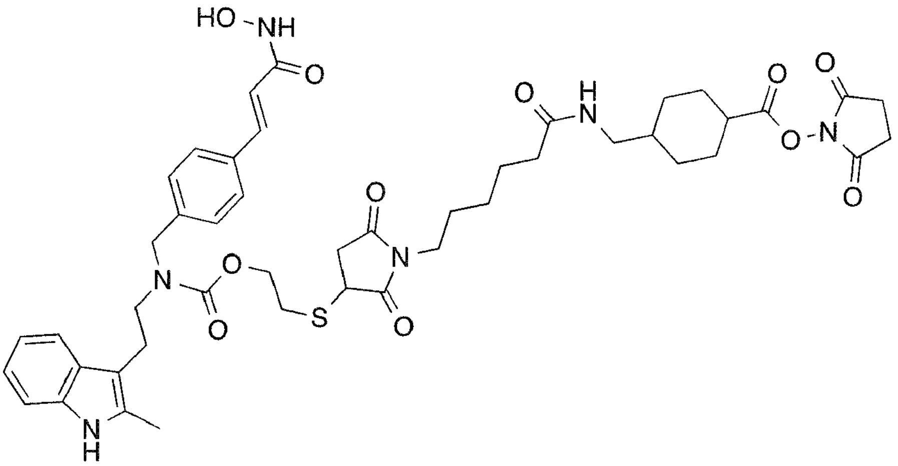

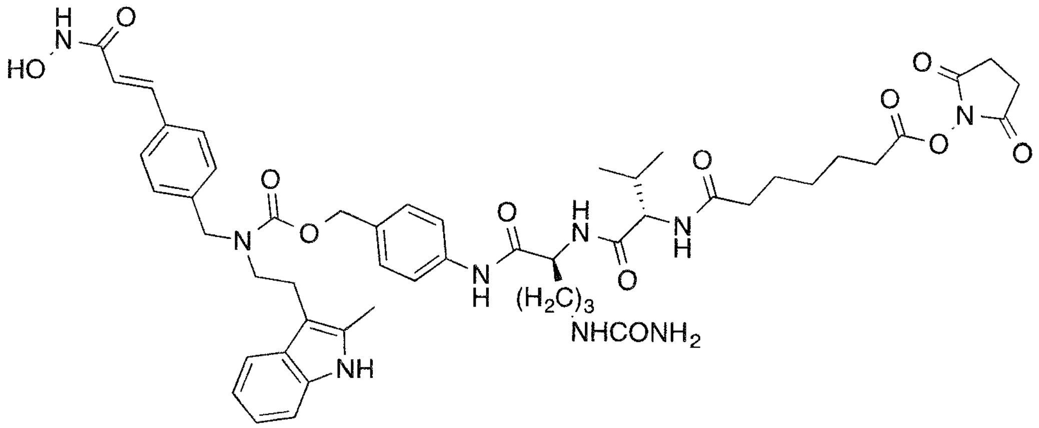

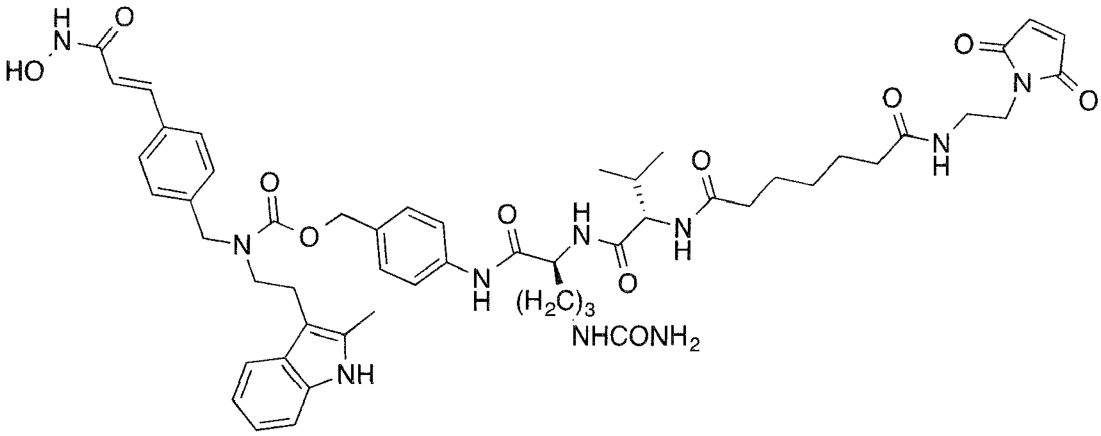

In a further embodiment, the invention relates to antibody-drug-conjugates, wherein warhead antibody-drug conjugate (ADC) is selected from Benzamide based histone deacetylase inhibitors (HDAC) such as Entinostat (MS275), having the following formula, wherein warhead is the cytotoxic agent of payload:

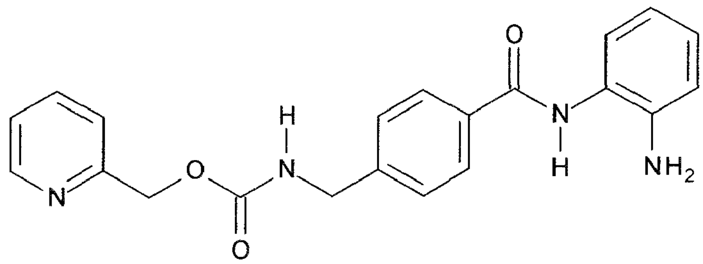

Entinostat

and the payload comprising D-$(CU)_m$-$(S1)_n$-L-$(S2)_o$-$(CG)_p$- is a compound selected from:

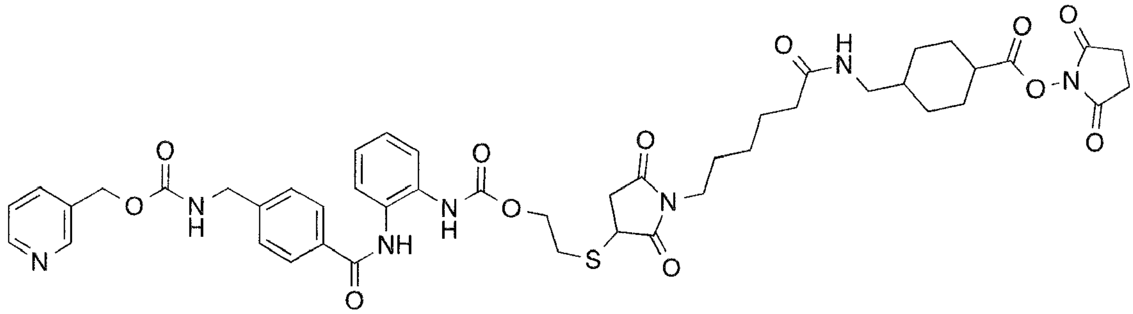

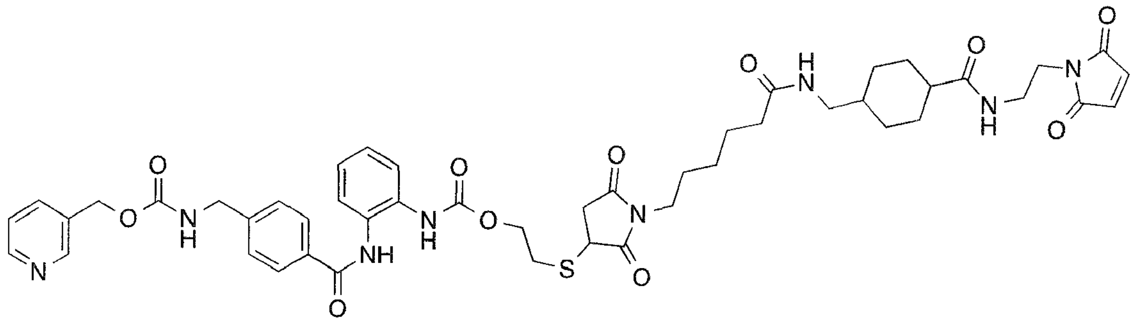

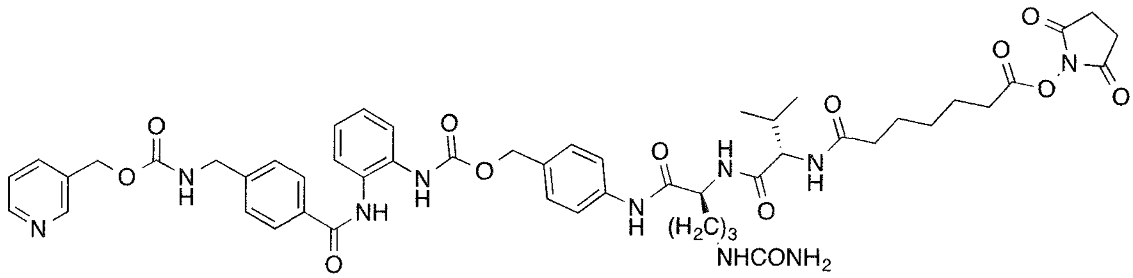

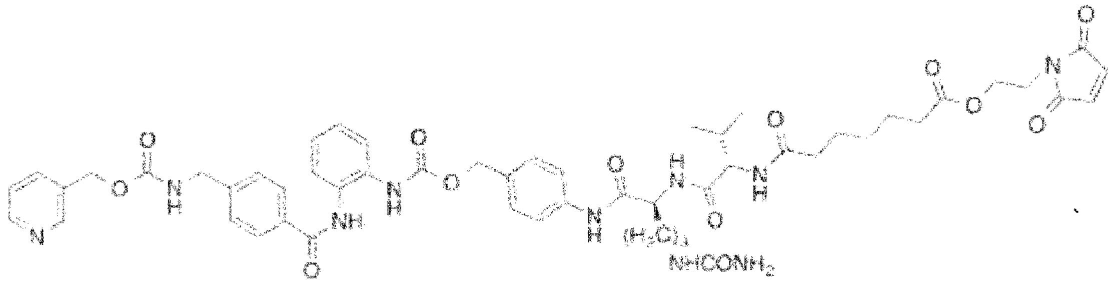

In a further embodiment of invention, the most preferred antibody-drug-conjugates are selected from:

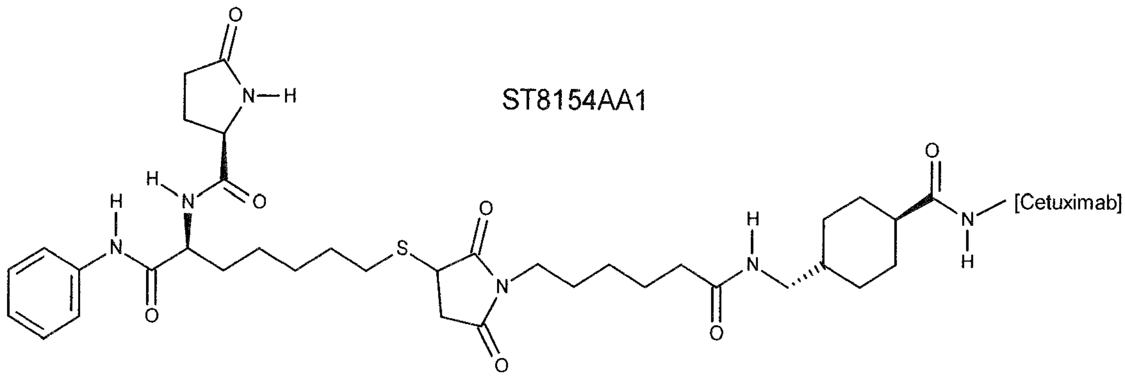

ST8154AA1

Formula (Ia)

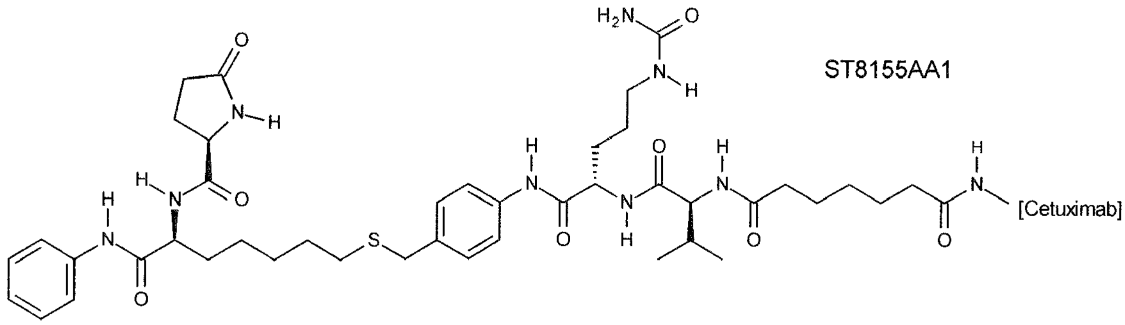

ST8155AA1

Formula (Ib)

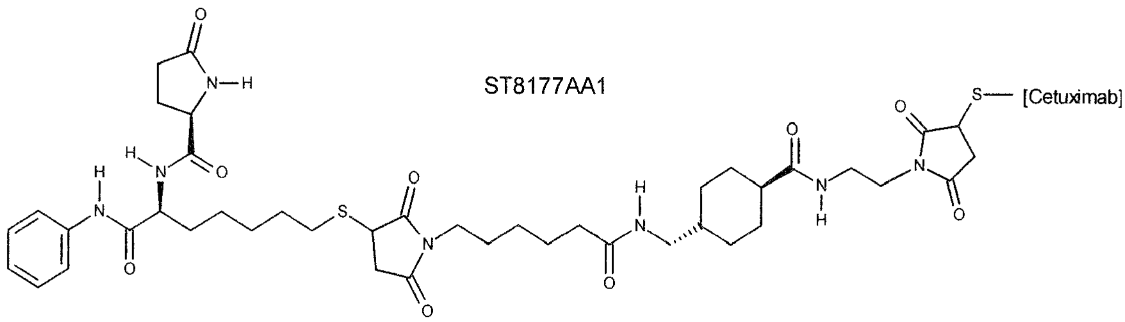

ST8177AA1

Formula (Ic)

ST8178AA1

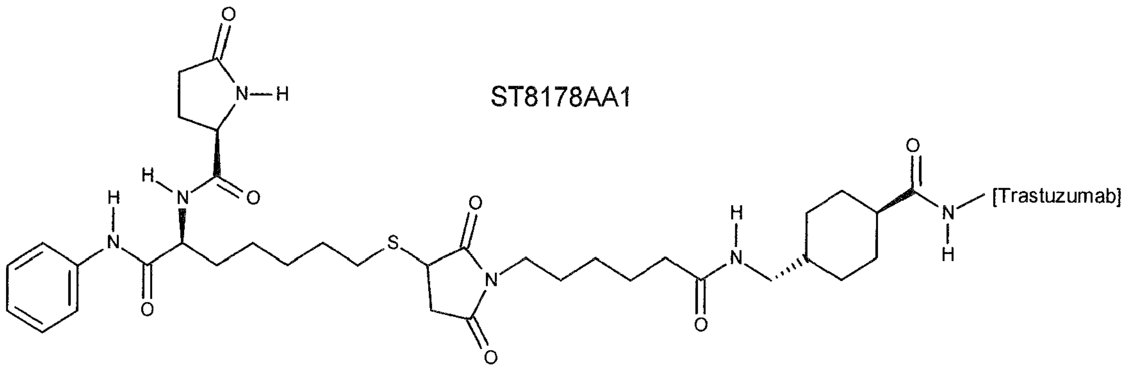

Formula (Id)

ST8178AA1

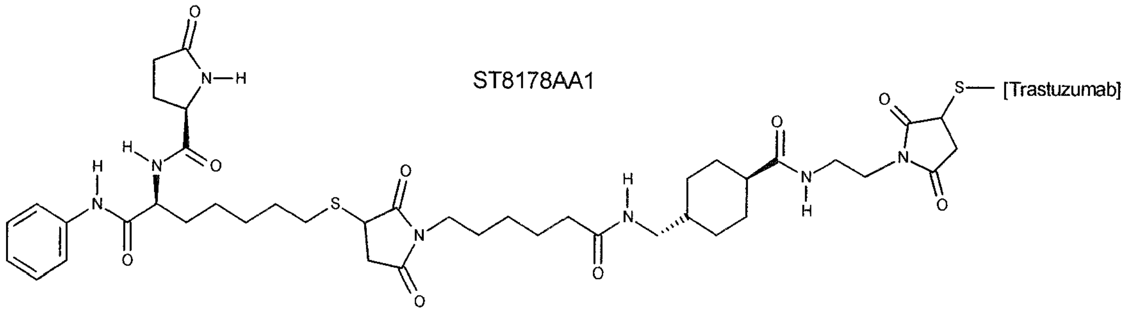

Formula (Ie)

ST8179AA1

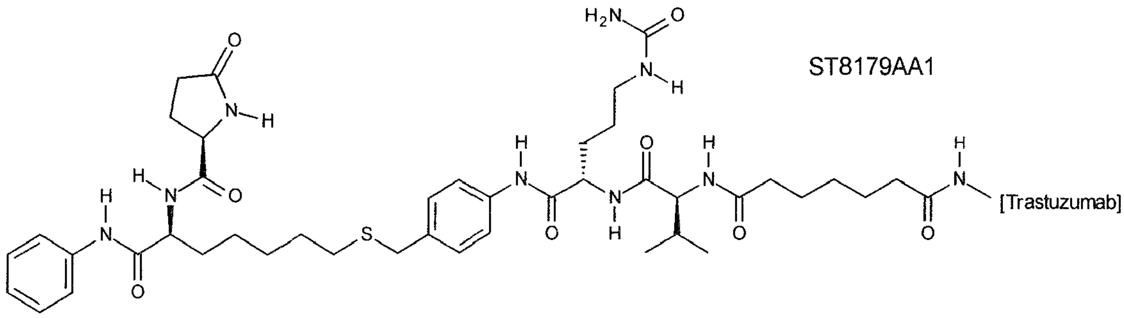

Formula (If).

Further embodiments of the invention are other ADC where mAb may be one selected from those indicated in the present invention:

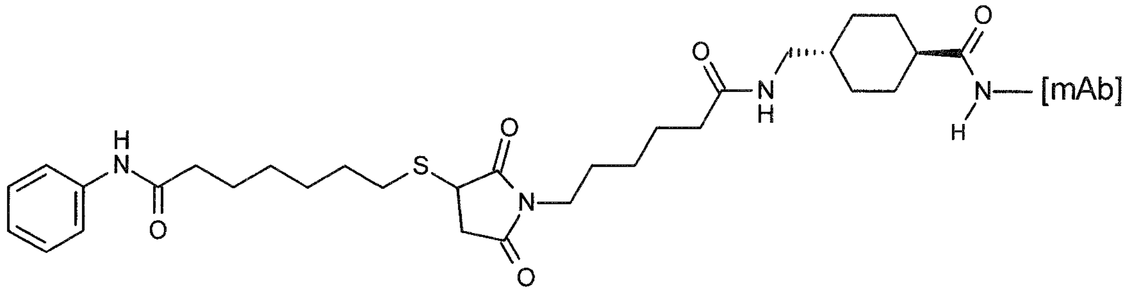

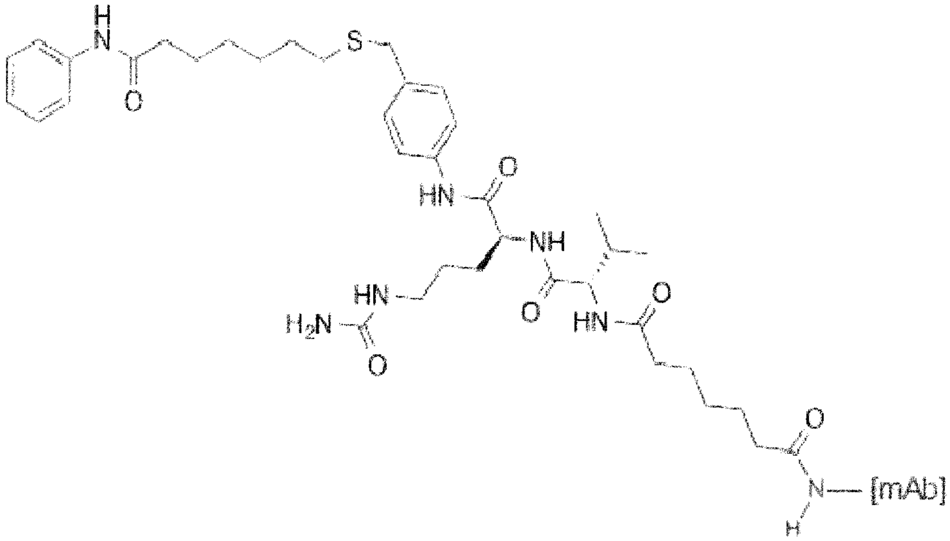

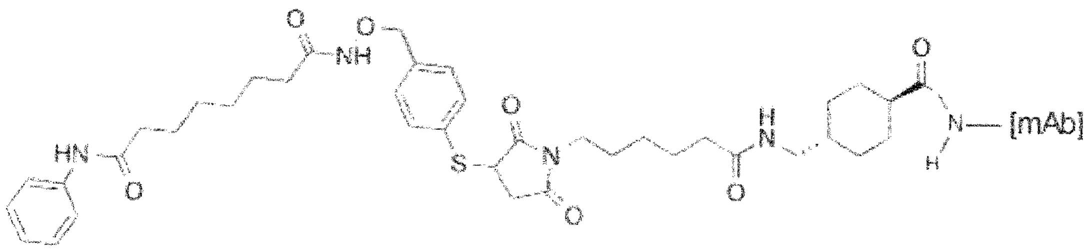

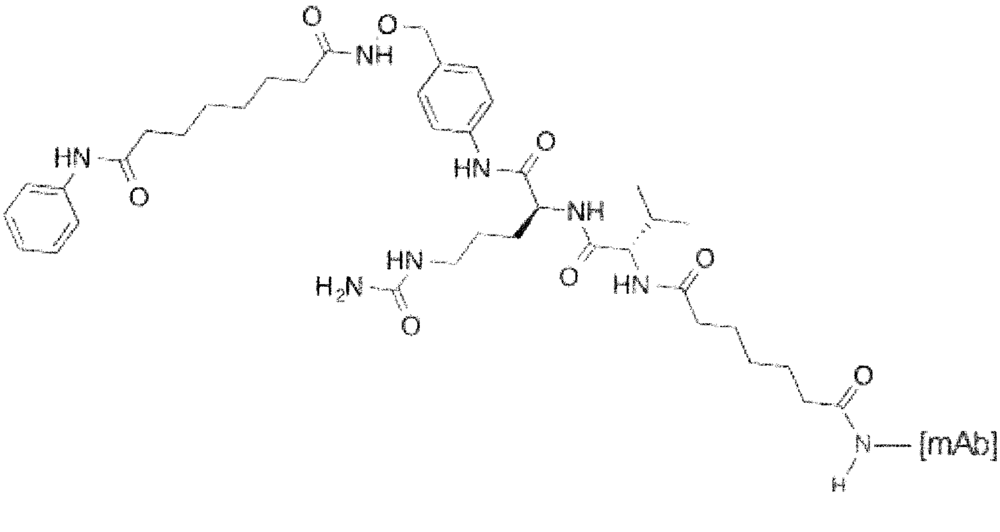

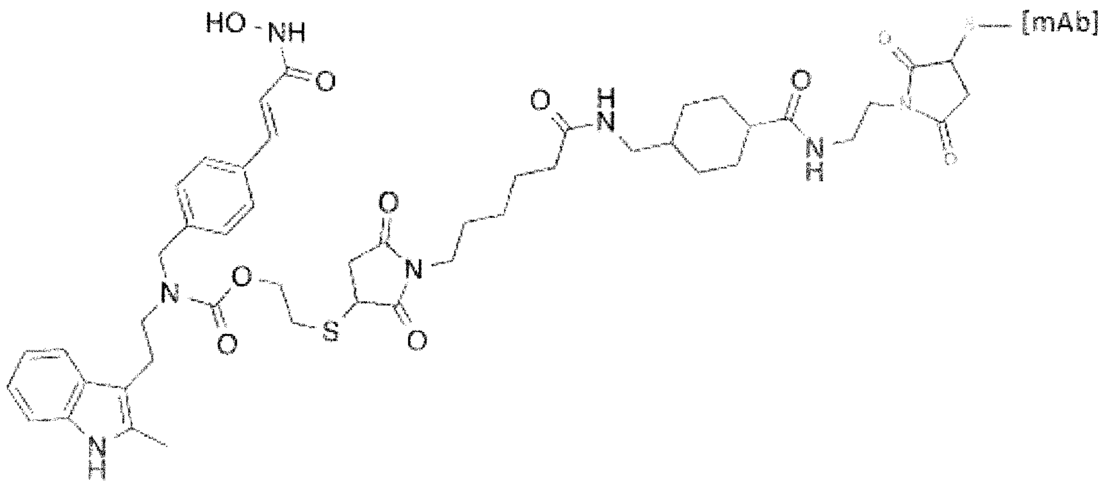

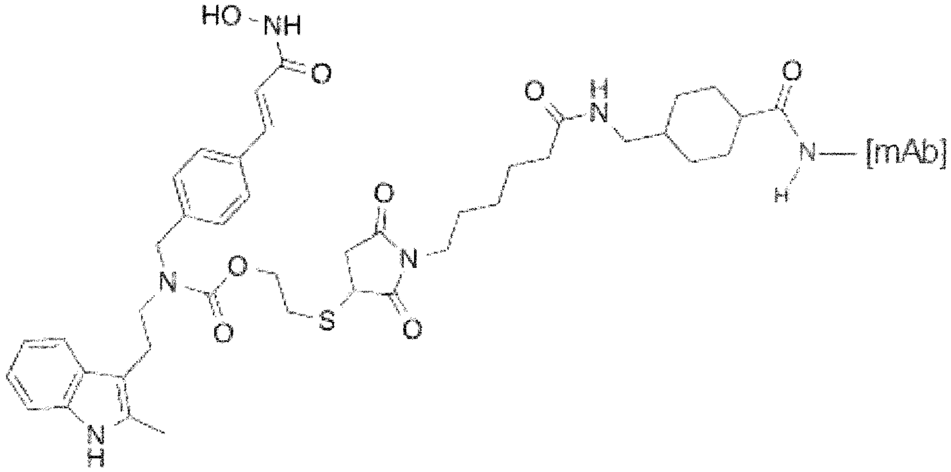

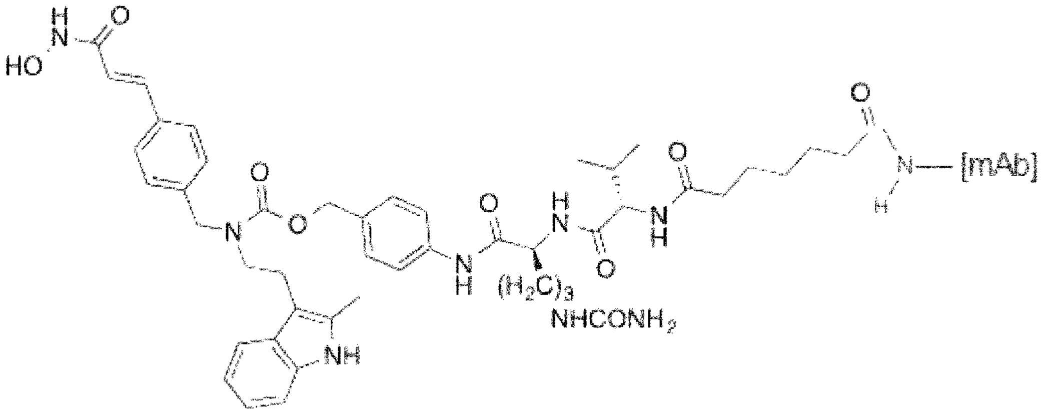

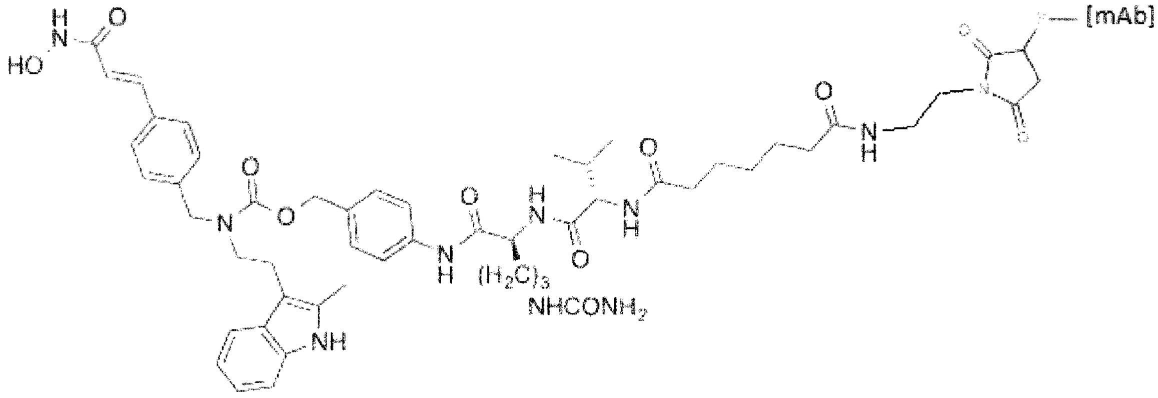

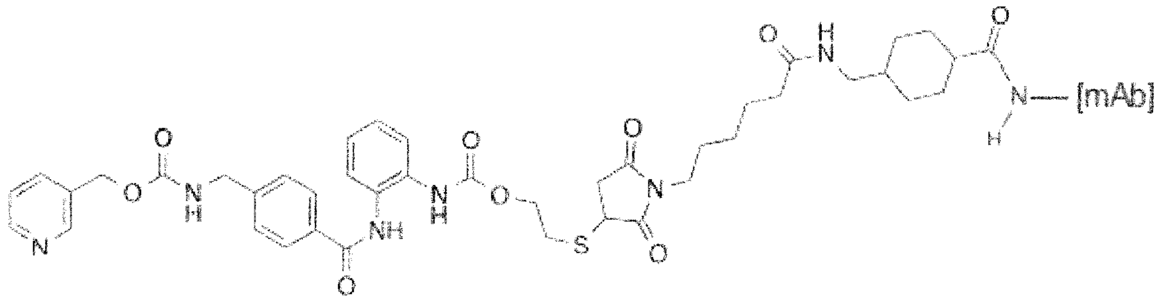

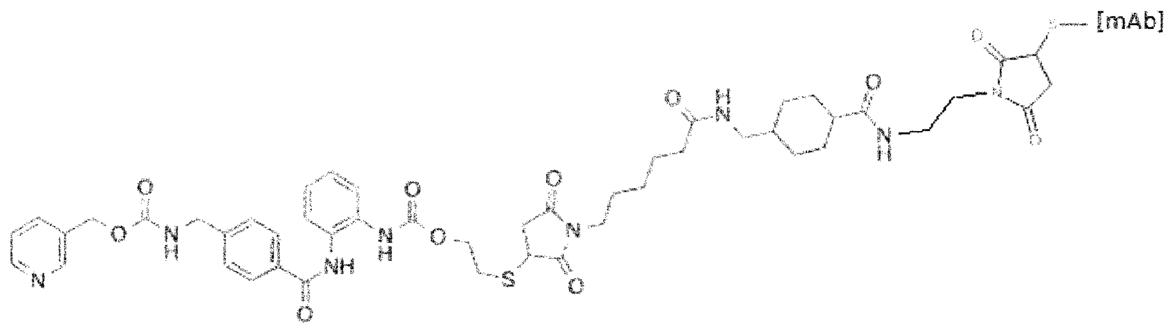

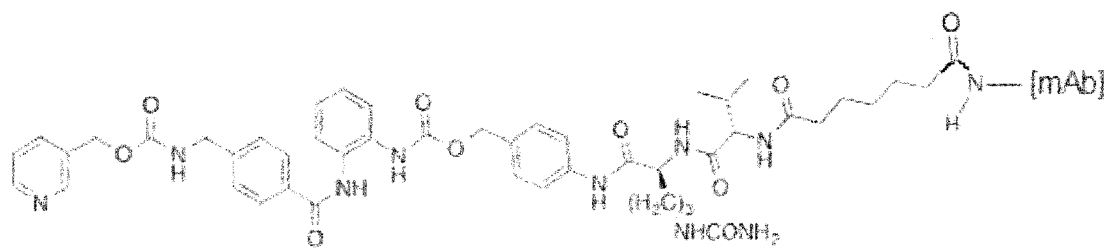

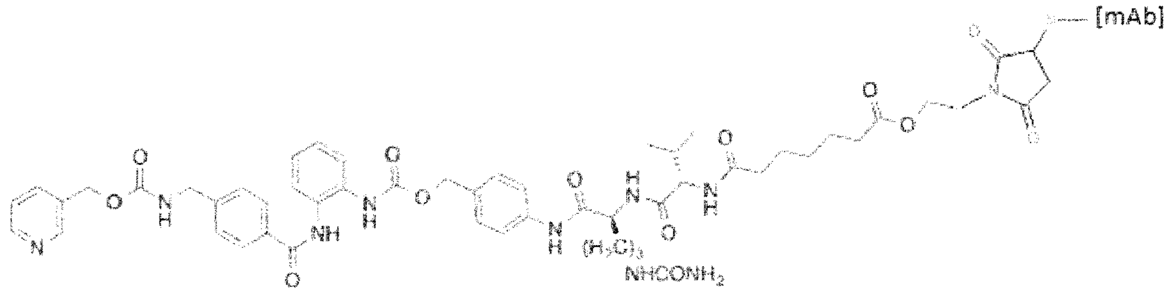

Moreover, the invention to relates to a pharmaceutical composition comprising said antibody-drug-conjugates

as well as to said antibody-drug-conjugates or said pharmaceutical composition for use in the treatment of a cancer or a tumor expressing a receptor selected from ErbB1, ErbB2 or ErbB3.

## Advantageous effects

The antibody-drug-conjugates of the present invention were more potent than the single antibodies and the cytotoxic agent, given at the same concentration, route and schedule. Moreover, it was surprising that the cytotoxic agent such as an HDAC inhibitor conjugated to the antibodies, at suboptimal dosages, resulted very effective, not depending on the linkers or type conjugation (lysine or cysteine).Subsequently, these HDAC inhibitors-based ADCs allow to obtain antitumor efficacy at lower dosages than the corresponding antibodies, thus resulting in minor toxicity.

## Brief description of the drawings

**Figure 1** shows MALDI mass spectra of Cetuximab (up) and of its conjugated forms (ST8154AA1) with Kad-HDAC-NHS (ST8128AA1) (down). The DAR calculated from the mass difference was 8.9.

**Figure 2** shows MALDI mass spectra of Trastuzumab (up) and of its conjugated forms (ST8178AA1) with Kad-HDAC-NHS (ST8128AA1) (down). The DAR calculated from the mass difference was 6.9.

**Figure 3** shows MALDI mass spectra of Fab Erb3 (down) and of its conjugated forms with Kad-HDAC-NHS (ST8128AA1) (up). The calculated DAR was 2.5.

**Figure 3a** shows MALDI mass spectra of Vectibix (up) and of its conjugated forms with Kad-HDAC-NHS (down). The DAR calculated from the mass difference was 12.

**Figure 4** shows HIC chromatograms of Cetuximab (down) and of its conjugated forms (ST8177AA1) with Kad-HDAC-maleimide (ST8152AA1) (up).

**Figure 5** shows HIC chromatograms of Trastuzumab (up) and of its conjugated forms (ST8178AA1) with Kad-HDAC- maleimide (ST8152AA1) (down).

**Figure 6** shows binding (FACS analysis) of native Cetuximab (grey line) and cetuximab-derived ADCs, Cet(Lys)-Kad-HDAC (ST8154AA1) and Cet(Cys)-Kad-HDAC (ST8177AA1) (black line) to different tumor cell lines. Antibody binding detected by FITC-conjugated mouse anti-human Ig (BD). Grey peaks refer to cells without primary antibody.

**Figure 7** shows binding (FACS analysis) of native Trastuzumab (grey lines) and trastuzumab-derived ADCs, Tras(Lys)-Kad-HDAC (ST8178AA1) and Tras(Cys)-Kad-HDAC (ST8176AA1) (black lines) to different tumor cell lines. Antibody binding detected by FITC-conjugated mouse anti-human Ig (BD). Grey peaks refer to cells without primary antibody.

**Figure 8** shows internalization of native Cetuximab (left boxes), and cetuximab-derived ADCs (central and right boxes), Cet(Lys)-Kad-HDAC (ST8154AA1) and Cet(Cys)-Kad-HDAC (ST8177AA1), by different tumor cell lines. Cells were cultivated 3 hours at 37°C with antibodies (5 μg/mL). Following two washings, cells were then fixed and stained with FITC-conjugated mouse anti-human IgG. Draq5 dye staining of nucleus and cytoplasm. Insects show fluorescence signals specifically associated to cetuximab or ADCs within the cells. Fluorescence imaging by High Content Screening (HCS) Operetta. Each image is representative of at least 5 fields of duplicate wells. Magnification 60x. Data are from one representative experiment out of two.

**Figure 9** shows internalization of native Trastuzumab (left boxes), and trastuzumab-derived ADCs (central and right boxes), Tras(Lys)-Kad-HDAC (ST8178AA1) and Tras(Cys)-Kad-HDAC (ST8176AA1), by different tumor cell lines. Cells were cultivated 3 hours at 37°C with antibodies (5 μg/mL). Following two washings, cells were then fixed and stained with FITC-conjugated mouse anti-human IgG. Draq5 dye staining of nucleus and cytoplasm. Insects show fluorescence signals specifically associated to Trastuzumab or ADCs within the cells. Fluorescence imaging by High Content Screening (HCS) Operetta. Each image is representative of at least 5 fields of duplicate wells. Magnification 60x. Data are from one representative experiment out of two.

**Figure 10** shows immunoreactivity of ADCs, tested by antigen-specific ELISA. Activity measured against A) recombinant human EGF-R/Erb1 Fc chimera, or B) recombinant Human ErbB2/HER2 protein. Detection through anti-human K light chain horseradish peroxidase (HRP)-conjugated antibody and TMB substrate addition. Optical density at 450 nm measured by ELISA spectrophotometer. Results are the mean (±SD) of two independent replicates.

**Figure 11** shows antiproliferative activity of ST8154AA1 on NCI-H1975 non-small cell lung carcinoma cells upon 6 days of treatment. IC50 value±SD of the ADC was 250±10 nM, in comparison with Cetuximab, which was ineffective (IC50 >500 nM).

**Figure 12** shows antiproliferative activity of ST8154AA1 on Calu-3 non-small cell lung carcinoma cells upon 6 days of treatment. IC50 value ±SD of the ADC was 450±10 nM, in comparison with Cetuximab, which was ineffective (IC50 >500 nM).

**Figure 13** shows the effect of Cetuximab and cetuximab-derived (ST8154AA1 and ST8177AA1) ADCs on the level of acetylated-α-tubulin in different tumor cell lines. Cells were cultivated 3 hours at 37°C with antibodies (5 μg/mL). Following two washings, cells were then fixed and stained with mouse anti-acetylated-α-tubulin IgG and then with FITC-conjugated goat anti-mouse IgG. Draq5 dye staining of nucleus and cytoplasm. Insects show fluorescence signals specifically associated to acetylated-α-tubulin. Fluorescence imaging by High Content Screening (HCS) Operetta. Each image is representative of at least 5 fields of duplicate wells. Magnification 60x. Data are from one representative experiment out of two.

**Figure 14** shows the effect of Trastuzumab and trastuzumab-derived (ST8178AA1 and ST8176AA1) ADCs on the level of acetylated-α-tubulin in different tumor cell lines. Cells were cultivated 3 hours at 37°C with antibodies (5 μg/mL). Following two washings, cells were then fixed and stained with mouse anti-acetylated-α-tubulin IgG and then with FITC-conjugated goat anti-mouse IgG. Draq5 dye staining of nucleus and cytoplasm. Insects show fluorescence signals specifically associated to acetylated-α-tubulin. Fluorescence imaging by High Content Screening (HCS) Operetta. Each image is representative of at least 5 fields of duplicate wells. Magnification 60x. Data are from one representative experiment out of two.

**Figure 15** shows the effect of Cetuximab and cetuximab-derived (ST8154AA1 and ST8177AA1) ADCs on the level of acetylated-α-histone H3 in different tumor cell lines. Cells were cultivated 3 hours at 37°C with antibodies (5 μg/mL). Following two washings, cells were then fixed and stained with rabbit anti-acetylated-α-histone H3 IgG and then with FITC-conjugated goat anti-rabbit IgG. Draq5 dye staining of nucleus and cytoplasm. Insects show fluorescence signals specifically associated to acetylated-α-histone H3. Fluorescence imaging by High Content Screening (HCS) Operetta. Each image is representative of at least 5 fields of duplicate wells. Magnification 60x. Data are from one representative experiment out of two.

**Figure 16** shows the effect of Trastuzumab and trastuzumab-derived (ST8178AA1 and ST8176AA1) ADCs on the level of acetylated-α-histone H3 in different tumor cell lines. Cells were cultivated 3 hours at 37°C with antibodies (5 μg/mL). Following two washings, cells were then fixed and stained with rabbit anti-acetylated-α-histone H3 IgG and then with FITC-conjugated goat anti-rabbit IgG. Draq5 dye staining of nucleus and cytoplasm. Insects show fluorescence signals specifically associated to acetylated-α-histone H3. Fluorescence imaging by High Content Screening (HCS) Operetta. Each image is representative of at least 5 fields of duplicate wells. Magnification 60x. Data are from one representative experiment out of two.

**Figure 17** shows the effect of ADCs on acetylation of α-tubulin and histone H4 in A549 (A) and SKBR3 (B) cell lines. Cells were cultivated 3 hours at 37°C with antibodies (20 μg/mL) and then Western Blot analysis was carried out on total protein lysates. Representative blots are shown.

**Figure 18** shows antitumor activity of ST8155AA1 given intraperitoneally according to the schedule q4dx4, in comparison with Cetuximab in NCI-H1975 tumor bearing mice.

**Figure 19** shows antitumor activity of ST8154AA1 given intraperitoneally according to the schedule q4dx4, in comparison with Cetuximab in NCI-H1975 tumor bearing mice.

**Figure 20** shows antitumor activity of ST8154AA1 given intraperitoneally according to the schedule q4dx4, in comparison with Cetuximab in A549 tumor bearing mice.

**Figure 21** shows antimetastatic activity of ST8154AA1 given by aerosol in comparison with Cetuximab in A549-luc tumor bearing mice.

**Figure 22** shows antitumor activity of ST8178AA1 given intraperitoneally according to the schedule q4dx4, in comparison with Trastuzumab in SKOV-3 tumor bearing mice.

**Figure 23** shows antitumor activity of ST8176AA1 given intraperitoneally according to the schedule q4dx4, in comparison with Trastuzumab in SKOV-3 tumor bearing mice.

**Figure 24** shows antitumor activity of ST8176AA1 given intraperitoneally according to the schedule q4dx4, in comparison with Trastuzumab in LS-174T ip tumor bearing mice.

## Detailed description of the invention

Surprisingly the present inventors found that a safe HDACi can be conjugated with a linker to an antibody and in particular an immunoglobulin resulting in an effective ADC anti-cancer therapeutic. This is the first time that an epigenetic modulator such as an HDAC inhibitor is used for ADC construction instead of potent cytotoxic agents.

The immunoglobulin vectors herein described are directed against receptors of the tyrosine kinase (RTK) family. This is a superfamily of transmembrane proteins that mediate intracellular signaling by phosphorylating substrate proteins involved in cell proliferation, survival, differentiation or migration.

In particular, the Human Epidermal growth factor Receptor (HER) family belongs to the RTKs superfamily, and comprises four members: ErbB1/EGFR (epidermal growth factor receptor), ErbB2, ErbB3 and ErbB4. Physiologically, these receptors are activated by the ligands of the EGF family.

EGFR plays a causal role in the development and maintenance of many human carcinomas, with mutation and overexpression observed in a number of tumor types *(*Burgess AW 2008 Growth Factors 26: 263-74*)*.

EGFR has become a clinically validated target for antibodies as well as for tyrosine kinase inhibitors having gained widespread use in lung, head and neck, colon, and pancreatic cancers *(*Mendelsohn J 2006 Semin Oncol 33: 369-85; Feiner 2016 Exp Rev Proteomics, Sep 13: 817-32; Enrique AA 2012 Front Biosci 4: 12-22*;* Landi L 2014 Expert Opin Pharmacol Ther 15: 2293-305*)*.

Despite the success of these inhibitors, significant numbers of patients with EGFR-positive tumors fail to respond to current EGFR-targeting therapeutics as a range of mutations (e.g., EGFR, KRAS, BRAF, PI3K, and PTEN) may contribute to intrinsic or acquired resistance *(*Chong CR 2013 Nat Med 19: 1389-400*)*.

A microtubule inhibitor-based ADC targeting EGFR is a questionable therapeutic strategy in that it may improve the activity of anti-EGFR antibodies by circumventing resistance mediated by downstream signaling mutations, but, because of the known toxicity of these antibodies (i.e. skin rash, diarrhea, constipation, stomatitis, fatigue, and electrolyte disturbances) *(*Li T 2009 Target Oncol 4: 107-19*)* it might have limited applicability. It was surprisingly found that ADCs made of anti-EGFR family protein antibodies conjugated to low toxicity HDACi are effective anti-cancer agents.

The invention relates to novel ADCs made of anti-cancer antibodies conjugated to HDACi-based payloads. Such ADCs are shown to specifically bind tumor receptors, to be internalized and delivered to lysosomes. These properties surprisingly correlate with *in vitro* cytotoxicity and *in vivo* antitumor activities despite the low potency of the HDAC payloads. The antibody-drug-conjugates of the invention are particularly useful in the treatment of tumors or any other diseases where a modulation of one or more histone deacetylase isoforms and/or the expression of ErbB receptors are effective for therapeutic intervention.

For example, HDACs 1, 2 and 3 primarily nuclear have been found expecially in late stage, aggressive malignacies in tumor cells and they have correlated with poor survival rates *(*Gryder 2012 Future Med Chem 4: 505-24*);* HDAC6 as its primary cellular localization in the cytoplasm, regulates acetylation states and thereby the functionality of tubulin, HSP90 and other extranuclear proteins, thus suggesting its involment in removal of misfolded proteins in cells, cell motility and metastatic potential *(*Clawson 2016 Ann Transl Med 4: 287*)*.

HDAC isoforms (2, 3, 6, 9, 10) are also involved in chronic intestinal inflammation, so HDAC inhibitors in addition to apoptosis induction of tumor cells can be used for inflammatory bowel disease *(*Felice 2015 Aliment Pharmacol Ther 41: 26-38).

Because epigenetic aberrations are implicated to the onset and progression of human diseases such as cancer, inflammatory diseases, viral infections and neurological disorders, via the loss or gain of function of epigenetic regulatory proteins *(*Berdasco 2013 Hum Genet 132: 359-83), clinical developments of histone deacetylase inhibitors regard all the human diseases linked to abnormal expression and/or function of HDACs.

A preferred embodiment of the present invention is the use of HDACi-based ADCs for the therapy of cancer expressing receptors such as ErbB1, ErbB2 or ErbB3 including as example, lung, breast, colon, brain, head and neck, endometrial, renal, pancreatic, gastric, oesophageal, ovarian and prostate cancer and leukaemia.

Growth factors mediate their diverse biologic responses (regulation of cellular proliferation, differentiation, migration and survival) by binding to cell-surface receptor tyrosine kinases (RTK) and the ErbB receptor family of RTKs are often overexpressed, amplified, or mutated in many forms of cancer, making them important therapeutic targets. In particular, EGFR has been found to be amplified in gliomas and non-small-cell lung carcinoma while ErbB2 overexpression has been seen in breast, ovarian, bladder, non-small-cell lung carcinoma, as well as several other tumor types *(*Collin 2015 Front Pharmacol 6: 283).

The present invention relates to an antibody-drug-conjugate of formula (I)

$$D\text{-}(CU)_m\text{-}(S1)_n\text{-}L\text{-}(S2)_o\text{-}(CG)_p\text{-}Ab \qquad \text{(Formula I)}$$

or a pharmaceutically acceptable salt thereof, wherein

D is a histone deacetylase inhibitor drug,
CU is a connecting unit,
S1 and S2 are spacers, which may be the same or different,
L is a linker, which can be cleavable or not cleavable,
CG is a connecting group,
Ab is an antibody or an antigen binding fragment thereof, and
m, n, o and p represent integers of 0 or 1.

The histone deacetylase inhibitor used in the payload may be any histone deacetylase inhibitor known in the art. Preferably, the histone deacetylase inhibitor is an oral thiol-based histone deacetylase inhibitor.

Preferably, the histone deacetylase inhibitor is ST7464AA1, drug of corresponding prodrug ST7612AA1

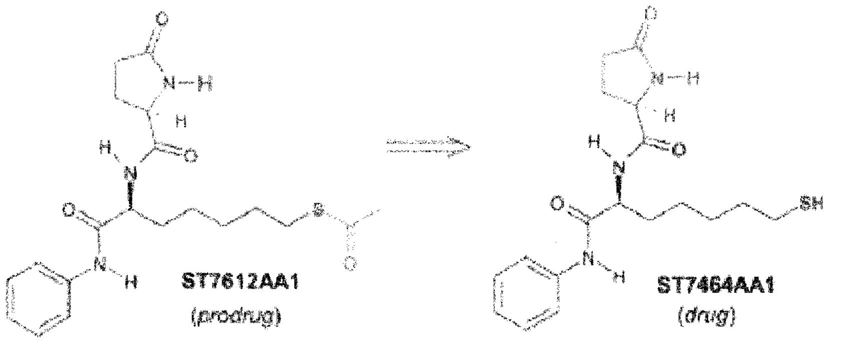
ST7612AA1 (*prodrug*)
ST7464AA1 (*drug*)

ST7464AA1

and the payload comprising $D\text{-}(CU)_m\text{-}(S1)_n\text{-}L\text{-}(S2)_o\text{-}(CG)_p\text{-}$ is a compound selected from:

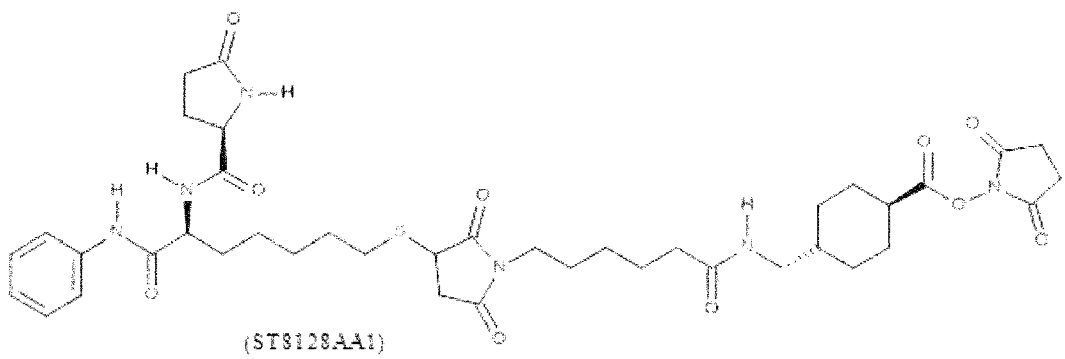
(ST8128AA1)

ST8128AA1

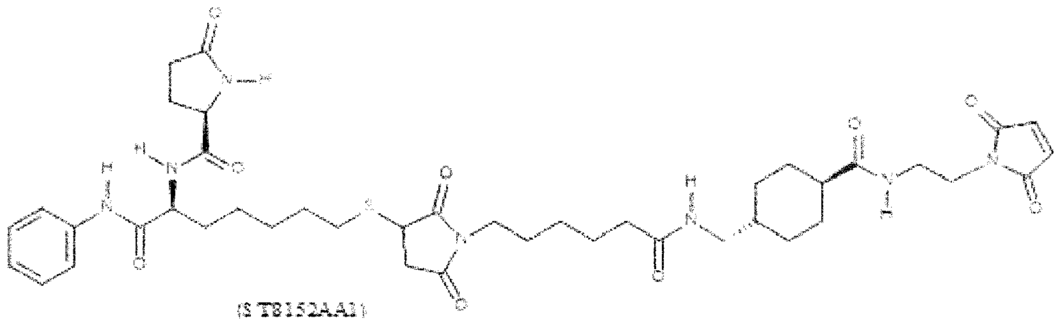

(ST8152AA1)

ST8152AA1

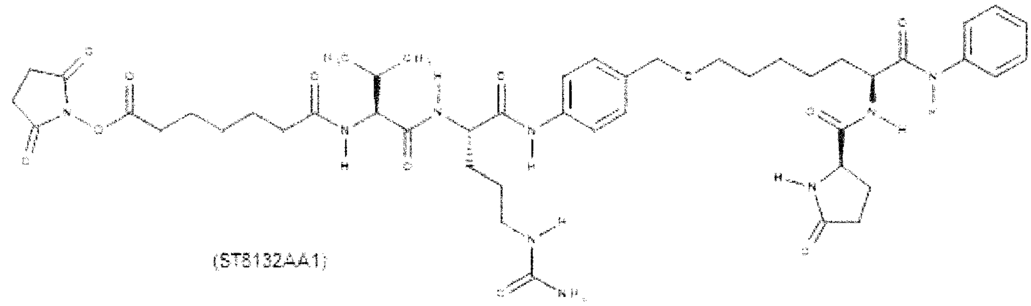

(ST8132AA1)

ST8132AA1

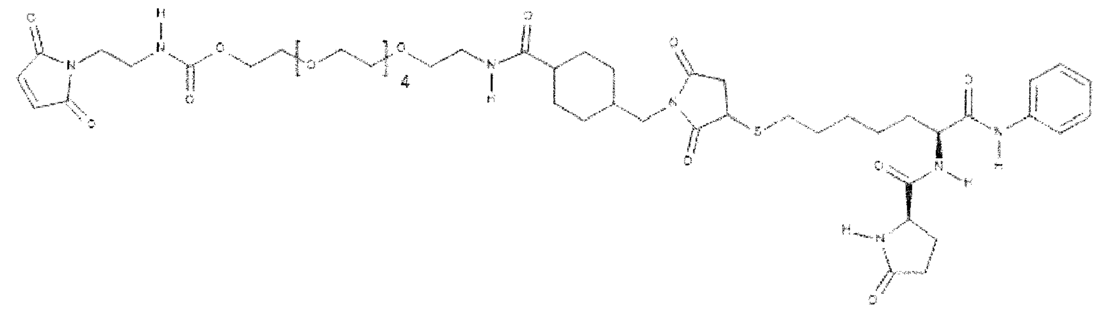

or

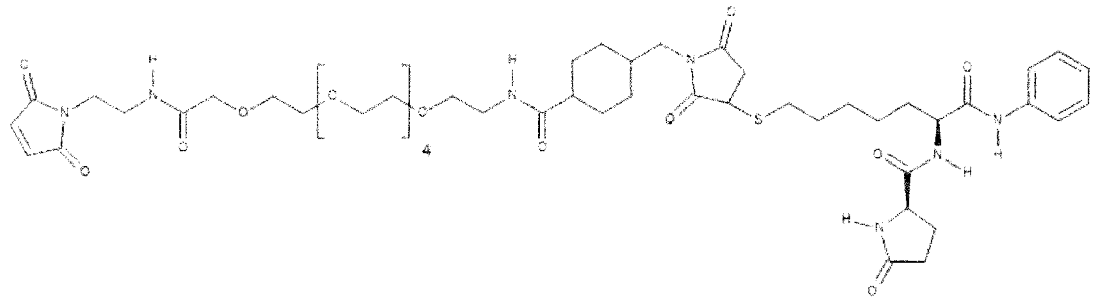

.

In a particular preferred embodiment, the histone deacetylase inhibitor is the compound ST7612AA1 being a prodrug of the compound ST7464AA1 as shown above.

Further examples of payloads useful in the present invention are the compounds of the following Table 1. The compounds ST8128AA1, and ST8132AA1 ST8190AA1 and ST8189AA1 are embodiments of a histone deacetylase inhibitor in combination with a specific linker containing N-hydroxysuccinimide (NHS) moiety able to covalently bound, through an amide bond, to the side chain of a Lys residue of mAbs. The compounds ST8152AA1, ST8189AA1 and ST8190AA1 are examples of payloads with a specific linker containing maleimide moiety able to covalently bond, through a maleimide-thiol conjugation reaction to Cys on mAbs, after their reduction.

**Table 1.**

| **Payloads** | | | |
|---|---|---|---|
| Code number | Chemical Structure | M.W. | Class |
| ST8128AA1 | 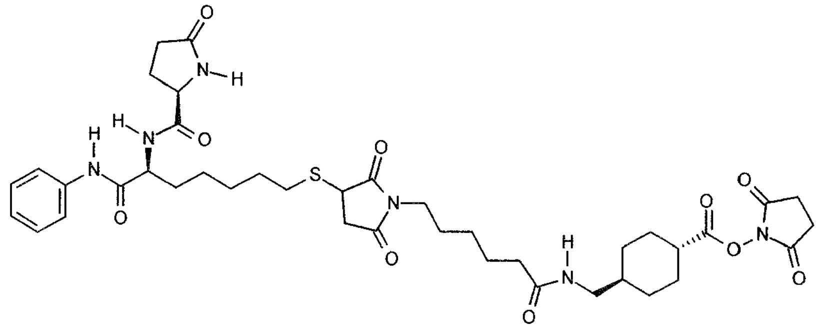 | 811 | NHS |
| ST8152AA1 | 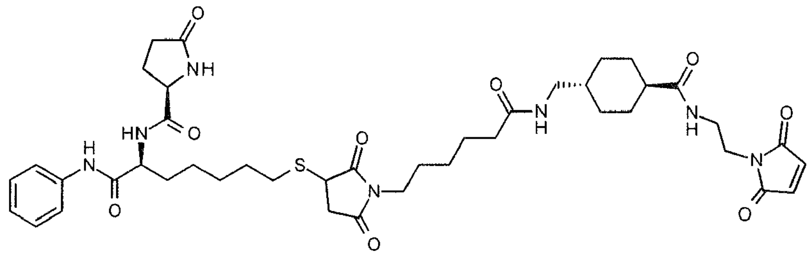 | 836 | Maleimide |
| ST8132AA1 | 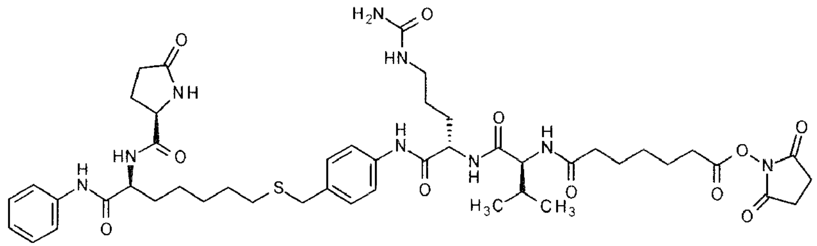 | 964 | NHS |

(continued)

| Payloads | | | |
|---|---|---|---|
| Code number | Chemical Structure | M.W. | Class |
| ST8190AA1 | 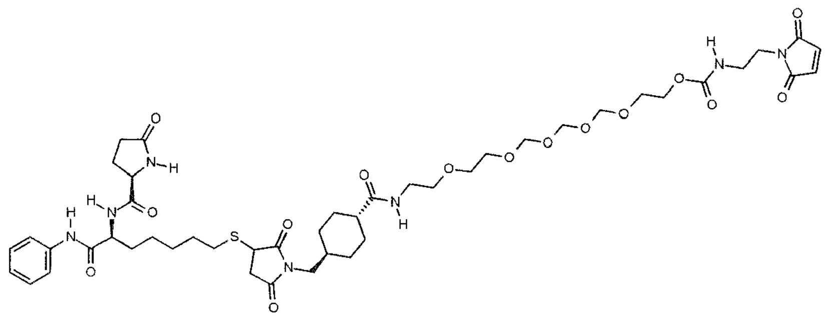 | 1030 | Maleimide |
| ST8189AA1 | 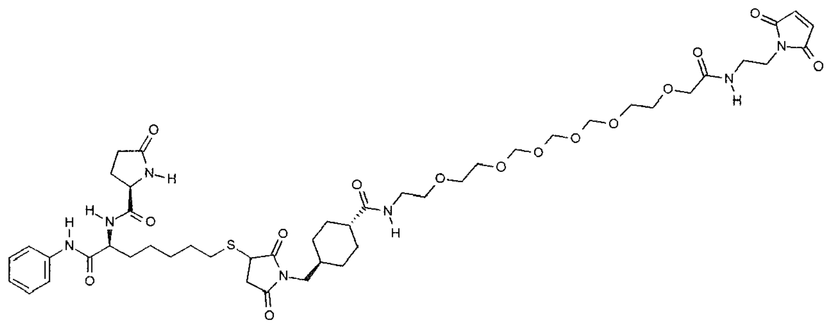 | 1043 | Maleimide |
| ST8197AA1 | 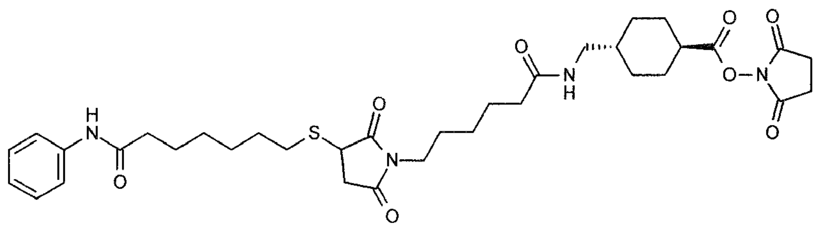 | 684 | NHS |

CU is a connecting unit which may be absent or present and may be succinimide, or 3,4-disubstituted succinimide, or succinamic acid of the following formulas

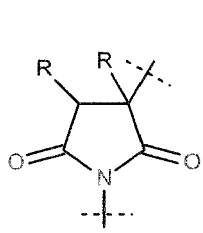

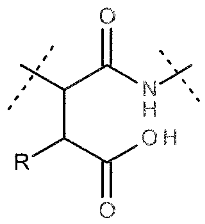

wherein R can be H or a C1-C3 alkyl group.

As a further component the antibody-drug-conjugate may comprise a spacer 1, which can be alkylcarbonyl, cycloalkylcarbonyl, ω-aminoalkylcarbonyl, amino-arylcarbonyl. alkylaminocarbonyl, cycloalkylaminocarbonyl.

Specific examples of the above residues are: propanoyl; 3-aminopropanoyl; butanoyl; 4-aminobutanoyll; pentanoyl; 5-aminopentanoyl; hexanoyl; 6-aminohexanoyl; {2-[2-ethoxy]ethoxy}acetyl; [2-(2-aminoethoxy)ethoxy]acetyl; (2-{2-[2-ethoxy]ethoxy}ethoxy)acetyl; (2-{2-[2-aminoethoxy]ethoxy}ethoxy)acetyl; cyclohexanoyl; 4-aminocyclohexanoyl; 4-methylhexanoyl; 4-aminomethylcyclohexanoyl; benzoyl; *p*-aminobenzoyl; *p*-methylbenzoyl; *p*-aminomethylbenzoyl; phenylacetyl; *p*-aminophenylacetyl; furanoyl; 5-aminofuranoyl; 5-metylfuranoyl; 5-aminomethylfuranoyl.

The antibody-drug-conjugate further contains a linker, which is a chemical moiety that allows basically the drug to bind to the antibody. The linker can be a cleavable or non-cleavable linker. An example of a cleavable linker is

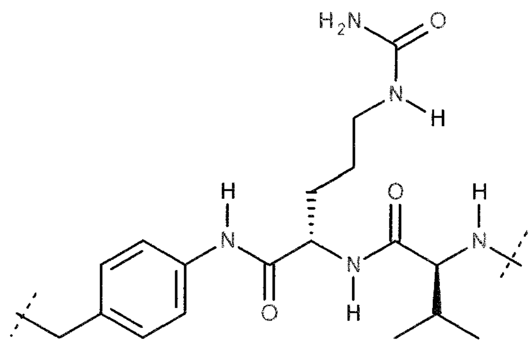

Examples of a non-cleavable linker are

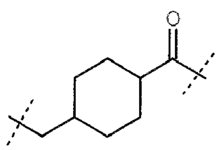

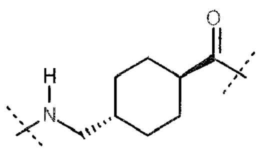

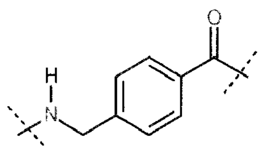

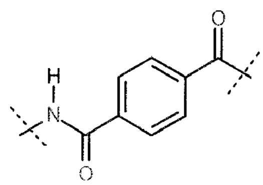

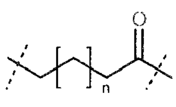

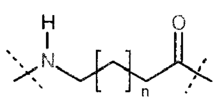

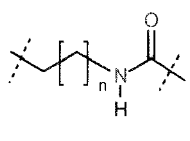

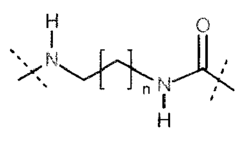

n = 0-4

The claimed antibody drug conjugates may further contain a spacer 2, which may have the formula:

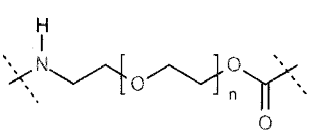

;

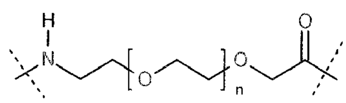

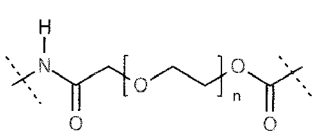

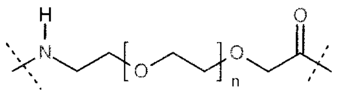; 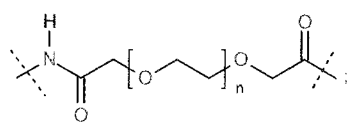; 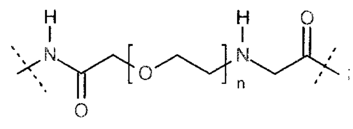;

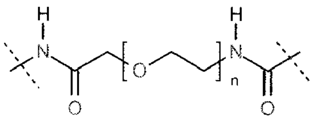 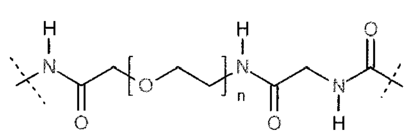

n = 2-5

The claimed antibody-drug-conjugates may further contain a connecting group which may be NHS or an activated acylderivative (including 1-hydroxybenzotriazole ester, ethyl 2-cyano-2-(hydroximino)acetate ester, N-ethoxycarbonyl-2-ethoxy-1,2-dihydro-quinolone ester, pentaflurophenyl ester, p-nitro and 2,4-dinitrophenol ester, thiophenol ester, acylimidazole, isobutylcarbonate, trichlorobenzoic anhydride, pivalic anhydride, 3,5-dimethoxytriazine) or maleimide- or 3-methylenesuccinimide, 3,4-dibromo maleimide or {amino-carbonyl}-3-butenoic acid of the following formulae:

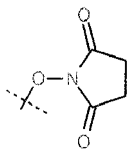; 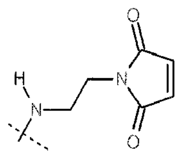; 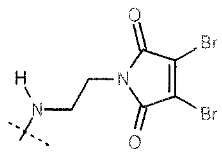; 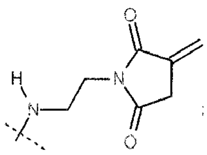; 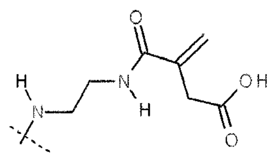

The antibody used in the claimed antibody-drug-conjugates are basically directed against a histone deacetylase inhibitor.

The antibody used in the claimed antibody-drug-conjugate is particularly an antibody directed against an EGFR family protein. Specifically, the antibody may be directed to the ErbB1, ErbB2 or ErbB3 receptors. The same payloads conjugated to other antibodies can be directed against other receptors internalized by tumor cells to release the HDACi such as c-met or integrin receptors.

For example, in analogy to EGFR, c-Met implicated in the growth, survival and spread of various human cancers and overexpressed in different solid tumors is internalized in response to HGF (hepatocyte growth factor) binding, leading to c-Met ubiquitination and degradation *(*Mellman 2013 Cold Spring Harb Perspect Biol 5:a016949*).*

In addition, integrins have major roles in tumor-stroma interactions and aberrant recycling of 25 different integrin heterodimers is involved in tumor growth, invasion, metastasis and evasion of apoptosis *(*Mosesson 2008 Nat Rev Cancer 8: 835-50*).*

Preferably, the antibody is selected from Trastuzumab, Cetuximab, Bevacizumab, Panitumumab and anti-ErbB3 Fab Sigma Tau proprietary or related biosimilars.

The terms antibody or immunoglobulin may be used interchangeably in the broader sense and include monoclonal antibodies, polyclonal antibodies, isolated, engineered or recombinant antibodies, full-length or intact antibodies, multivalent or multispecific antibodies such as bispecific antibodies or antibody fragments thereof as long as they exhibit the desired biological activity. In case of a recombinant antibody, which is the result of the expression of recombinant DNA within living cells, the antibody may be derived from any species and is preferably derived from humans, rats, mice and rabbits. If the antibody is derived from a species other than a human species, it is preferably a chimeric or humanized antibody prepared according to techniques well-known in the art.

The antibody may also be a chemically synthesized antibody.

The antibody is capable of targeting the cancer or tumor cells in question, in particular cancer or tumor cells expressing ErbB1, ErbB2 and/or ErbB3 receptors. Particularly, the antibody has the property of recognizing said cancer or tumor cells, has a property of binding to said cancer or tumor cells and a property of internalizing into a tumor or a cancer cell.

Methods of preparing said antibodies are well-known in the art [i.e., Chem. Soc. Rev., 2016, 45, 1691-1719; Bioorganic & Medicinal Chemistry Letters 26 (2016) 1542-1545].

Instead of an antibody, an antigen-binding fragment can also be used which indicates any peptide, polypeptide or protein retaining the ability to bind to the target (antigen) of the antibody. Examples of antigen-binding fragments are Fv, ScFv (Sc means single-chain), Fab, F(ab')2, Fab', ScFv', Fc fragments or Diabodies or fragments the half-life of which has been increased by a chemical modification, such as, for example, pegylation or by incorporation into a liposome.

In an embodiment of Formula I, there is provide a compound, such as an antibody drug conjugate (ADC), or pharmaceutically acceptable salt thereof, of Formula (Ia):

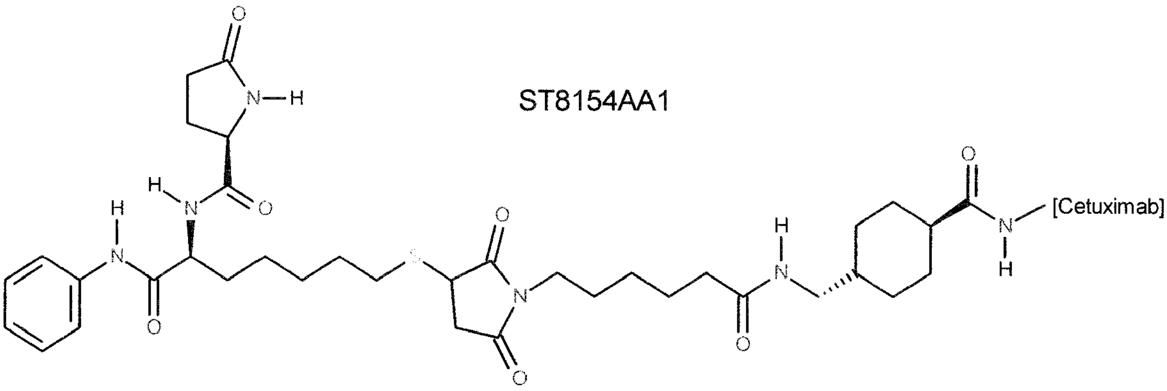

ST8154AA1

**Formula (Ia)**

In another embodiment of Formula I, there is provide a compound, such as an antibody drug conjugate (ADC), or pharmaceutically acceptable salt thereof, of Formula (Ib):

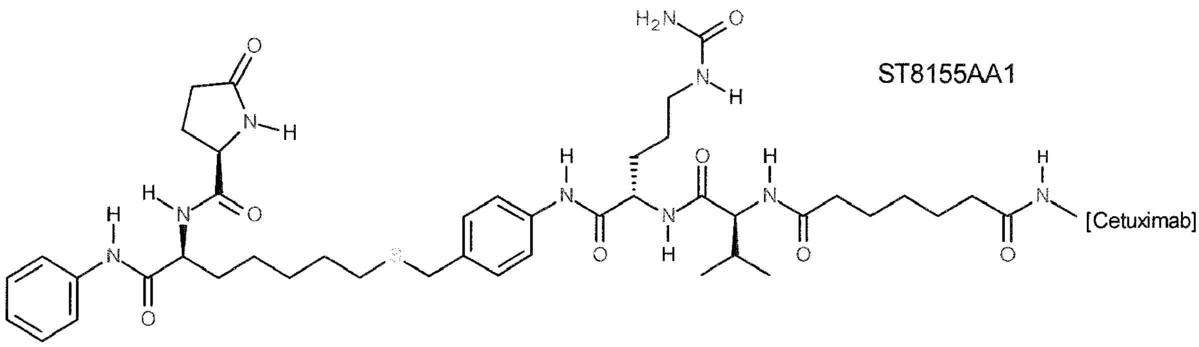

ST8155AA1

**Formula (Ib)**

In another embodiment of Formula I, there is provide a compound, such as an antibody drug conjugate (ADC), or pharmaceutically acceptable salt thereof, of Formula (Ic):

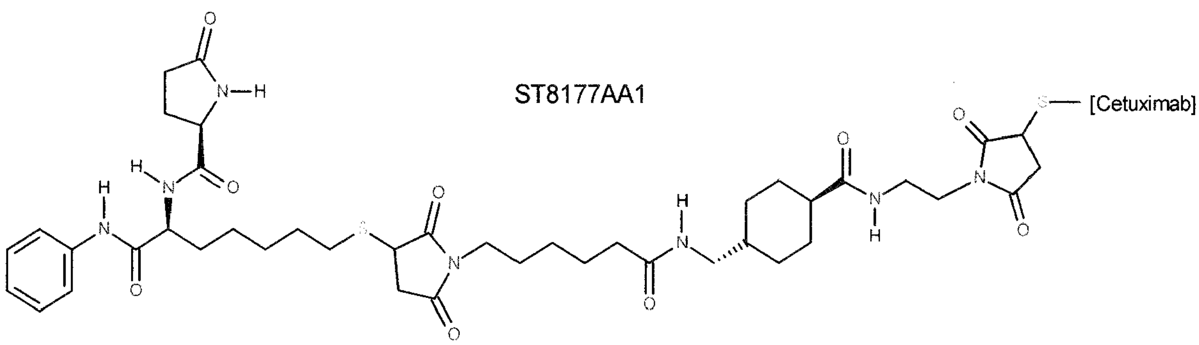

ST8177AA1

**Formula (Ic)**

In another embodiment of Formula I, there is provide a compound, such as an antibody drug conjugate (ADC), or pharmaceutically acceptable salt thereof, of Formula (Id):

ST8178AA1

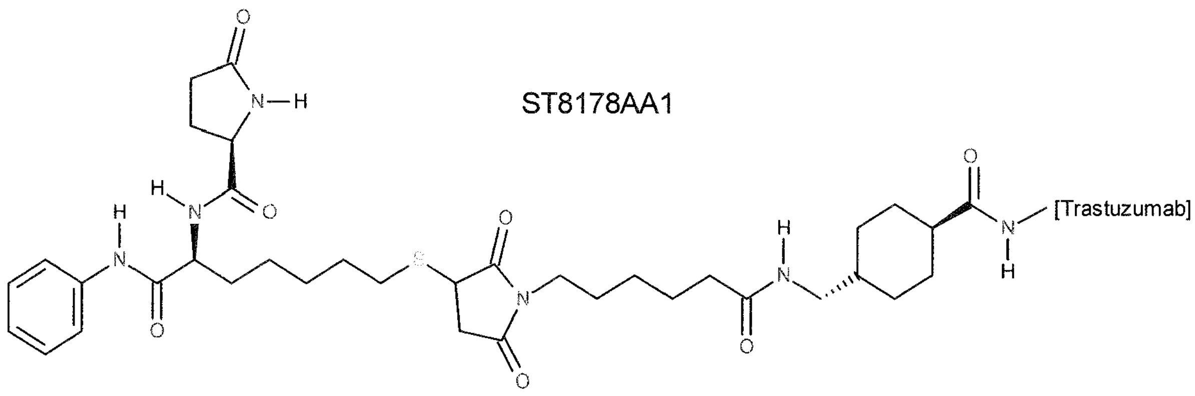

**Formula (Id)**

In another embodiment of Formula I, there is provide a compound, such as an antibody drug conjugate (ADC), or pharmaceutically acceptable salt thereof, of Formula (Ie):

ST8178AA1

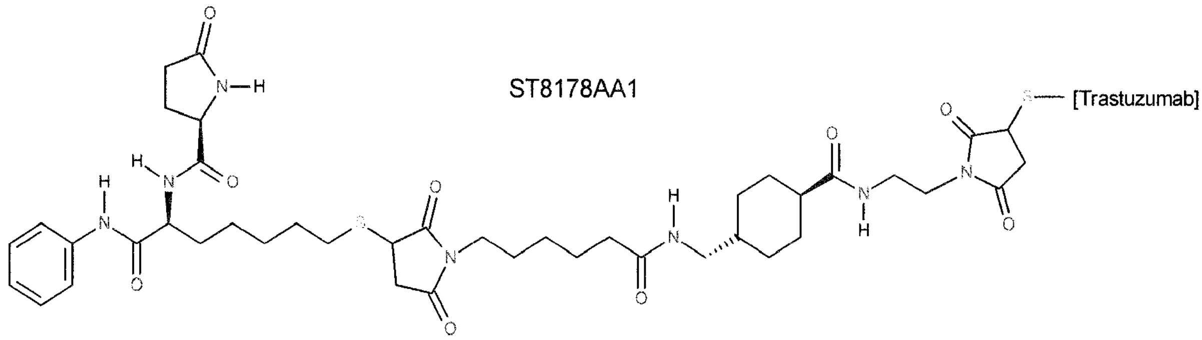

**Formula (Ie)**

In another embodiment of Formula I, there is provide a compound, such as an antibody drug conjugate (ADC), or pharmaceutically acceptable salt thereof, of Formula (If):

ST8179AA1

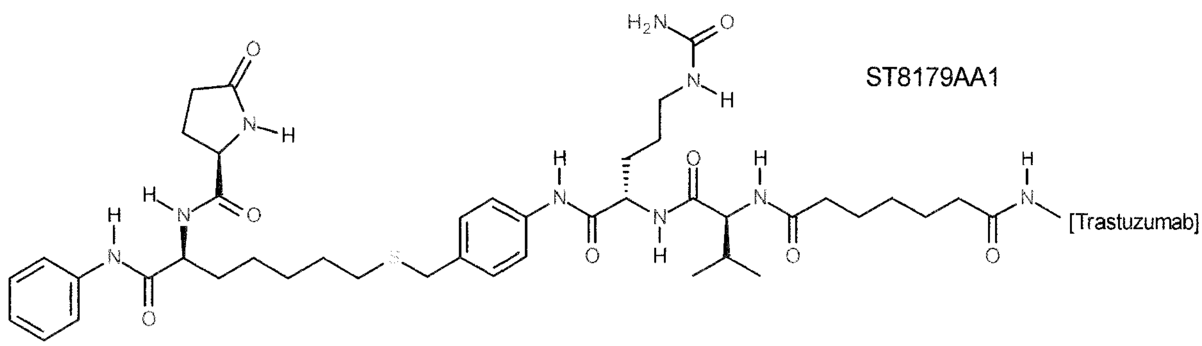

In another aspect, there is provide a method of synthesis of payloads suitable for conjugation to immunoglobulins like mAbs (monoclonal antibodies) or their corresponding fragments (FABs). Preferred examples of the payloads are:

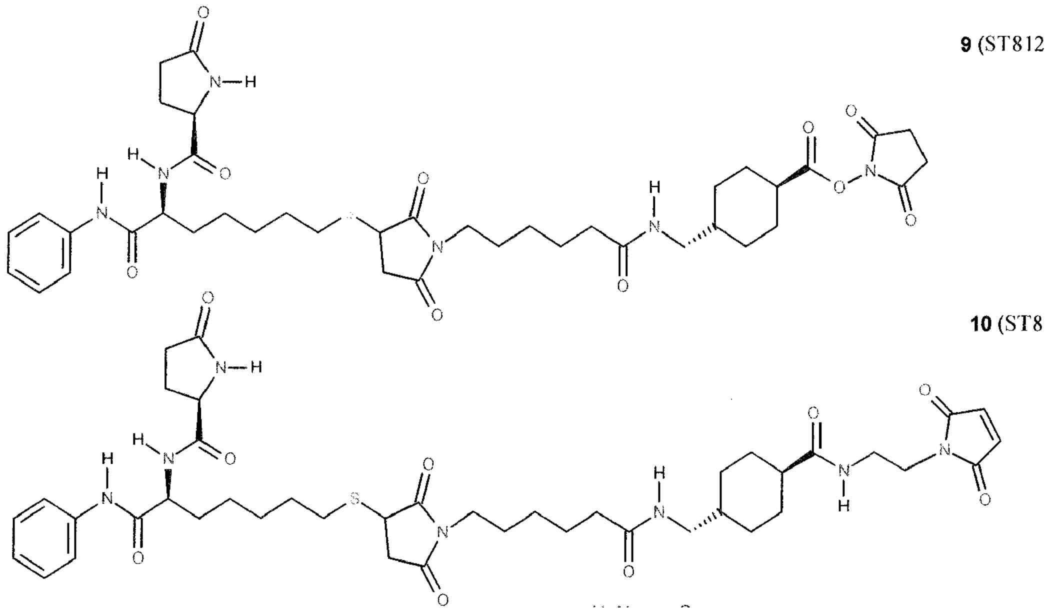

**9** (ST8128AA1)

**10** (ST8152AA1)

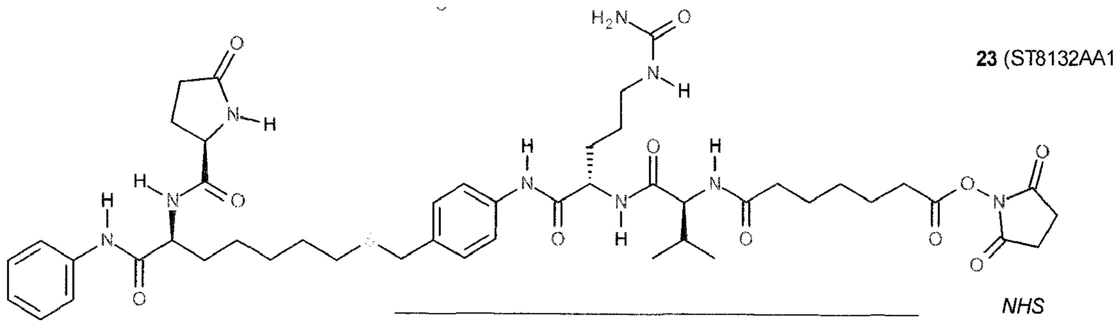

**23** (ST8132AA1)

*ST7464AA1 (drug of ST7612AA1)*

*linker*

*NHS*

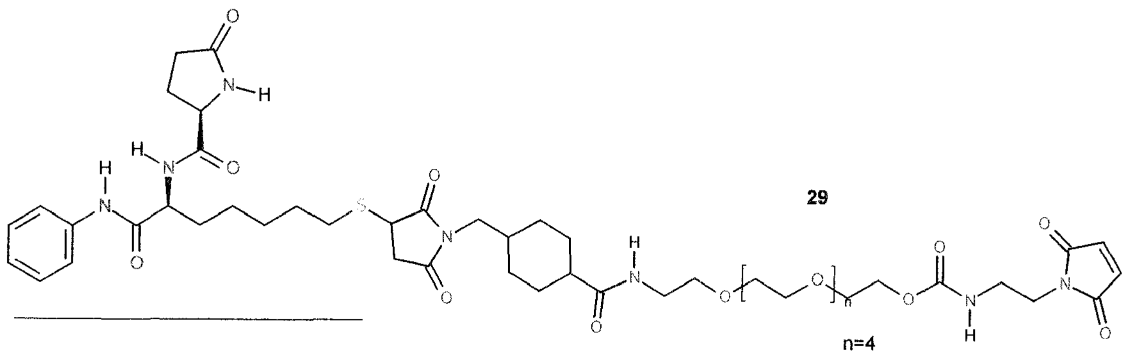

**29**

n=4

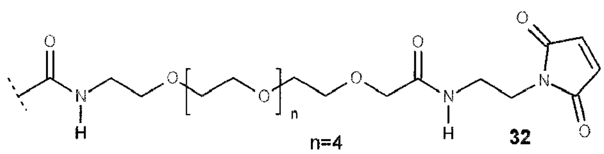

n=4

**32**

In the following a general scheme of HDACi-based ADCs is shown. The active drug (D) is represented by ST7464AA1 that corresponds to the active drug of ST7612AA1. ST7464AA1 is the drug of payloads (D-(C.U.)-Spacer1-Linker-Spacer2-) described in the present invention.

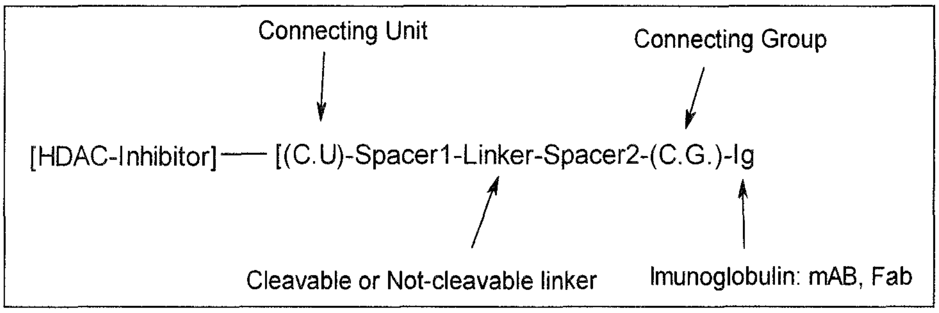


The HDAC-Inhibitors may be of the following categories:

**Thiol-based HDAC Inhibitors, such as**

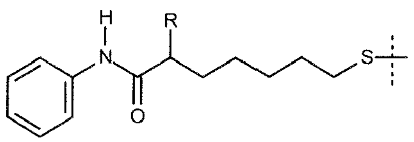

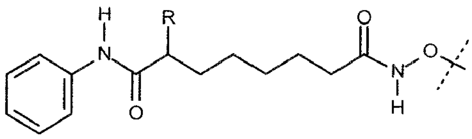

**Hydroxamic acid-based HDAC Inhibitors, such as**

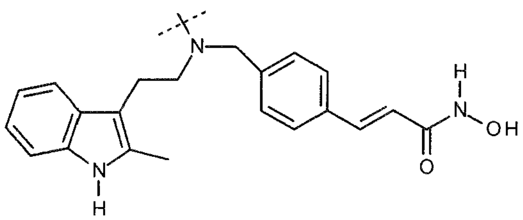

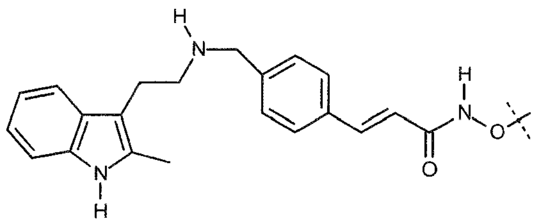

**Benzamid-based HDAC Inhibitors, such as**

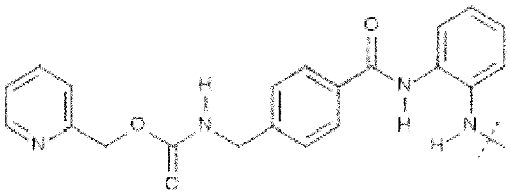

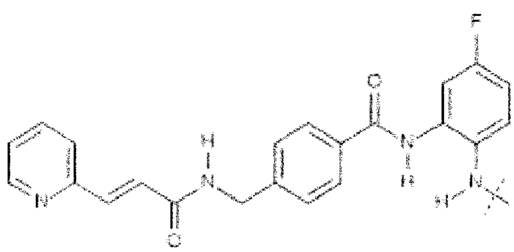

The above is a general scheme of representative HDAC inhibitors-based Antibody Drug Conjugates (ADCs).

The present invention also relates to a pharmaceutical composition comprising the above antibody-drug conjugates. Said pharmaceutical compositions contain at least an excipient and/or a pharmaceutically acceptable vehicle. The active ingredient can be administered in unit form of administration in admixture with conventional pharmaceutical carriers to animals or to human beings. Suitable unit forms of administration comprise forms via oral route or forms for administration via parenteral route (subcutaneous, intradermal, intramuscular or intravenous).

A solid composition for oral administration can be a tablet, a pill, a powder, a capsule or a granulate. In these

compositions the antibody-drug-conjugate of the invention is mixed with one or more inert diluent such as starch, cellulose, sucrose, lactose or silica. These compositions may also comprise further substances, such as lubricants, such as magnesium stearate or talc or coloring agents or coating agents.

A sterile composition for parenteral administration may preferably be an aqueous or non-aqueous solution, suspension or emulsion. A solvent or vehicle used can be made of water, propylene glycol or polyethylene glycol, vegetable oils, injectable organic esters or other suitable organic solvents. These compositions may also contain adjuvants, in particular wetting, isotonic, emulsifying, dispersing and stabilizing agents.

A particular embodiment of the invention is a formulation suitable for local delivery by nebulization. In this regard the antibody-drug-conjugates of the invention are particularly suitable in the treatment of diseases associated with the lung or the peritoneum such as lung or peritoneal cancer or cancer from ovarian, cervix-endometrium, gastric, colon, appendiceal, pseudomyxoma peritonei, pancreas, liver metastases, rare neoplasie (abdominal sarcoma of not gut tissues).

The present invention moreover relates to the antibody-drug-conjugates or a pharmaceutical composition comprising the drug antibody conjugates of the invention for use in the treatment of cancer or a tumor. The invention particularly relates to the treatment of a cancer or tumor expressing ErbB1, ErbB2 and/or ErbB3 receptors. Specific examples of the cancer to be treated according to the present invention are lung, breast, colon, brain, head and neck endometrial cancer, renal cancer, pancreatic cancer, gastric cancer, oesophageal cancer, ovarian and prostate cancer and leukemia. In addition, the HIV reactivation induced by the HDACi-based ADCs can be potentially useful for new therapies aiming at the eradication of the viral reservoirs *(*Badia 2015 Antiviral Res 123: 62-9).

In this regard, the above described antibody-drug-conjugates may be used as an adjuvant therapeutic in the treatment of HIV.

## Examples

The present invention will now be further described with reference to the following examples. It will be appreciated by a person skilled in the art that these examples are only for illustrative purpose and are not to be construed to limit the scope of the present invention.

### Example 1:

### A) Synthesis and characterization of payloads

**Scheme 1.** Synthesis of ST8128AA1

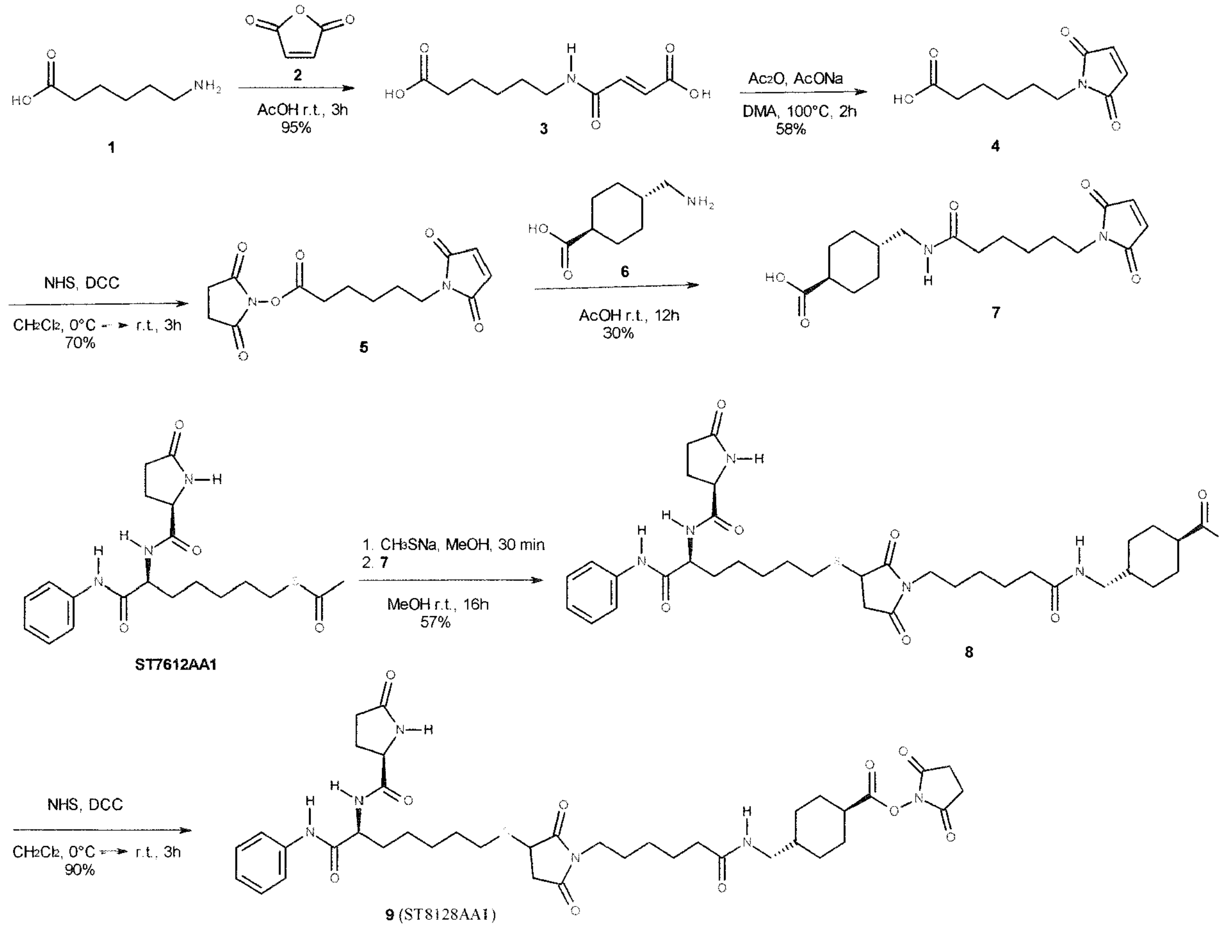


(E)-6-(3-Carboxyacrylamido)hexanoic acid (3)

A solution of maleic anhydride (2; 1.95 g, 19.9 mmol) in glacial acetic acid (10 mL) is added dropwise to a stirring solution of 6-aminocaproic acid (1; 2.6 g, 19.9 mmol) in glacial acetic acid (10 mL). The mixture is maintained at room temperature for 3 hours. The precipitate formed during the reaction is then filtrated, washed with diethyl ether in order to obtain compound 3 as a white solid, 4.3 g (95% yield). Any further purification of the solid is not required.
**$^1$H NMR** (400 MHz, DMSO) δ 9.10 (s, 1H), 6.35 (d, *J* = 12.6 Hz, 1H), 6.18 (d, *J* = 12.6 Hz, 1H), 3.12 (m, 2H), 2.15 (t, *J* = 7.4 Hz, 2H), 1.44 (m, 4H), 1.33 - 1.12 (m, 2H).
**$^{13}$C NMR** (100 MHz, DMSO) δ 174.8, 172.4, 165.8, 133.5, 132.2, 33.9, 28.5, 26.3, 24.5, 21.4.

**6-(2,5-Dioxo-2,5-dihydro-1H-pyrrol-1-yl)hexanoic acid (4)**

Acetic anhydride (3.8 mL, 40.75 mmol) and sodium acetate (418 mg, 5.09 mmol) are added at room temperature to a solution of compound 3 (4.3 g, 20.37 mmol) in dimethylacetamide (20 mL) under vigorous stirring and then heated to 100° C for 2 hours. The mixture is then cooled to room temperature, diluted with dichloromethane (35 mL) and washed 5-6 times with HCl 0.5 M (6 portions of 10 mL each) to remove the dimethylacetamide. The organic layer is dried over anhydrous sodium sulfate, filtered and the solvent removed under vacuum to give product **4** as a colorless oil, 2.5 g (58% yield).
**$^1$H NMR** (400 MHz, MeOD) δ 9.87 (bs, 1H), 6.76 (d, *J* = 1.6 Hz, 2H), 3.54 - 3.38 (m, 2H), 2.52 - 2.39 (m, 1 H), 2.30 - 2.26 (m, 1 H), 1.70 - 1.46 (m, 4H), 1.39 - 1.19 (m, 2H).
**$^{13}$C NMR** (100 MHz, MeOD) δ 179.4, 171.1, 133.9, 36.9, 33.2, 29.2, 24.1, 23.3.

**2,5-Dioxopyrrolidin-1-yl 6-(2,5-dioxo-2,5-dihydro-1H-pyrrol-1-yl)hexanoate (5)**

Dicyclohexyl-carbodiimide (2.68 g, 13.02 mmol) and N-hydroxysuccinimide (1.36 g, 11.84 mmol) are added at room temperature to a stirrer solution of compound **4** (2.5 g, 11.84 mmol) in anhydrous dichloromethane (15 mL) and the mixture stirred at room temperature for 3 hours. The white solid is filtered with dichloromethane to remove the dicyclohexylurea, the organic phase is washed with HCl 0.1 N and water, then dried over anhydrous sodium sulfate and the solvent removed by rotatory evaporation. The resulting residue is subjected to flash column chromatography with a medium pressure system Sepacore® Buchi (silica gel; gradient A: petroleum ether/B: ethyl acetate; B% 0-80 in 15 minutes) to give the activated acid 5 as a white solid, 2.4 g (70%). MS: m/z 309 $[M+H]^+$
**$^1$H NMR** (400 MHz, MeOD) δ 6.78 (s, 2H), 3.49 - 3.46 (m, 2H), 2.82 (s, 4H), 2.62 - 2.59 (m, 2H), 1.78 - 1.65 (m, 2H), 1.64 - 1.50 (m, 2H), 1.46 - 1.26 (m, 2H).
**$^{13}$C NMR** (100 MHz, MeOD) δ 169.8, 169.0, 167.3, 132.5, 35.4, 28.6, 26.1, 23.8, 23.6, 22.3.

**4-((6-(2,5-Dioxo-2,5-dihydro-1 H-pyrrol-1-yl)hexanamido)methyl)cyclohexanecarboxylic acid (7)**

trans-4-(Aminomethyl)-cyclohexane carboxylic acid (**6**; 2.38g, 15.17 mmol) is added at room temperature to a stirrer solution of compound **5** (2.46 g, 7.98 mmol) in acetic acid glacial (20 mL) and the reaction mixture is stirred at room temperature for 16 hours. The acetic acid is removed under reduced pressure, the residue diluted with dichloromethane and washed with water to eliminate the N-hydroxysuccinamide. The organic layer is dried over anhydrous sodium sulfate, filtered and the solvent removed under vacuum. The residue is subjected to flash chromatography with a medium pressure system Sepacore® Buchi, silica gel in gradient 0-100 % of ethyl acetate in petroleum ether in 7 minutes, and 100% of ethyl acetate in 5 minutes, to give compound 7 as a white solid, 807 mg (30% yield). MS: m/z 349 $[M-H]^+$
**$^1$H NMR** (400 MHz, MeOD) δ 10.47 (bs, 1H), 7.81 (s, 1H), 6.66 (d, *J* = 2.3 Hz, 2H), 3.35 (s, 2H), 2.88 (s, 2H), 2.04 (s, 2H), 1.86 (d, *J* = 10.5 Hz, 2H), 1.68 (d, *J* = 10.1 Hz, 2H), 1.47 (s, 2H), 1.36 - 1.03 (m, 8H), 0.85 (d, *J* = 11.7 Hz, 2H).
**$^{13}$C NMR** (100 MHz, MeOD) δ 176.8, 173.0, 169.5, 132.4, 43.4, 41.5, 35.9, 35.5, 33.8, 27.9, 26.6, 25.9, 24.4, 23.6.

**4-((6-(2,5-Dioxo-3-((7-oxo-6-(5-oxopyrrolidine-2-carboxamido)-7-(phenylamino)heptyl)thio)pyrrolidin-1-yl)hexanamido)methyl)cyclohexanecarboxylic acid (8)**

A solution of sodium thiomethylate 1M in degassed methanol (17 mg, 0.25 mmol, 0.250 mL) is added at room temperature to a solution of **ST7612AA1** (100 mg, 0.25 mmol) in degassed methanol. The stirring mixture is maintained at room temperature for 30 minutes. The reaction mixture is flushed with $N_2$ for 5-10 minutes to remove MeSH and compound **7** (88 mg, 0.25 mmol) is added. The mixture is kept at room temperature for 16 hours. The solvent is then removed by rotatory evaporation and the crude purified by column chromatography in gradient 2-20% methanol in dichloromethane. The compound **8** is obtained as a white solid, 102 mg (57%). MS: m/z 737 $[M+Na]^+$
**$^1$H NMR** (400 MHz, MeOD) δ 9.97 (bs, s), 7.92 (m 2H), 7.60 (m, 2H), 7.34 (m, 2H), 7.14 (m, 1H), 4.57 (m, 1H), 4.31 (m, 1 H), 3.54 (m, 2H), 3.06 (d, *J* = 4.7 Hz, 2H), 2.49-2.14 (m, 4 H), 2.10-2.02 (m, 7 H), 1.87-1.62 (m, 12 H), 1.76 - 1.23 (m, 12H), 1.01 (d, *J* = 12.4 Hz, 2H).
**$^{13}$C NMR** (100 MHz, MeOD) δ 179.6, 178.4, 177.0, 175.3, 174.7, 173.0, 170.7, 128.0 (2C), 123.7, 119.5 (2C), 77.6, 56.2, 53.5, 44.6 (2C), 42.7, 38.6, 37.7, 35.5, 35.0, 31.4, 30.3, 29.1 (2C), 28.9, 28.0 (3C), 27.4, 26.4, 25.5, 25.0, 24.6, 24.4.

**2,5-Dioxopyrrolidin-1-yl 4-((6-(2,5-dioxo-3-((7-oxo-6-(5-oxopyrrolidine-2-carboxamido)-7-(phenylamino)heptyl)thio)pyrrolidin-1-yl)hexanamido)methyl)cyclohexanecarboxylate (9)**

In a 50 mL flask under $N_2$ compound **8** (102 mg, 0.14 mmol) is dissolved in anhydrous dichloromethane (5 mL) containing 0.1 mL of dry dimethylformamide. N-Hydroxysuccinamide (24 mg, 0.21 mmol) and dicyclocarbodiimide (50 mg, 0.24 mmol) are added at room temperature and the reaction mixture is stirred at room temperature for 16 hours. The solvent is removed and the residue purified by flash chromatography in 2-6% methanol in dichloromethane. The product **9** is obtained as a colorless viscous liquid; 101 mg (90% yield). MS: m/z 811 $[M+H]^+$; 833$[M+Na]^+$
**$^1$H NMR** (400 MHz, $CDCl_3$) δ 9.43 (s, 1 H), 7.78 (d, *J* = 7.0 Hz, 1 H), 7.54 (d, *J* = 7.6 Hz, 2H), 7.35 - 7.14 (m, 2H), 7.08 (t, *J* = 7.0 Hz, 1 H), 6.12 (s, 1 H), 4.67 (d, *J* = 6.4 Hz, 1H), 4.23(s, 1H), 3.69 (s, 1 H), 3.48 (s, 2H), 3.17 - 2.93 (m, 3H), 2.83 (s, 5H), 2.26 - 2.03 (m, 9H), 1.97 - 1.71 (m, 8H), 1.71 - 1.18 (m, 16H), 1.02 (dd, *J* = 24.0, 11.7 Hz, 2H).
**$^{13}$C NMR** (100 MHz, CDCb) δ 178.9, 176.7, 176.4, 174.4, 173.2, 172.7, 170.5, 170.3, 168.9, 137.4, 128.48 (2C), 124.06, 119.58 (2C), 53.9, 45.4, 45.1, 42.1, 39.2, 38.7, 38.3, 37.6, 37.3, 36.3, 32.1, 31.3 (4C), 29.9, 28.8, 28.2, 27.2, 26.2, 25.3, 18.6, 17.3, 12.0.

## Scheme 2. Synthesis of ST8152AA1

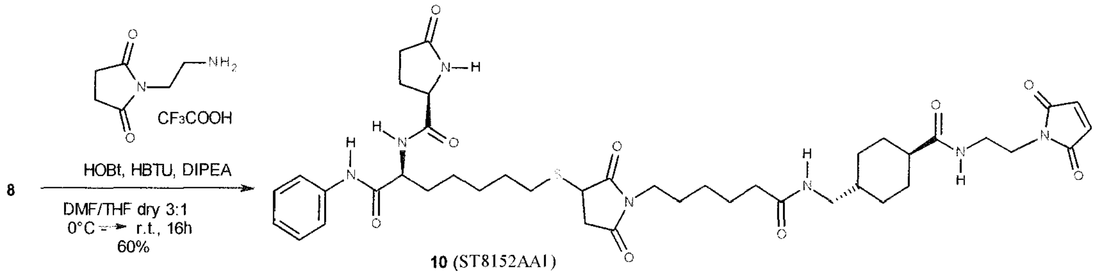


10 (ST8152AA1)

**N-(7-((1-(6-(((4-((2-(2,5-Dioxo-2,5-dihydro-1H-pyrrol-1-yl)ethyl)carbamoyl)cyclohexyl)methyl)amino)-6-oxohexyl)-2,5-dioxopyrrolidin-3-yl)thio)-1-oxo-1-(phenylamino)heptan-2-yl)-5-oxopyrrolidine-2-carboxamide (10)**

In a 50 mL flask under $N_2$ compound **8** (87 mg, 0.11 mmol) is dissolved in a mixture of tetrahydrofuran/dimethylformamide 3:1(4 mL) at 0° C. Then N-(2-aminoethyl)maleimide trifluoroacetate salt (33 mg, 0.13 mmol), 1-hydroxybenzotriazole hydrate (22 mg, 0.16 mmol), HBTU (61 mg, 0.16 mmol) and *N,N*-diisopropylethylamine (0.048 mL, 0.27 mmol) are added and the mixture kept at 0° C for 30 minutes, and then at room temperature for 16 hours. The reaction mixture is diluted with dichloromethane and washed two times with water and two times with brine, dried over anhydrous sodium sulfate and the solvent removed by rotatory evaporation. The residue is purified by flash column chromatography 2-20% methanol in dichloromethane. The product 10 is obtained as a colorless viscous liquid, 55 mg (60% yield). MS: m/z 859 $[M+Na]^+$

**$^1$H NMR** (400 MHz, $ADCl_3$) $\delta$ 9.46 (bs, 1 H), 7.60 (d, *J* = 7.9 Hz, 2H), 7.36 - 7.23 (m, 2H), 7.09 (t, *J* = 7.4 Hz, 1 H), 6.72 (s, 2H), 6.49 (t, *J* = 5.6 Hz, 1 H), 6.32 (t, *J* = 5.7 Hz, 1 H), 4.65 (d, *J* = 6.1 Hz, 1 H), 4.37 - 4.24 (m, 1 H), 3.83 - 3.60 (m, 2H), 3.60 - 3.37 (m, 5H), 3.25 - 2.97 (m, 4H), 2.89 - 2.61 (m, 2H), 2.60 - 2.11 (m, 4H), 2.00 (m, 5H), 1.90 - 1.72 (m, 13H), 1.72 - 1.15 (m, 8H), 0.94 (dd, *J* = 24.3, 11.4 Hz, 2H).

## Scheme 3. Synthesis of ST8132AA1

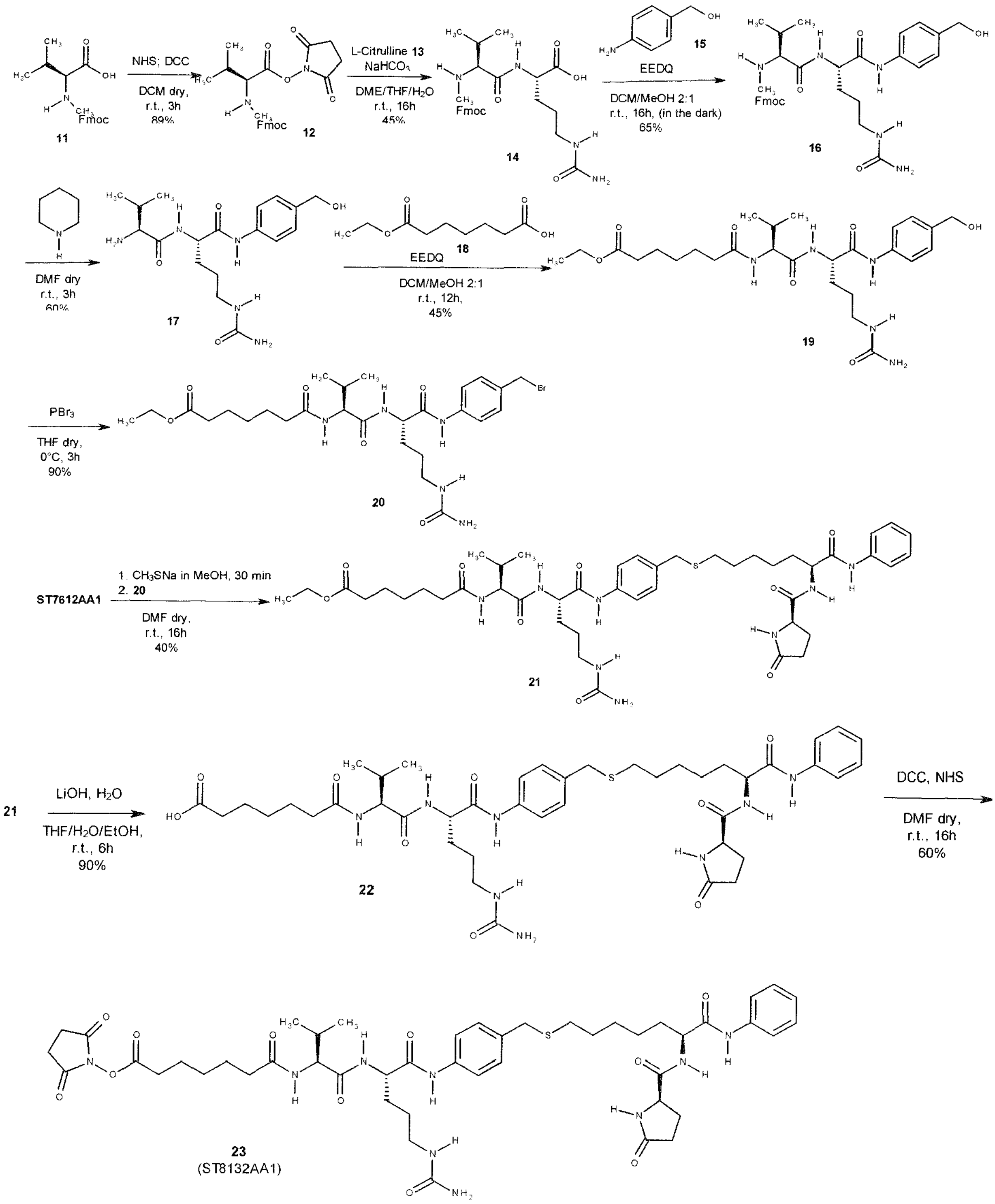


**(S)-2,5-Dioxopyrrolidin-1-yl 2-((((9H-fluoren-9-yl)methoxy)carbonyl)amino)-3-methylbutanoate (12)**

Dicyclocarbodiimide (1.55 g, 7.52 mmol) and N-hydroxysuccinimide (762 mg, 6.63 mmol) are added at room temperature to a stirrer solution of Fmoc-Val-OH (**11**, 1.5 g, 4.42 mmol) in anhydrous dichloromethane (25 mL). The mixture is kept at room temperature for 3 hours. The white solid formed in this reaction is filtrated with dichloromethane to remove the dicyclohexylurea, the organic phase is washed with HCl 0.1 N and water, then dried over anhydrous sodium sulfate and the solvent removed by rotatory evaporation. The residue is subjected to a flash column chromatography in 1% methanol in dichloromethane to afford product **12** as a with solid, 1.7 g (89% yield). MS: m/z 459 [M+Na]$^+$
**$^1$H NMR** (400 MHz, $CDCl_3$) δ 7.80 (d, *J* = 7.5 Hz, 2H), 7.68 - 7.55 (m, 2H), 7.43 (t, *J* = 7.4 Hz, 2H), 7.34 (dd, *J* = 15.9, 8.5 Hz, 2H), 4.70 (d, *J* = 4.6 Hz, 1 H), 4.56 - 4.40 (m, 3H), 4.28 (t, *J* = 6.6 Hz, 1 H), 2.85 (s, 4H), 2.37 (dd, *J* = 12.3, 6.5 Hz, 1 H), 1.08 (dd, *J* = 11.0, 6.9 Hz, 6H).

**(S)-2-((S)-2-((((9H-Fluoren-9-yl)methoxy)carbonyl)amino)-3-methylbutanamido)-5-ureidopentanoic acid (14)**

Compound **12** (1.7 g, 3.9 mmol) in dimethoxyethane (10 mL) is added to a solution of L-citrulline **(13,** 700 mg, 4 mmol) dissolved in a mixture of tetrahydrofuran and aqueous sodium bicarbonate (344 mg, 4 mmol in 10 mL of water). The reaction is stirred at room temperature for 16 hours. A solution of citric acid 15% in water (50 mL) is added and the mixture extracted with 10% isopropyl alcohol in ethyl acetate (2x75 mL). The solvent is removed by rotatory evaporation. After addition of diethyl ether and irradiation with ultrasounds, the formation of a solid is obtained. Filtration followed by washing with diethyl ether gave **14** as a white solid, 870 mg (45%). MS: m/z 495 [M-H]-**$^1$H NMR** (400 MHz, DMSO) δ 8.19 (bm 3H), 7.87 (t, *J* = 25.1 Hz, 2H), 7.77 (t, *J* = 6.8 Hz, 2H), 7.49 - 7.23 (m, 4H), 5.99 (s, 1 H), 5.43 (s, 2H), 4.45 - 4.08 (m, 4H), 3.97 (t, *J* = 7.5 Hz, 1 H), 2.99 (d, *J* = 5.1 Hz, 2H), 2.02 (d, *J* = 6.1 Hz, 1 H), 1.74 (s, 1 H), 1.61 (d, *J* = 7.5 Hz, 1 H), 1.44 (s, 2H), 0.91 (dd, *J* = 12.5, 6.3 Hz, 6H).
**$^{13}$C NMR** (101 MHz, DMSO) δ 173.4, 171.3, 169.8, 167.8, 158.8, 156.0, 143.8, 140.7, 127.6, 127.0, 65.7, 59.8, 51.9, 46.7, 30.5, 26.6, 19.18 (2C).

**(9H-Fluoren-9-yl)methyl ((S)-1-(((S)-1-((4-(hydroxymethyl)phenyl)amino)-1-oxo-5-ureidopentan-2-yl)amino)-3-methyl-1-oxobutan-2-yl)carbamate (16)**

EEDQ (840 mg, 3.4 mmol) is added to a solution containing compound **14** (870 mg, 1.7 mmol) and p-aminobenzyl alcohol (227 mg, 1.8 mmol) in dichloromethane/methanol 2:1 (15 mL). The reaction is left in the dark at room temperature for 16 hours. The solvents are removed and the resulting solid residue filtrated using diethyl ether to give product **16** as a white solid, 660 mg (65% yield). MS: m/z 624 [M+Na]$^+$
**$^1$H NMR** (400 MHz, DMSO) δ 10.00 (bs, 1 H), 8.13 (m, 4H), 7.92 (d, *J* = 7.3 Hz, 2H), 7.76 (d, *J* = 7.6 Hz, 2H), 7.58 (d, *J* = 8.1 Hz, 2H), 7.51 - 7.10 (m, 2H), 6.02 (s, 1 H), 5.43 (m, 4H), 5.13 (s, 1 H), 4.47 (s, 3H), 4.31 (m 4H), 3.13 - 2.74 (m, 2H), 2.03 (s, 1 H), **1.83 -** 1.55 (m, 2H), 1.43 (s, 2H), 0.90 (d, *J* = 6.7 Hz, 6H).
**$^{13}$C NMR** (101 MHz, DMSO) δ 171.2, 170.4, 158.93, 156.15, 144.6, 143.8, 140.7, 137.5, 127.6, 127.2 (2C) 125.3, 120.1 (2C), 118.9, 65.7, 62.6, 60.1, 53.0, 46.7, 31.0, 30.5, 26.7, 19.6, 18.7.

**(S)-2-((S)-2-Amino-3-methylbutanamido)-N-(4-(hydroxymethyl)phenyl)-5-ureidopentanamide (17)**

Piperidine (2.2 mL) is added to a solution of compound **16** (660 mg, 1.09 mmol) in anhydrous dimethylformamide (5 mL) and the reaction left at room temperature for 3 h. The solvent is removed under vacuum and the residue treated with dichloromethane in order to obtain a solid that is filtered to give product **17,** 240 mg (60% mg). MS: m/z 380 [M+H]$^+$
**$^1$H NMR** (400 MHz, DMSO) δ 10.11 (s, 1 H), 8.25 (s, 1 H), 7.58 (d, *J* = 8.2 Hz, 2H), 7.27 (d, *J* = 8.2 Hz, 2H), 6.10 (s, 1 H), 5.46 (s, 2H), 4.48 (m, 3H), 3.23 - 3.09 (m, 2H), 3.11 - 2.88 (m, 2H), 1.99 (dd, J = 12.2, 6.4 Hz, 2H), 1.99 (dd, *J* = 12.2, 6.4 Hz, 1 H), 1.81 - 1.55 (m, 2H), 1.55 - 1.28 (m, 2H), 1.04 - 0.68 (m, 6H).
**$^{13}$C NMR** (101 MHz, DMSO) δ 173.3, 170.4, 158.9, 137.5, 126.9 (2C), 125.3, 119.9 (2C), 62.6, 59.3, 52.6, 31.1, 29.9, 26.6, 19.3, 17.0 (2C).

**Ethyl 7-(((S)-1-(((S)-1-((4-(hydroxymethyl)phenyl)amino)-1-oxo-5-ureidopentan-2-yl)amino)-3-methyl-1-oxobutan-2-yl)amino)-7-oxoheptanoate (19)**

EEDQ (311 mg, 1.26 mmol) is added to a solution of compound 17 (240 mg, 0.63 mmol) and monoethyl pimelate **(18,** 0. 0.123 mL, 0.69 mmol) in a mixture of dichloromethane/methanol 2:1 (20 mL). The reaction is left in the dark at room temperature for 12 hours. The solvents are removed and the residue purified by column chromatography 2-20% methanol in dichloromethane to give product **19** as a viscous solid, 170 mg (45% yield). MS: m/z 572 [M+Na]$^+$
$^1$H **NMR** (400 MHz, MeOD) δ 7.59 (d, *J* = 8.3 Hz, 2H), 7.34 (d, *J* = 8.3 Hz, 2H), 4.65 - 4.44 (m, 4H), 4.25 (d, *J* = 7.4 Hz, 1 H), 4.15 (dd, *J* = 14.2, 7.1 Hz, 2H), 3.30 - 3.06 (m, 2H), 2.34 (dd, *J* = 13.9, 6.7 Hz, 2H), 2.13 (d, *J* = 6.9 Hz, 1 H), 1.92 (dd, *J* = 14.1, 6.2 Hz, 2H), 1.89 - 1.74 (m, 2H), 1.74 - 1.34 (m, 12H), 1.30 (dd, *J* = 18.0, 10.9 Hz, 3H), 1.06 - 0.84 (m, 8H).
**$^{13}$C NMR** (101 MHz, MeOD) δ 174.5, 172.1, 171.7, 170.4, 158.9, 136.5, 126.8 (2C), 124.1, 120.9 (2C), 62.9, 59.5, 58.6, 53.0, 38.4, 34.7, 33.0, 29.8, 29.2, 27.9, 27.2, 25.0, 23.6, 17.5, 16.9, 12.7.

**Ethyl 7-(((S)-1-(((S)-1-((4-(bromomethyl)phenyl)amino)-1-oxo-5-ureidopentan-2-yl)amino)-3-methyl-1-oxobutan-2-yl)amino)-7-oxoheptanoate (20)**

Phosphorus tribromide (0.043 mL, 0.46 mmol) is added at 0° C to a solution of compound **19** (170 mg, 0.31 mmol) in anhydrous tetrahydrofuran (5 mL). The mixture is kept at 0° C for 3 hours. The reaction mixture is directly submitted to a silica column for the purification by flash chromatography 1-6% methanol in dichloromethane to give the product **20** as a white solid, 170 mg (90% yield). MS: m/z 613 $[M+H]^+$; 635$[M+Na]^+$
**$^1$H NMR** (400 MHz, Acetone-d6) $\delta$ 9.85 (d, *J* = 6.1 Hz, 1 H), 7.85 (dd, *J* = 25.2, 8.1 Hz, 2H), 7.39 (dt, *J* = 44.7, 22.3 Hz, 2H), 4.90 - 4.54 (m, 5H), 4.19 - 3.97 (m, 2H), 3.58 (t, *J* = 6.6 Hz, 1H), 3.36 (d, *J* = 20.6 Hz, 2H), 2.41 (dd, *J* = 21.3, 6.3 Hz, 2H), 2.33 - 2.16 (m, 3H), 2.14 - 2.02 (m, 2H), 2.04 - 1.93 (m, 2H), 1.93 - 1.73 (m, 2H), 1.71 - 1.46 (m, 2H), 1.48 - 1.13 (m, 6H), 1.11 - 0.77 (m, 8H).

**Ethyl 7-(((S)-3-methyl-1-oxo-1-(((S)-1-oxo-1-((4-((((S)-7-oxo-6-((R)-5-oxopyrrolidine-2-carboxamido)-7-(phenylamino)heptyl)thio)methyl)phenyl)amino)-5-ureidopentan-2-yl)amino)butan-2-yl)amino)-7-oxoheptanoate (21)**

**ST7612AA1** (125 mg, 0.31 mmol) is dissolved in degassed methanol (8mL) in a 50 mL flask under $N_2$ atmosphere. A solution of sodium thiomethylate 1M in degassed methanol (22 mg, 0.31 mmol) is added at room temperature to the first solution. The stirring mixture is maintained at room temperature for 30 minutes. The solution is flushed with $N_2$ for 5-10 minutes to eliminate MeSH, then the solvent is removed by rotatory evaporation. Compound **20** (170 mg, 0.31 mmol) dissolved in a minimum amount of anhydrous dimethylformamide is added to the residue and the mixture kept at room temperature for 16 hours. The solvent is removed by rotatory evaporation and the crude purified by column chromatography, 1-40% methanol in dichloromethane to give **21** as an amorphous solid; yield, 106 mg (40% yield). MS: m/z 896 $[M+H]^+$; 918 $[M+Na]^+$
**$^1$H NMR** (400 MHz, MeOD) $\delta$ 7.60 (s, 4H), 7.43 - 7.18 (m, 4H), 7.13 (s, 1H), 4.55 (m, 4H), 4.40 (m, 2H), 4.13 (m, 2H), 3.66 (m, 6H), 3.19 (s, 4H), 2.75 (s, 2H), 2.37 (m, 6H), 2.11 (s, 2H), 1.97 (s, 1H), 1.82 (s, 2H), 1.64 (s, 7H), 1.42 (s, 4H), 1.27 (s, 4H), 1.01 (s, 6H).

**7-(((S)-3-Methyl-1-oxo-1-(((S)-1-oxo-1-((4-((((S)-7-oxo-6-((R)-5-oxopyrrolidine-2-carboxamido)-7-(phenylamino)heptyl)thio)methyl)phenyl)amino)-5-ureidopentan-2-yl)amino)butan-2-yl)amino)-7-oxoheptanoic acid (22)**

Lithium hydroxide monohydrate (42 mg, 1 mmol) is added to a solution of **21** (300 mg, 0.33 mmol) in a mixture of tetrahydrofuran/water/ethanol 1:1:1 (12 mL). The reaction is kept at room temperature for 6 hours, then it is diluted with ethyl acetate and washed with HCl 1N. The crude (180 mg) is directly used for the next step without any purification. MS: m/z 874 $[M+Li]^+$; 890$[M+Na]^+$

**2,5-Dioxopyrrolidin-1-yl 7-(((S)-3-methyl-1-oxo-1-(((S)-1-oxo-1-((4-((((S)-7-oxo-6-((R)-5-oxopyrrolidine-2-carboxamido)-7-(phenylamino)heptyl)thio)methyl)phenyl)amino)-5-ureidopentan-2-yl)amino)butan-2-yl)amino)-7-oxoheptanoate (23)**

Dicyclohexylcarbodiimide (70 mg, 0.35 mmol) and N-hydroxysuccinimide (34 mg, 0.3 mmol) are added at room temperature to a stirrer solution of compound **22** (180 mg, 0.20 mmol) in anhydrous dimethylformamide (3 mL). The mixture is kept at room temperature for 16 hours. The white solid formed in this reaction is filtrated to remove the dicyclohexylurea, the organic phase is washed with HCl 0.1 N and water, then dried over anhydrous sodium sulfate and the solvent removed under vacuum. The resulting residue is subjected to flash column chromatography 2-20% methanol in dichloromethane to give the activated acid **23** as a white viscous solid, 109 mg (60% yield). MS: m/z 987 $[M+Na]^+$.
**$^1$H NMR** (400 MHz, DMSO) $\delta$ 10.10 (s, 4H), 9.95 (s, 1H), 7.59 (m, 4H), 7.33 (t, *J* = 7.5 Hz, 2H), 7.24 (d, *J* = 7.8 Hz, 2H), 7.08 (t, *J* = 7.2 Hz, 1 H), 6.00 (s, 1 H), 5.42 (s, 2H), 4.43 (d, *J* = 14.3 Hz, 2H), 3.67 (s, 1 H), 3.12 - 2.90 (m, 2H), 2.84 (s, 4H), 2.65 (m, 5H), 2.53 (s, 6H), 2.17 (m, 2H), 1.63 (m, 10H), 1.36 (s, 4H), 0.94 - 0.75 (m, 8H).

## Scheme 4. Synthesis of (29)

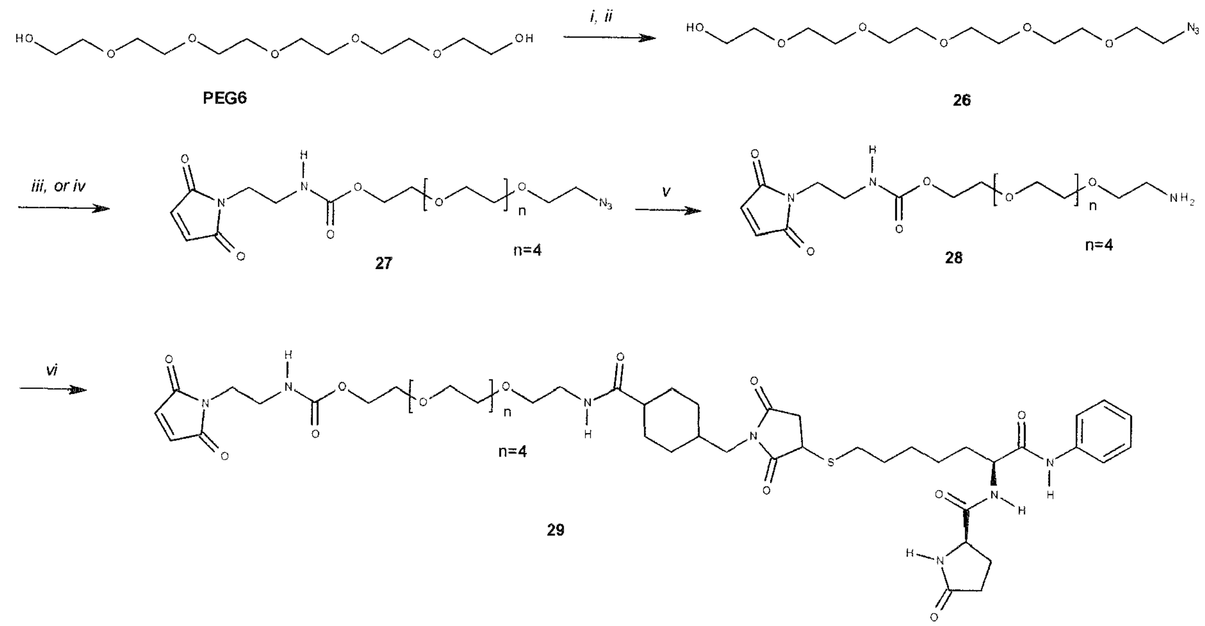


PEG6

26

27

28

29

i. Tosyl-Cl, TEA, THF; ii. $NaN_3$, DMF; iii. p-nitrophenylchloroformate, DMAP, DCM; iv. *N*-(2-aminoethyl)maleimide trifluoroacetate, DBU, DCM; v. $PPh_3$, THF; vi. 4-((2,5-dioxo-3-(((6S)-7-oxo-6-(5-oxopyrrolidine-2-carboxamido)-7-(phenylamino)heptyl)thio)pyrrolidin-1-yl)methyl)cyclohexane-1-carboxylic acid HOBt, HBtU, DIPEA, THF.

**2[-2-(2-{2-[2-(2-Hydroxyethoxy)-ethoxy]-ethoxy}-ethoxy)-ethoxy]- ethyl azide (26)**

To a solution of hexaethyleneglycol (50 g, 177 mmol) and triethylamine (14 mL, 100 mmol) in THF (250 mL) para-toluensulfonyl-chloride (13 g, 70 mmol) in THF (250 mL) was added and the solution was stirred overnight. The solution was then diluted with CH2Cl2 (200 mL) washed with 1 N HCl (3 x 150 mL) and brine (150 mL) and dried over $Na_2SO_4$ After filtration and evaporation of the solvents *in vacuo* the residue was purified by column chromatography (silica, 2% methanol in CH2Cl2) to give the monotosyl derivative (24.4 g, 56 mmol, 80% compared to p-TsCl) as colourless oil: TLC ($CH_2Cl_2$:MeOH 95:5): Rf = 0.32.

**$^1$HNMR** (400 MHz, *CDCl3*) δ = 7.79 (d, *J* = 8.0 Hz, 2H), 7.33 (d, *J* = 8.0 Hz, 2H), 4.09-4.07 (m, 2H), 3.74-3.56 (m, 22H), 2.97 (br, 1H, O*H*), 2.44 (s, 3H); $^{13}$C NMR (100 MHz, *CDCl3)* δ = 145.0, 133.3, 130.0, 128.2, 72.7, 70.9-70.5, 69.5, 68.9, 61.9, 21.8; MS (ESI): calcd. for $[M+H]^+$ *m/z* = 437.

Then a mixture of the tosylderivative (20.4 g, 56 mmol) and $NaN_3$ (3.7 g, 57 mmol) in DMF (150 mL) was stirred at room temperature overnight. DMF was evaporated *in vacuo* and the residue dissolved in AcOEt, and filtered over Celite®. The AcOEt was evaporated under reduced pressure to yield pure **26** (14.6 g, 48 mmol, 85%): TLC ($CH_2Cl_2$:MeOH 94:6): Rf = 0.38; $^1$H NMR (400 MHz, *$CDCl_3$)* δ = 3.72-3.57 (m, 22H), 3.38-3.36 (m, 2H), 2.80 (bs, 1 H); $^{13}$C NMR (100 MHz, *CDCl3*) δ = 72.8, 70.7- 70.1, 61.8, 50.7.

**17-Azido-3,6,9,12,15-pentaoxaheptadecyl(2-(2,5-dioxo-2,5-dihydro-1*H*-pyrrol-1-yl)ethyl)carbamate (27)**

To a solution of **26** (500 mg, 1.63 mmol) in $CH_2Cl_2$ (50 ml) and DMAP (1 g, 9 mmol), 4-nitrophenylchloroformiate (1.15 g, 5.7 mmol) was added at 0 °C. The mixture was stirred at room temperature 2 h. The solution was then diluted with $CH_2Cl_2$ (50 mL) washed with 1N HCl (3 x 25 mL) and brine (50 mL) and dried over $Na_2SO_4$ dry. After filtration and evaporation of the solvents *in vacuo* the residue was purified by column chromatography ($CH_2Cl_2$:MeOH 95:5 ). A yellow solid was isolated (501 mg g, 61%): Rf = 0.75; $^1$H NMR (400 MHz, *CDCl3*) δ = 8.23 (dd, J = 9.6, 2.8 Hz, 2H), 7.35 (dd, J = 9.5, 2.9 Hz, 2H), 4.43 - 4.35 (m, 2H), 3.81 - 3.74 (m, 2H), 3.74 - 3.52 (m, 24H), 3.34 (dd, J = 6.6, 3.5 Hz, 2H); MS (ESI): $[M-H]^-$ *m/z* = 471. The para-nitro derivative (501 mg, 1 mmol) and DBU (1.5 ml, 10 mmol) were dissolved in $CH_2Cl_2$ and N-(2-aminoethyl)maleimide trifluoroacetate (254 mg, 1 mmol) was added. The reaction mixture was stirred at room temperature overnight. The solution was then diluted with $CH_2Cl_2$ (25 mL) washed with 1 N HCl (3 x 10 mL) and brine (20 mL) and dried over $Na_2SO_4$ dry. After filtration and evaporation of the solvents *in vacuo* the residue was purified by column chromatography ($CH_2Cl_2$:MeOH 95:5) obtaining a white solid (47 mg, 0.1 mmol, 10%). $^1$H NMR (400 MHz,

*CDCl3*) δ: 7.80 (s, 1H), 3.92-3.50 (m, 24H), 3.38-3.36 (m, 4H); $^{13}C$ NMR (100 MHz, *CDCl3*) δ = 171.2, 157.2, 135.6, 72.8, 70.7- 70.1, 61.8, 50.7 MS (ESI): $[M+H]^+$ *m/z* = 474.

**17-Amino-3,6,9,12,15-pentaoxaheptadecyl (2-(2,5-dioxo-2,5-dihydro-1*H*-pyrrol-1-yl)ethyl)carbamate (28)**

A solution of **27** (47 mg, 0.1 mmol) in dry THF (10 mL) was cooled to 0 °C. Triphenyl phosphine (53 g, 0.2 mmol) was added, and the mixture was stirred for 24 h at room temperature. $H_2O$ (5 mL) was then added to hydrolyse the intermediate phosphorus adducts and the solution was stirred for another 24 h at room temperature. THF was evaporated and the solid residue was suspended in water (10 mL). The insoluble salts were filtered, and the filtrate washed with toluene (5 x 5 mL) and evaporated to yield **C** (44 mg, 0.1 mmol, 99%) as pale yellow oil, which was used in the next step without further purification. $^1H$ NMR (400 MHz, *CDCl3*) δ = 7.80 (s, 1H), 3.92-3.50 (m, 22H), 3.38-3.36 (m, 4H) 3.01-3.11 (m, 2H), 1.81 (bs, 2H); $[M+H]^+$ *m/z* = 448.

**1-((1S,4R)-4-((2,5-Dioxo-3-(((6S)-7-oxo-6-(5-oxopyrrolidine-2-carboxamido)-7-(phenylamino)heptyl)thio)pyrrolidin-1-yl)methyl)cyclohexyl)-1-oxo-5,8,11,14,17-pentaoxa-2-azanonadecan-19-yl (2-(2,5-dioxo-2,5-dihydro-1H-pyrrol-1-yl)ethyl)carbamate (29)**

4-((2,5-Dioxo-3-(((6S)-7-oxo-6-(5-oxopyrrolidine-2-carboxamido)-7-(phenylamino)heptyl)thio)pyrrolidin-1-yl)methyl)cyclohexane-1-carboxylic acid (60 mg, 0.1 mmol) was dissolved in dry THF (15 mL) and the reaction mixture cooled down to 0 °C. HOBt (20 mg, 0.15 mmol), HBTU (57 mg, 0.15 mmol), **28** (44 mg, 1 mmol) and DIPEA (48 μL, 0.25 mmol) were added and the reaction mixture stirred at room temperature overnight. The solution was then diluted with AcOEt (15 mL) and extracted with $H_2O$ (3 x 10 mL). The organic phases were dried over $Na_2SO_4$ dry and after filtration and evaporation of the solvents *in vacuo* the residue was purified by column chromatography ($CH_2Cl_2$:MeOH 98:2) obtaining a colourless oil (31 mg, 0.03 mmol, 10%). $[M+H]^+$ *m/z* = 1031.

## Scheme 5. Synthesis of (32)

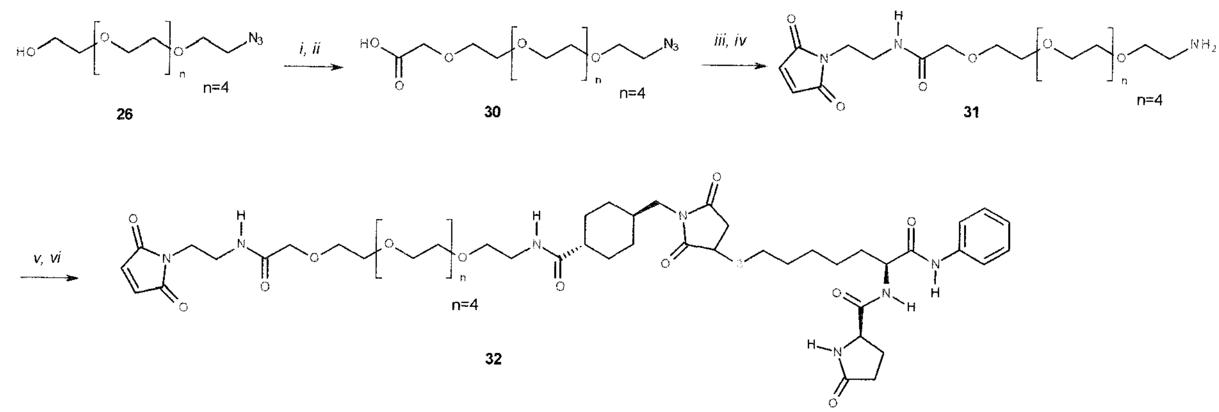

26 30 31 32

i. $EtOCOCH_2Br$, NaH, THF; ii. LiOH, THF, $H_2O$, EtOH; iii. N-(2-aminoethyl)maleimide trifluoroacetate, DBU, DCM; iv. $PPh_3$, THF; v. 4-((2,5-dioxo-3-(((6S)-7-oxo-6-(5-oxopyrrolidine-2-carboxamido)-7-(phenylamino)heptyl)thio)-pyrrolidin-1-yl)methyl)cyclohexane-1-carboxylic acid HOBt, HBtU, DIPEA, THF.

**20-Azido-3,6,9,12,15,18-hexaoxaicosanoic acid (30)**

NaH 60%wt in mineral oil (29 mg, 0.39 mmol) was suspended in dry THF (5 mL) and a solution of **26** (100 mg, 0.32 mmol) in dry THF (3 mL) was slowly added at 0 °C. After stirring for 20 min at 0 °C, a solution of ethyl-bromoacetate (53 μL, 0.48 mmol) in dry THF (3 mL) was added and the mixture stirred at room temperature over-night. $H_2O$ (1 mL) was slowly added and the solvent evaporated under reduced pressure. The solid obtained was purified by flash chromatography ($CH_2Cl_2$:MeOH 95:5) giving a pale rose oil in quantitative yields (51 mg, 0.13 mmol). $^1H$ NMR (400 MHz, *$CDCl_3$*) δ = 4.28 (s, 2H); 4.21 (q, 2H); 3.92-3.50 (m, 22H), 3.38-3.36 (m, 4H), 2.05-1.98 (m, 2H); 1.23 (t, 3H); MS (ESI): $[M+H]^+$ *m/z* = 394. The ethyl ester (51 mg, 0.13 mmol) was dissolved in a a 1:1:1 mixture of $H_2O$, THF and EtOH (15 mL) and LiOH monohydrate was added (16 mg, 0.39 mmol). The mixture was stirred at reflux for 2 h, then HCl added until pH 7. The solvents were evaporated under reduced pressure and the solid obtained washed with EtOH (2 x 10 mL). The solution was evaporated arising a colourless oil (47 mg, 0.13 mmol) directly used for the next step. MS (ESI):

[M+H]- *m/z* = 364.

**20-Amino-N-(2-(2,5-dioxo-2,5-dihydro-1H-pyrrol-1-yl)ethyl)-3,6,9,12,15,18-hexaoxaicosanamide (31)**

To a solution of **30** (47 mg, 0.13 mmol) was dissolved in dry THF (15 mL) and the reaction mixture cooled down to 0 °C. HOBt (26 mg, 0.2 mmol), HBtU (76 mg, 0.2 mmol), *N*-(2-aminoethyl)maleimide trifluoroacetate (33 mg, 0.13 mmol) and DIPEA (63 μL, 0.33 mmol) were added and the reaction mixture stirred at room temperature over-night. The solution was then diluted with AcOEt (15 mL) and extracted with $H_2O$ (3 x 10 mL). The organic phases were dried over $Na_2SO_4$ dry and after filtration and evaporation of the solvents *in vacuo* the residue was purified by column chromatography ($CH_2Cl_2$:MeOH 98:2) obtaining a colourless oil (49 mg, 0.10 mmol). $[M+H]^+$ *m/z* = 488. The azido derivative obtained (49 mg, 0.10 mmol) was dissolved in dry THF (10 mL) at 0 °C. Triphenyl phosphine (53 g, 0.2 mmol) was added and the mixture was stirred for 24 h at room temperature. $H_2O$ (5 mL) was then added to hydrolyse the intermediate phosphorus adducts and the solution was stirred for another 24 h at room temperature. THF was evaporated and the solid residue was suspended in water (10 mL). The insoluble salts were filtered, and the filtrate washed with toluene (5 x 5 mL) and evaporated to yield **31** (46 mg, 0.1 mmol, 99%) as a pale yellow oil, which was used in the next step without further purification. $^1H$ NMR (400 MHz, *$CDCl_3$*) δ = 8.03 (bs, 1H); 7.80 (s, 1H), 4.26 (s, 2H); 3.92-3.50 (m, 26H), 3.03-3.07 (m, 2H), 1.81 (bs, 2H); $[M+H]^+$ *m/z* = 462.

**N-((2S)-7-((1-((4-((1-(2,5-Dioxo-2,5-dihydro-1H-pyrrol-1-yl)-4-oxo-6,9,12,15,18,21-hexaoxa-3-azatricosan-23-yl)carbamoyl)cyclohexyl)methyl)-2,5-dioxopyrrolidin-3-yl)thio)-1-oxo-1-(phenylamino)heptan-2-yl)-5-oxopyrrolidine-2-carboxamide (32)**

4-((2,5-Dioxo-3-(((6S)-7-oxo-6-(5-oxopyrrolidine-2-carboxamido)-7-(phenylamino)heptyl)thio)pyrrolidin-1-yl)methyl)cyclohexane-1-carboxylic acid (60 mg, 0.1 mmol) was dissolved in dry THF (15 mL) and the reaction mixture cooled down to 0 °C. HOBt (20 mg, 0.15 mmol), HBtU (57 mg, 0.15 mmol), **F** (46 mg, 1 mmol) and DIPEA (48 μL, 0.25 mmol) were added and the reaction mixture stirred at room temperature over-night. The solution was then diluted with AcOEt (15 mL) and extracted with $H_2O$ (3 x 10 mL). The organic phases were dried over $Na_2SO_4$ dry and after filtration and evaporation of the solvents *in vacuo* the residue was purified by column chromatography ($CH_2Cl_2$:MeOH 98:2) obtaining a colourless oil (21 mg, 0.02 mmol, 20%). $[M+H]^+$ *m/z* = 1045.

## Scheme 6. Synthesis of (34)

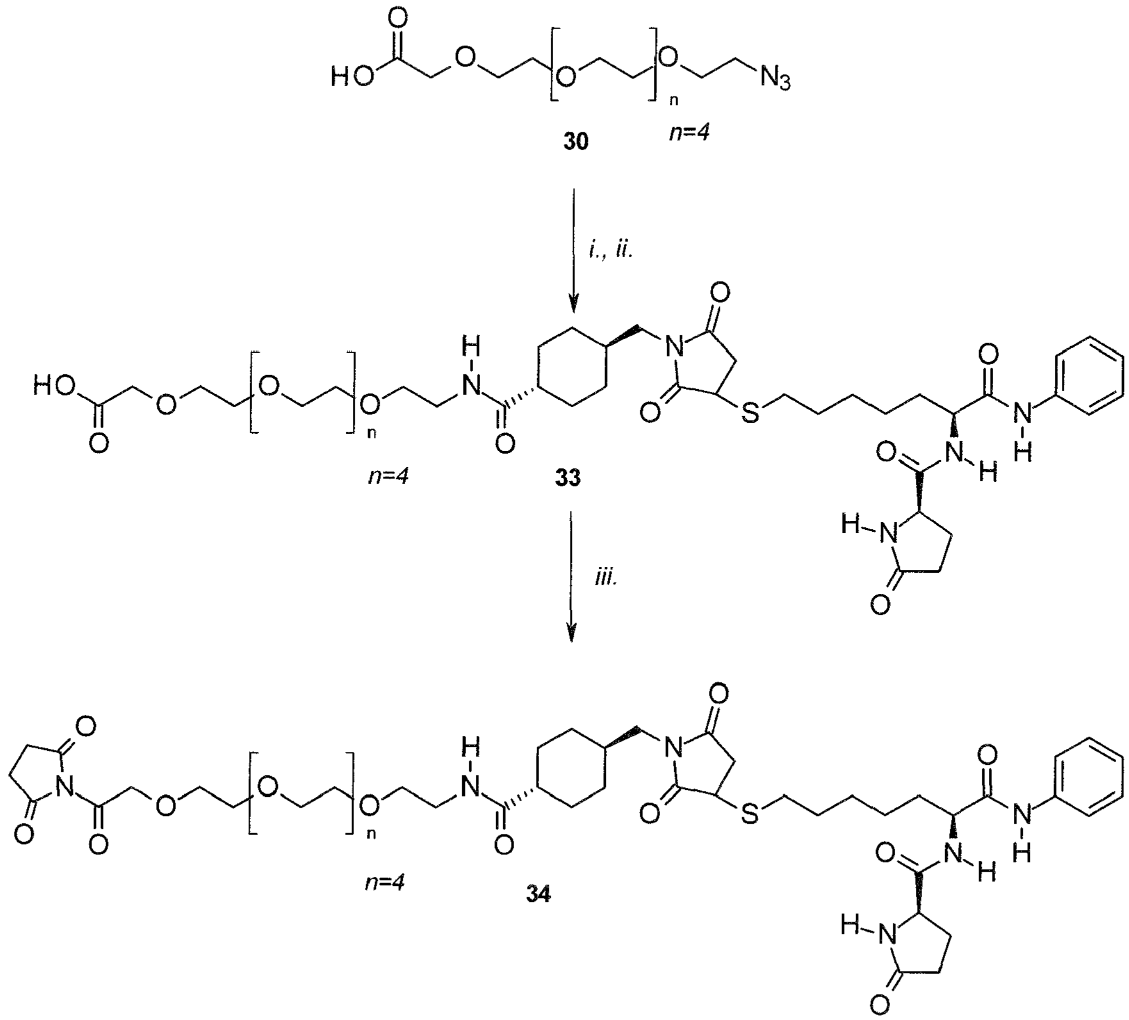


30 n=4

33 n=4

34 n=4

i.$PPh_3$, THF; ii. **8,** DCC, NHS, DCM; iii. DCC, NHS, DCM.

**20-Amino-3,6,9,12,15,18-hexaoxaicosanoic acid**

The azido 30 (49 mg, 0.10 mmol) was dissolved in dry THF (10 mL) at 0 °C. Triphenyl phosphine (53 mg, 0.2 mmol) was added and the mixture was stirred for 24 h at room temperature. $H_2O$ (5 mL) was then added to hydrolyse the intermediate phosphorus adducts and the solution was stirred for another 24 h at room temperature. THF was evaporated and the solid residue was suspended in water (10 mL). The insoluble salts were filtered, and the filtrate washed with toluene (5 x 5 mL) and evaporated to yield 20-amino-3,6,9,12,15,18-hexaoxaicosanoic acid (46 mg, 0.1 mmol, 99%) as pale yellow oil, which was used in the next step without further purification. $^1H$ NMR (400 MHz, *$CD_3OD$*) δ = 12.0 (bs, 1 H); 4.26 (s, 2H); 3.92-3.50 (m, 22H), 3.03-3.07 (m, 2H), 1.81 (bs, 2H); $[M+H]^+$ *m/z* = 340.

**1-(4-((2,5-Dioxo-3-(((6*S*)-7-oxo-6-(5-oxopyrrolidine-2-carboxamido)-7-(phenylamino)heptyl)thio)pyrrolidin-1-yl)methyl)cyclohexyl)-1-oxo-5,8,11,14,17,20-hexaoxa-2-azadocosan-22-oic acid (33)**

Compound 8 (80 mg, 0.11 mmol) in dry $CH_2Cl_2$ (5 mL) was added at room temperature to a solution of dicyclohexyl-carbodiimide (0.11 mmol) and N-hydroxysuccinimide (0.1 mmol), and the mixture stirred at room temperature for 3 hours. The white solid is filtered with dichloromethane to remove the dicyclo-hexylurea, the organic phase is washed with HCl 0.1 N and $H_2O$, then dried over dry sodium sulfate and the solvent removed under reduced pressure. The resulting residue is purified by flash column chromatography to give the activated acid as a white waxy material (yield 90%) that is dissolved into dimethoxyethane (5 mL) and treated with 20-amino-3,6,9,12,15,18-hexaoxaicosanoic acid

(46 mg, 0.1 mmol) dissolved in a mixture of tetrahydrofuran and aqueous sodium bicarbonate (15 mg, 0.15 mmol in 2 mL of water). The reaction is stirred at room temperature for 16 hours. A solution of citric acid 15% in water (2.5 mL) is added and the mixture extracted with 10% isopropyl alcohol in ethyl acetate (2 x 5 mL). The solvent is removed by rotatory evaporation. After addition of diethyl ether and irradiation with ultrasounds, the formation of a solid is obtained. Filtration followed by washing with diethyl ether gave 33 as a white solid, 65 mg (70%). MS: m/z 918 $[M-H]^-$

**2,5-Dioxopyrrolidin-1-yl 1-(4-((2,5-dioxo-3-(((6S)-7-oxo-6-(5-oxopyrrolidine-2-carboxamido)-7-(phenylamino)heptyl)thio)pyrrolidin-1-yl)methyl)cyclohexyl)-1-oxo-5,8,11,14,17,20-hexaoxa-2-azadocosan-22-oate (34)**

Compound **33** (65 mg, 0.07 mmol) in dry $CH_2Cl_2$ (5 mL) was added at room temperature to a solution of dicyclohexyl-carbodiimide (0.1 mmol) and N-hydroxysuccinimide (0.07 mmol), and the mixture stirred at room temperature for 3 hours. The white solid is filtered with dichloromethane to remove the dicy-clohexylurea, the organic phase is washed with HCl 0.1 N and $H_2O$, then dried over dry sodium sulfate and the solvent removed under reduced pressure. The resulting residue is purified by flash column chromatography to give the activated acid as a white solid 61 mg (85%). MS: m/z 1020 $[M+H]^+$.

## Scheme 7. Synthesis of (39)

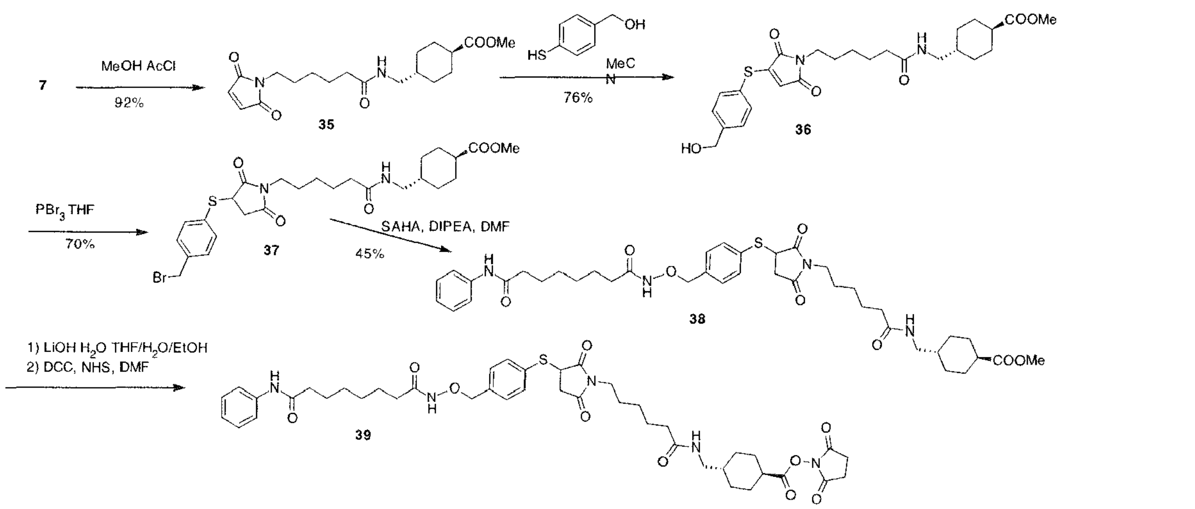


**Methyl (1R,4R)-4-((6-(2,5-dioxo-2,5-dihydro-1H-pyrrol-1-yl)hexanamido)methyl)cyclohexane-1-carboxylate (35)**

Acetyl chloride (1.01 mL, 14.26 mmol) was added to a solution of acid **7** (1.00 g, 2.85 mmol) in MeOH (50 mL) in a round bottom flask under $N_2$. The resulting solution was stirred at room temperature for 3 h, and then the solution was concentrated in vacuo. The residue was dissolved in $CH_2Cl_2$ (30 mL) and washed with a saturated solution of aqueous sodium bicarbonate (3 x 15 mL) and brine (2 x15 mL). The organic phase was dried over sodium sulfate, filtered and concentrated in vacuo to provide 950 mg (2.61 mmol) of compound **35** as a white solid (yield 92%). **$^1$H NMR** (400 MHz, $CDCl_3$, δ ppm, *J* Hz): δ 6.65 (s, 2H), 3.62 (s, 3H), 3.50-3.46 (m, 2H), 3.07 (t, *J* = 6.4, 2H), 2.23-2.11 (m, 3H), 1.97 (dd, *J* = 13.6, 2.8, 2H), 1.78 (dd, *J* = 12.8, 2.0, 2H), 1.66-1.55 (m, 4H), 1.44-1.26 (m, 5H), 0.99-0.92 (m, 2H).**MS:** *m/z* 365 $[M + 1]^+$; 387 $[M + 23]^+$. **TLC $R_f$:** 0.75 ($CH_2Cl_2$:MeOH 9:1).

**Methyl (1R,4R)-4-((6-(3-((4-(hydroxymethyl)phenyl)thio)-2,5-dioxopyrrolidin-1-yl)hexanamido)methyl)cyclohexane-1-carboxylate (36)**

Thiol **2** (548 mg, 3.91 mmol) was added to a solution of compound **4** (950 mg, 2.61 mmol) in $CH_3CN$ (20 mL) in a round bottom flask under an atmosphere of $N_2$. The resulting solution was stirred at room temperature for 3 h, and then the solution was concentrated in vacuo. The crude reaction mixture was purified by silica gel flash chromatography (petroleum ether:EtOAc 1:4) to provide 1024 mg (2.03 mmol) of compound **36** as a pale yellow viscous oil (yield 78%). **$^1$H NMR** (400 MHz, $CDCl_3$, δ ppm, *J* Hz): δ 7.46 (d, *J* = 7.6, 2H), 7.31 (d, *J* = 8.0, 2H), 5.81 (bs, 1H), 4.67 (s, 2H), 3.91-3.89 (m, 1H), 3.63 (s, 3H), 3.38-3.32 (m, 2H), 3.16-3.03 (m, 4H), 2.82 (bs, 1H), 2.21-1.96 (m, 5H), 1.77 (d, *J* = 12.8, 2H), 1.50-1.23 (m, 9H), 1.02-0.92 (m, 2H). **MS:** *m/z* 527 $[M + 23]^+$; 543 $[M + 39]^+$. **TLC $R_f$:** 0.3 (EtOAc).

**Methyl (1R,4R)-4-((6-(3-((4-(bromomethyl)phenyl)thio)-2,5-dioxopyrrolidin-1-yl)hexanamido)methyl)cyclohexane-1-carboxylate (37)**

$PBr_3$ (28 μL, 0.30 mmol) was added at 0 °C to a solution of alcohol **36** (100 mg, 0.20 mmol) in THF dry (5 mL) in a round bottom flask under an atmosphere of $N_2$. The resulting solution was stirred at 0 °C for 2 h and then was allowed to warm to room temperature; after the addition of $CH_2Cl_2$ (1 mL) the solution turned orange and was concentrated in vacuo. The crude reaction mixture was purified by silica gel flash chromatography (EtOAc 100%) to provide 78 mg (0.14 mmol) of compound **37** as a bright orange oil (yield 70%). **$^1$H NMR** (400 MHz, $CDCl_3$, δ ppm, *J* Hz): δ 7.56 (d, *J* = 8, 2H), 7.31 (d, *J* = 8.0, 2H), 4.66 (s, 2H), 3.92-3.89 (m, 1 H), 3.60 (s, 3H), 3.37-3.32 (m, 2H), 3.16-3.05 (m, 4H), 2.21-1.99 (m, 5H), *1.77* (d, *J* = 12.4, 2H), 1.50-1.28 (m, 9H), 1.01-0.92 (m, 2H).**MS**: *m/z* 589,591 $[M + 23]^+$. **TLC $R_f$**: 0.6 (EtOAc).

***p*-{[N-8-Anilino-8-oxooctanoyl(aminooxy)]methyl}phenylthio)-2,5-dioxo-1-pyrrolidinyl]hexanoylamino}methyl)cyclohexanecarboxylate (38).**

In a vial under $N_2$ atmosphere, bromide **(37)** (78 mg, 0.14 mmol), **SAHA** (50 mg, 0.18 mmol) and 1 mL of DMF dry are mixed at room temperature. Freshly distilled DIPEA (45 mg, 0.36 mmol) is added dropwise and the solution is stirred at room temperature for 12 h. The solvent is then removed via rotator evaporation and high vacuum. The residue is purified by flash column chromatography with a gradient 0-20% methanol in dichloromethane to provide the product **(48)** as a white solid 48 mg, 45% of yield. MS: m/z 751.4 $[M+H]^+$

**2,5-Dioxo-1-pyrrolidinyl 4-({6-[3-(*p*-{[N-8-anilino-8-oxooctanoyl(aminooxy)]methyl}phenylthio)-2,5-dioxo-1-pyrrolidinyl]hexanoylamino}methyl)cyclohexanecarboxylate (39).**

Lithium hydroxide monohydrate (8 mg, 0,18 mmol) is added to a solution of **38** (48 mg, 0.063 mmol) in a mixture of tetrahydrofuran/water/ethanol 1:1:1 (6 mL). The reaction is kept at room temperature for 2 hours, then it is diluted with ethyl acetate and washed with HCl 1 N. The crude (25 mg) is directly used for the next step without any purification. Dicyclocarbodiimide (11 mg, 0.05 mmol) and N-hydroxysuccinimide (6 mg, 0.045 mmol) are added at room temperature to a stirrer solution of the crude product previously obtained (25 mg, 0.034 mmol) in DMF dry (0,80 mL). The mixture is kept at room temperature for 16 hours. The white solid formed in this reaction is filtered with dichloromethane to remove the dicylohexylurea, the organic phase is washed with HCl 0.1 N and water, then dried over anhydrous sodium sulfate and the solvent removed by rotatory evaporation. The resulting residue is subjected to flash column chromatography in gradient 0-2% methanol in dichloromethane to affords the activated acid 41 as a white viscous solid; MS: m/z 856.4$[M+Na]^+$

## Scheme 8. Synthesis of (41)

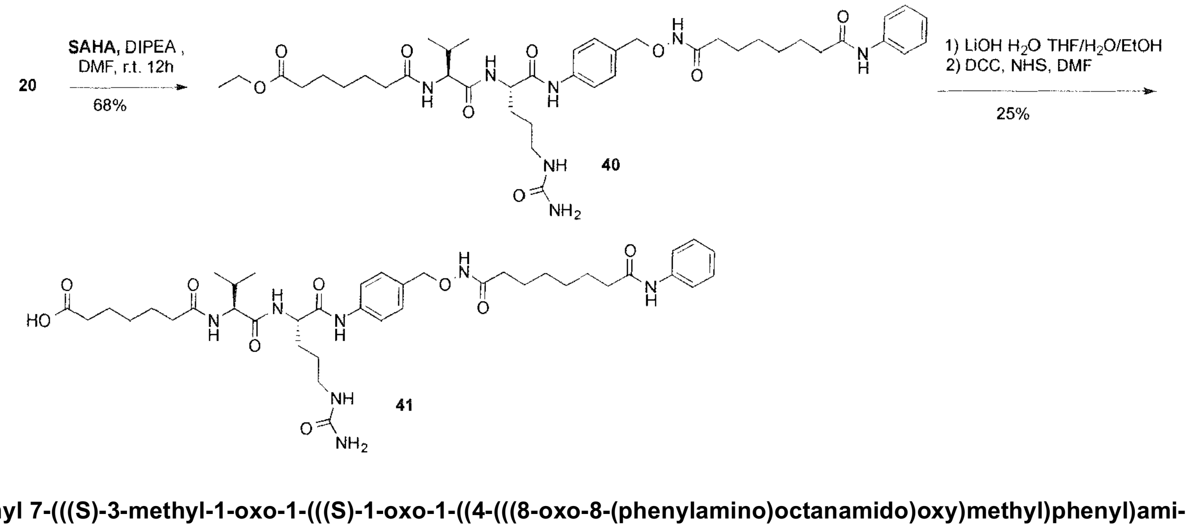


**Ethyl 7-(((S)-3-methyl-1-oxo-1-(((S)-1-oxo-1-((4-(((8-oxo-8-(phenylamino)octanamido)oxy)methyl)phenyl)amino)-5-ureidopentan-2-yl)amino)butan-2-yl)amino)-7-oxoheptanoate(40).**

In a vial under $N_2$ atmosphere, bromide **(20)** (60 mg, 0.09 mmol), **SAHA** (28 mg, 0.10 mmol) and 1 mL of DMF dry are mixed at room temperature. Freshly distilled DIPEA (25 mg, 020 mmol) is added dropwise and the solution is

stirred at room temperature for 12 h. The solvent is then removed via rotator evaporation and high vacuum. The residue is purified by flash column chromatography with a gradient 0-20% methanol in dichloromethane to provide the product **(40)** as a white solid 48 mg, 68% of yield. MS: m/z 795.9 $[M+H]^+$, 818.0 $[M+Na]^+$. **$^1$H NMR** (400 MHz, DMSO) δ 7.63-7.598 (m, 2H), 7.57 (d, J= 8 Hz, 2H), 7.39 (d, J= 8.4 Hz,2H), 7.313 (t, J= 8 Hz, 2H), 7.09 (t, J= 8; 1H), 4,89 (s, 2H), 4.58 (s, 2H), 4.20 (d, J= 7.2, 1H), 4.14 (t, J= 7.2 Hz, 2H), 4.10 (t, J= 7.4 Hz, 1H),3,56-3,45(m, 1H), 3,34 (d, J= 7.8 Hz, 2H), 2,41-2,37 (m, 1H), 2,34-2,30 (m, 2H), 2,13-2,06 (m, 2H), 1,79-1,60 (m, 4H), 1,42-1,36 (m, 4H), 1,28-1,24 (m, 3H), 1,01-0,97 (m, 6H).

**2,5-Dioxopyrrolidin-1-yl 7-(((S)-3-methyl-1-oxo-1-(((S)-1-oxo-1-((4-((((S)-7-oxo-6-((R)-5-oxopyrrolidine-2-carboxamido)-7-(phenylamino)heptyl)thio)methyl)phenyl)amino)-5-ureidopentan-2-yl)amino)butan-2-yl)amino)-7-oxoheptanoate (41).**

Lithium hydroxide monohydrate (8 mg, 0,18 mmol) is added to a solution of **40** (48 mg, 0.06 mmol) in a mixture of tetrahydrofuran/water/ethanol 1:1:1 (6 mL). The reaction is kept at room temperature for 6 hours, then it is diluted with ethyl acetate and washed with HCl 1N. The crude (25 mg) is directly used for the next step without any purification. MS: m/z 765.7 $[M-H]^-$. Dicyclocarbodiimide (11 mg, 0.05 mmol) and N-hydroxysuccinimide (6 mg, 0.045 mmol) are added at room temperature to a stirrer solution of the crude product previously obtained (25 mg, 0.03 mmol) in DMF dry (0,80 mL). The mixture is kept at room temperature for 16 hours. The white solid formed in this reaction is filtered with dichloromethane to remove the dicylohexylurea, the organic phase is washed with HCl 0.1 N and water, then dried over anhydrous sodium sulfate and the solvent removed by rotatory evaporation. The resulting residue is subjected to flash column chromatography in gradient 0-2% methanol in dichloromethane to affords the activated acid **41** as a white viscous solid; MS: m/z 887.2$[M+Na]^+$

## Scheme 10. Synthesis of (43)

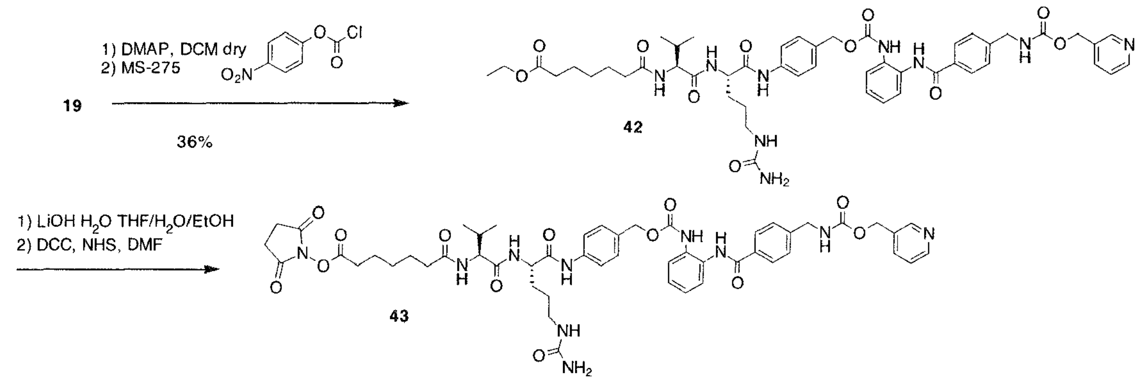


**Ethyl 7-(((S)-3-methyl-1-oxo-1-(((S)-1-oxo-1-((4-((((2-(4-((((pyridin-3-ylmethoxy)carbonyl)amino)methyl)benzamido)phenyl)carbamoyl)oxy)met hyl)phenyl)amino)-5-ureidopentan-2-yl)amino)butan-2-yl)amino)-7-oxoheptanoate (25):**

In a vial cooled to 0° C, under $N_2$ atmosphere compound **19** (50 mg, 0.09 mmol) and 4-nitrophenylchloroformate (62 mg, 0.3 mmol), 3 mL of DCM dry (with 1 drop of DMF dry) and 4-dimethylaminopyridine (60 mg, 0.49 mmol) are mixed together. Then the mixture is stirred at room temperature and monitored by TLC. When the reaction conversion of 19 is completed, **MS275** (40 mg, 0.10 mmol) is added and the reaction is stirred for additional 12h at room temperature. The mixture is then diluted with dichloromethane and washed with HCl 1N, dried over anhydrous sodium sulfate and the solvent removed by rotatory evaporation. The residue is purified by flash column chromatography in gradient 0-20% methanol in dichloromethane to give product **(43)** as a solid (12 mg, 13% yield). MS: m/z 1044.3 $[M+Na]^+$

## Scheme 11. Synthesis of ST8197AA1

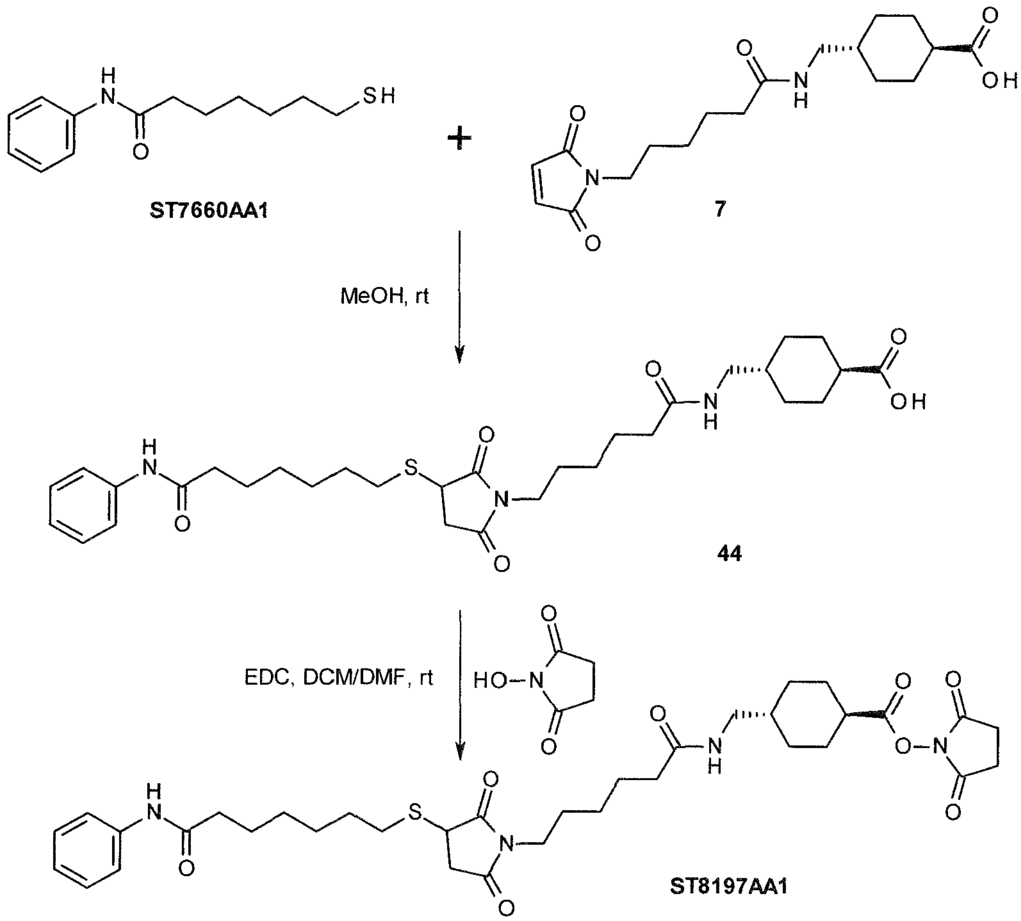


ST7660AA1

7

44

ST8197AA1

N-Phenyl-7-sulfanyl-heptanamide (ST7660AA1, 60 mg, 0.25 mmol) is suspended in degassed methanol (1 mL); compound **7** (84 mg, 0.24 mmol) is added at room temperature to the stirring mixture and after some minutes it becomes a clear solution. The solution is kept under stirring at room temperature for 20 hours till TLC monitoring shows complete conversion of 7. The solvent is then removed by rotatory evaporation and the raw material is purified by column chromatography in gradient 2-10% methanol in dichloromethane. The compound **44** is obtained as a white solid, 115 mg (81% yield). MS: m/z 610 $[M+Na]^+$

$^1$**H NMR** (500 Mhz, dmso-d6) δ 11.97 (bs, 1 H), 9.85 (s, 1 H), 7.72 (t, $J$ = 5.6 Hz, 1 H), 7.59 (d, $J$ = 7.8 Hz, 2H), 7.28 (t, $J$ = 7.8 Hz, 2H), 7.02 (t, $J$ = 7.3 Hz, 1 H), 3.93 (dd, $J$ = 9.0, 3.7 Hz, 1 H), 3.35 (t, $J$ = 6.8 Hz, 2H), 3.18 (dd, J = 18.3, 9.0, 1 H), 2.88 (t, $J$ = 6.3 Hz, 2H), 2.79 - 2.63 (m, 2H), 2.30 (t, $J$ = 7.3 Hz, 2H), 2.14 - 2.05 (m, 1H), 2.04 (t, $J$ = 7.5 Hz, 2H), 1.92 - 1.84 (m, 2H), 1.74 - 1.67 (m, 2H), 1.63 - 1.15 (m, 18H), 0.94 - 0.83 (m, 2H).

Compound **44** (110 mg, 0.19 mmol) is suspended in anhydrous dichloromethane (5.5 mL) containing 0.12 mL of dry dimethylformamide. N-hydroxysuccinimide (33 mg, 0.28 mmol) and N-(3-Dimethylaminopropyl)-N'-ethyl-carbodiimide hydrochloride (61 mg, 0.32 mmol) are added at room temperature and the reaction mixture is stirred at room temperature for 24 hours. The mixture is diluted with dichloromethane (70 mL) and washed with water (40 mL) and brine (30 mL). The organic phase is dried over $Na_2SO_4$, filtered and concentrated by rotary evaporation. The crude is purified by flash chromatography in 2-6% methanol in dichloromethane. The product **ST8197AA1** is obtained as a white solid, 97 mg (76% yield). MS: m/z 707 $[M+Na]^+$

$^1$**H NMR** (500 Mhz, dmso-d6) δ 9.83 (s, 1 H), 7.73 (t, $J$ = 5.4 Hz, 1 H), 7.59 (d, $J$ = 7.8 Hz, 2H), 7.28 (t, $J$ = 7.3 Hz, 2H), 7.02 (t, $J$ = 7.3 Hz, 1 H), 3.94 (dd, $J$ = 8.8, 3.4 Hz, 1 H), 3.36 (t, $J$ = 6.8 Hz, 2H), 3.18 (dd, $J$ = 18.3, 8.8, 1 H), 2.91 (t, $J$ = 6.0 Hz, 2H), 2.81 (s, 4H), 2.80 - 2.62 (m, 3H), 2.30 (t, $J$ = 7.3 Hz, 2H), 2.18 - 1.97 (m, 4H), 1.80 - 1.72 (m, 2H), 1.64 - 1.27 (m, 16H), 1.27 - 1.15 (m, 2H), 1.06 - 0.94 (m, 2H).

$^{13}$**C NMR** (500 Mhz, dmso-d6) δ 177.2, 175.6, 172.3, 171.6, 171.4, 170.7, 139.8, 129.1, 123.4, 119.5, 44.8, 39.6, 38.5,

37.1, 36.8, 36.3, 35.6, 30.8, 29.3 (4C), 29.0, 28.7, 28.6, 28.4, 27.3, 26.3, 25.9, 25.4, 25.3.

**B) Synthesis and characterization of ADCs**

Here, by way of example, ADCs made of HDAC is conjugated to four different antibodies and one Fab are described (Trastuzumab Herceptin® Roche, Cetuximab Erbitux® Merck, Bevacizumab Avastin® Genentech/Roche, Panitumumab Vectibix®; Amgen and anti-ErbB3 Fab Sigma Tau). Specifically, the approved antibodies are Trastuzumab recognizing ErbB2, Cetuximab and Panitumumab recognizing ErbB1, Bevacizumab recognizing VEGFR.

The antibody drug conjugates (ADCs) were prepared following two different main routes: for the conjugation to ε-amino groups of lysine residues were used N-Hydroxysuccinimide (NHS) containing linker-payloads while in the conjugation to cysteine thiol groups were used maleimide containing linker-payloads.

The average drug-to-antibody ratio (DAR) of the ADCs was measured by MALDI mass spectrometry.

**1) Methods for the preparation of ADCs through conjugation of drugs to ε-amino groups of lysine residues**

Antibodies were buffer exchanged using a 10 kDa cutoff dialysis membrane to yield antibodies solution in PBS pH 7.4 and to remove interfering preservative. The concentration after dialysis was determined measuring the $OD_{280}$ and - the absorbance reading of the sample - was divided by 1.36.

A 10 mM Kad-HDAC-NHS stock solution was prepared in DMSO and a 20-fold molar excess of payload was added to each one of antibody solution.

Reactions were incubated at room temperature, with gentle continuous mixing and after 1 hour, they were quenched adding a 20 mM glycine acqueous solution.

The final products were then dialyzed in PBS overnight at 4°C using a 10 kDa cutoff membrane in order to remove the excess of unreacted payload.

**DARs** were determined by MALDI mass spectrometry, using an Ultraflex III mass spectrometer (Bruker, GmbH), operating in positive linear mode.

Briefly, 100 μl of unconjugated antibodies and of obtained ADCs were desalted using PD spin trap G25 (GE Healthcare) eluting in water.

A 10 mg/ml s-DHB MALDI matrix solution was prepared in 0.1%TFA dissolved in $H_2O$:ACN (50:50, v/v). 2μl of samples solution (antibodies or ADCs) were deposited on MALDI target using a double layer sample deposition method. The mass spectra were acquired in a mass to charge range starting from 50 kDa to 180 kDa.

The mass difference between unconjugated and conjugated antibodies was used to determine the DAR.

The antibodies Trastuzumab, Cetuximab, Fab Erb3 and Vectibix were used for the conjugation reactions under the experimental conditions described above. ADCs with DAR ranging from 2 to 12 were obtained.

The same conditions were used in the conjugation of payloads to anti-ErbB3 Fab except for the mAb: drug ratio that was 1:200, instead of 1:20. The product obtained showed a maximum of four drug conjugated (DAR=4).

MALDI mass spectra of the obtained products are reported in **Figures 1-3a.**

**2) Methods for the preparation of ADCs through conjugation of drugs to the unpaired cysteine residues using payload maleimide**

Antibodies were buffer exchanged using a 10 kDa cutoff dialysis membrane to yield antibodies solution in PBS pH 7.4 and to remove interfering preservative. The concentration after dialysis was determined measuring the $OD_{280}$ and the absorbance reading of the sample was divided by 1.36.

An 1 mM TCEP-HCl stock solution was prepared in water and 5-fold molar excess was added to the antibody solutions. The reactions were maintained at 37°C for 2 hours with gentle continuous mixing. The reactions was cooled.

Since TCEP is a thiol-free compound, removing the excess of the reducing was not necessary and a 20-fold molar excess of a 10 mM Kad-HDAC-maleimide stock solution prepared in DMSO, was added to each one of reduced antibody solutions.

Reactions were incubated at room temperature, with gentle continuous mixing and after 1 hour they were quenched adding a 20mM cysteine acqueous solution.

The final products were then dialyzed extensively in PBS overnight at 4°C using a 10 kDa cutoff membrane in order to remove the excess of unreacted payload.

**DARs** were determined by hydrophobic chromatography, using an MabPac HIC-Butyl column 100mmx 4.6mm, 5μm (Thermo Fisher Scientific). The mobile phase A was 1.5 ammonium sulfate, 50mM sodium phosphate, pH 7 and isopropanol (95:5; v/v), and the mobile phase B was 50mM sodium phosphate, pH 7 and isopropanol (80:20; v/v).

The mobile phase A was maintained at 100% for 1 minute after the injection and then the mobile phase B was increased to 100% in 30 minutes and hold for 5 minutes.

The UV profiles were registered at 220 and 280 nm. All antibodies, such as Trastuzumab and Cetuximab, according to the present invention, were prepared by a process which involve conjugation reactions under the experimental conditions described above. ADCs with an average DAR ranging from 3.5 to 4.6 were obtained.

The same conditions were used in the conjugation of payloads to Fab ErbB3, except for the mAb: TCEP and mAb: drug ratio that were 1:50 and 1:200 respectively, instead of 1:5 and 1:20. The product obtained showed a maximum of three drugs conjugated (DAR=3).

Examples of the obtained products were reported in the following figures. The DAR were calculated considering the peak areas and applying the following formula:

$$DAR=\frac{\sum_{0}^{8} nAreaDARn}{\sum_{0}^{8} AreaDARn}$$

HIC chromatograms of Cetuximab and Trastuzumab and their cysteines conjugated forms with Kad-HDAC-maleimide in **Figures 4 and 5.**

**3) ADCs bind to ErbB1 and ErbB2 receptors on tumor cells**

Binding of the ADCs to ErbB1- and ErbB2-expressing tumor cells was confirmed by FACS analysis. Various tumor cell lines with different levels of EGFR and ErbB2 expression, including lung (A549, H1975), breast (SKBR3), colon (LS174T), ovarian (SKOV3), pancreas (CAPAN1 and MIAPACA-2) and stomach (N87) carcinoma cell lines, were used in the experiments. Cell pellets were incubated 1 hour, at 4°C, with 10 μg/mL antibodies or ADCs in PBS and then, after two washings in PBS, stained 1 hour, at 4°C, with mouse anti-human FITC-conjugated IgGs (BD Pharmingen). After two further washings, propidium iodide (PI) was added and cell-associated fluorescence analysis was performed by means of a FACScalibur (Becton Dickinson). All the compounds showed to bind to receptors in a comparable manner to that of the free antibodies, Cetuximab and Trastuzumab **(see Table 2 and Figures 6 and 7).**

**Table 2. ADC binding to ErbB1 and ErbB2 receptors and internalization in tumor cells**

| **ADCs** | | | | |
|---|---|---|---|---|
| **Code number** | **Short name** | **DAR** | **Payload** | **Drug** |
| ST8154AA1 | Cet(Lys)-Kad-HDAC | 5.0-9.0 | ST8128AA1 | ST7464AA1 |
| ST8155AA1 | Cet(Lys)-Ad-HDAC | 6.1-6.2 | ST8132AA1 | ST7464AA1 |
| ST8176AA1 | Tras(Cys)-Kad-HDAC | 4.1-4.5 | ST8152AA1 | ST7464AA1 |
| ST8177AA1 | Cet(Cys)-Kad-HDAC | 3.2-4.6 | ST8152AA1 | ST7464AA1 |
| ST8178AA1 | Tras(Lys)-Kad-HDAC | 7.9 | ST8128AA1 | ST7464AA1 |
| ST8179AA1 | Tras(Lys)-Ad-HDAC | 6.3 | ST8132AA1 | ST7464AA1 |

Cet means Cetuximab; Tras is Trastuzumab; cys is binding to cysteine; lys is binding to lysine; Kad is a not cleavable linker; Ad is a cleavable linker and HDAC means ST7464AA1.

**Table 2**

| **Code number** | **Short name** | **DAR** | **Binding (FACS analysis)** | **Internalization (HCS Imaging)** |
|---|---|---|---|---|
| ST8154AA1 | Cet(Lys)-Kad-HDAC | 5.0-9.0 | ++ | ++ |
| ST8155AA1 | Cet(Lys)-Ad-HDAC | 6.1-6.2 | ++ | ++ |
| ST8176AA1 | Tras(Cys)-Kad-HDAC | 4.1-4.5 | ++ | ++ |
| ST8177AA1 | Cet(Cys)-Kad-HDAC | 3.2-4.6 | ++ | ++ |
| ST8178AA1 | Tras(Lys)-Kad-HDAC | 7.9 | ++ | ++ |
| ST8179AA1 | Tras(Lys)-Ad-HDAC | 6.3 | ++ | ++ |

Cet means Cetuximab; Tras is Trastuzumab; cys is binding to cysteine; lys is binding to lysine; Kad is a not

cleavable linker; Ad is a cleavable linker and HDAC means ST7464AA1; ++ is a score representing a high extent of binding or internalization.

**4) ADCs are internalized by tumor cells as measured by HCS fluorescence imaging**

Ability of ADCs to internalize within tumor cells, following their binding to cognate receptors, was assessed by means of HCS fluorescence imaging, through Operetta system (Perkin Elmer). Several tumor cell types, including lung (A549, H1975), breast (SKBR3), colon (LS174T), ovarian (SKOV3), pancreas (CAPAN1) and stomach (N87) carcinoma cell lines, were tested. Cells were seeded in 96-well microtiter plates (0.5-1x$10^4$/well) and then incubated with 5 $\mu$g/mL antibodies in culture medium, for 3 hours at 37°C. Following two washings, cells were then fixed with 4% formaldehyde in PBS, permeabilized with PBS 0.2% Tween-20 (PBS-T) and blocked with 2% BSA in PBS-T, and ultimately stained with FITC-conjugated mouse anti-human IgG (BD Pharmingen). Fluorescence was acquired and analyzed by means of the High Content Screening (HCS) system Operetta (Perkin Elmer). Cells were counterstained with Draq5 dye (Cell Signaling).

All ADCs bound to their cognate receptors and internalized by the target cells in a comparable manner to that of their native counterpart antibodies, Cetuximab and Trastuzumab **(see Table 2 and Figures 8 and 9).**

**5) ADCs bind with high affinity to ErbB1 and ErbB2 recombinant proteins**

Immunoreactivity of ADCs was tested by antigen-specific ELISA. Briefly, Immuno MAXISORP 96-well plates (Nunc) were coated overnight at 4°C with 50 ng/well of recombinant human EGF-R/ErbB1 Fc chimera (R&D) or recombinant Human ErbB2/HER2 protein (Sino Biological Inc.). After washing with PBS/0.1 % Tween (PT) solution, plates were blocked 2 hours at room temperature (RT) with PT solution containing 1% BSA (PTB), and then incubated with serial dilutions of antibodies, 1 h at room temperature. After additional washings, anti-human K light chain horseradish peroxidase (HRP)-conjugated antibody (Sigma Aldrich), diluted 1:1,000 in blocking solution, was added 1 h at room temperature. Following four washings with PT solution, 200 $\mu$L/well TMB substrate (Sigma Aldrich) were added and plates were incubated 30 min at 37°C. The reaction was blocked by adding 100 $\mu$L/well of 0.5M H2SO4 solution, and optical density at 450 nm measured by ELISA spectrophotometer.

All tested ADCs showed to react with their specific receptor with potency comparable to that of native antibodies Cetuximab and Trastuzumab **(Figures 10 A, B).**

The binding affinity of cetuximab and cetuximab-Ad-HDACi (ST8155AA1), cetuximab-Kad-HDACi (ST8154AA1) ADCs to EGFR/ErbB1 Fc chimera protein was measured by means of surface plasmon resonance (SPR) analysis on a Biacore T200 biosensor (GE). Briefly, EGFR/ErbB1 Fc chimera protein was coupled to a research-grade carboxymethylated dextran sensor chip (CM5, Biacore) using the amine coupling kit supplied by the manufacturer. Kinetic analyses were performed employing the single cycle kinetics assay, in order to avoid the extensive use of the regeneration procedure that is detrimental to the ligand (regeneration turned out to be necessary, since the antibodies did not dissociate at the end of each cycle). Antibodies and ADCs were injected at a concentration range of 6.25-100 nM in PBS running buffer at pH 7.2. The compounds were injected from low to high concentration with 180 s contact time and 600 s dissociation time in between. Injections were performed at 25 °C with a flow rate of 30 $\mu$L/min. Sensor chip surface was regenerated with 10 mM glycine/HCl pH 2.5 for 12-20 s. All the experiments were performed in duplicate. The reported kon and koff values are the average of values arising from the two experiments. KD= koff/kon **(see Table 3).**

**Table 3. Biacore analysis for cet-lys-ad-HDACi (ST8155AA1) and cet-lys-kad-HDACi (ST8154AA1)**

| | Kon (1/Ms) | Koff (1/s) | KD (M) |
|---|---|---|---|
| **Cetuximab** | $1.59x10^5$ | $4.54x10^{-4}$ | $2.85x10^{-9}$ |
| **ST8155AA1** | $6.82x10^4$ | $3.51x10^{-4}$ | $5.15x10^{-9}$ |

| | Kon (1/Ms) | Koff (1/s) | KD (M) |
|---|---|---|---|
| **Cetuximab** | $1.59x10^5$ | $4.54x10^{-4}$ | $2.85x10^{-9}$ |
| **ST8154AA1** | $1.77x10^5$ | $4.24x10^{-4}$ | $2.40x10^{-9}$ |

**6) Nebulized ADCs preserve their integrity**

Nebulization has been recently shown to be a promising delivery method for mAbs in respiratory diseases, representing a non-invasive method suitable for targeting drugs directly to the lungs, limiting the exposure of secondary organs.

According to this premise, the present inventors sought to assess by HPLC analysis the recovery and integrity of ADCs following nebulization. Briefly, 300 μg/mL solutions (in PBS) of ADCs and their parental antibodies, cetuximab and trastuzumab, were nebulized for 5 minutes through a conventional jet nebuliser (AirFamily system, Pic indolor). Nebulized drugs were then collected by conveying the mist in falcon tubes and 100 μL of condensed solution was analysed by SEC-HPLC (TSKgel G3000 SWXL column, TOSOH Bioscience) in comparison to pre-nebulized samples.

Percentage of recovery after nebulization was calculated by measuring the area of each relative peak with respect to pre-nebulized samples, and ranged from 50% to 30% and from 40% to 20% for ADCs in the case of cetuximab-based and trastuzumab-based ADCs, respectively. Integrity and aggregation incidence were also assessed for each nebulized ADC, according to the profile of each chromatogram, and compared to those of not-nebulized samples **(see Table 4).**

**Table 4. Nebulization of ADCs in percentage measured by HPLC**

| Code number | Short name | | Integrity (%) | Aggregates (%) | Degrades (%) | Recover of principal peak (%) |
|---|---|---|---|---|---|---|
| | Cetuximab | Pre neb | 100 | 0 | 0 | |
| | | neb | 100 | 0 | 0 | 72 |
| ST8154AA1 | Cet(Lys)-Kad-HDAC | Pre neb | 98 | 2 | 0 | |
| | | neb | 76 | 24 | 0 | 47 |
| ST8155AA1 | Cet(Lys)-Ad-HDAC | Pre neb | 95 | 4 | 1 | |
| | | neb | 69 | 30 | 1 | 33 |

(continued)

| Code number | Short name | | Integrity (%) | Aggregates (%) | Degrades (%) | Recover of principal peak (%) |
|---|---|---|---|---|---|---|
| ST8177AA1 | Cet(Cys)-Kad-HDAC | Pre neb | 84 | 1 | 15 | |
| | | neb | 82 | 5 | 13 | 30 |
| | Trastuzumab | Pre neb | 100 | 0 | 0 | |
| | | neb | 100 | 0 | 0 | 58 |
| ST8178AA1 | Tras(Lys)-Kad-HDAC | Pre neb | 98 | 2 | 0 | |
| | | neb | 85 | 15 | 0 | 20 |
| ST8179AA1 | Tras(Lys)-Ad-HDAC | Pre neb | 97 | 3 | 0 | |
| | | neb | 76 | 24 | 0 | 27 |
| ST8176AA1 | Tras(Cys)-Kad-HDAC | Pre neb | 83 | 5 | 12 | |
| | | neb | 76 | 11 | 13 | 21 |

Cet means Cetuximab; Tras is Trastuzumab; cys is binding to cysteine; lys is binding to lysine; Kad is a not cleavable linker; Ad is a cleavable linker and HDAC means ST7464AA1.

**Example 2. ADCs inhibit tumor cell proliferation**

The effect of ADCs on **cell proliferation** was evaluated on two lung adenocarcinoma cell lines (NCl-H1975 and Calu-3). More in details, NCl-H1975 tumor cells are characterized by overexpression of double-mutant (L858R, T790M) *ErbB1* gene, whereas Calu-3 express the wild type form of EGFR but mutant K-Ras (G13D) gene, as well as mutant TP53 and CDKN2A genes.

Cells were seeded (at 3.000-5.000 cells/well) into 96-well plates in complete culture medium and then incubated for 6 days, in quadruplicate, with scalar concentrations of ADCs, ranging from 500 to 6.25 nM. Inhibition of cell proliferation was measured by CellTiter-Glo Luminescent Cell Viability Assay (Promega), through a Veritas luminometer (Promega). Data were expressed as the average ($\pm$ SD) of percentage inhibition of two independent experiments. The $IC_{50}$ values were ultimately calculated by using the GraphPad Prism 5.0 software. Results showed that ADC ST8154AA1 significantly inhibited tumor cell proliferation of both cell lines (with $IC_{50}$ values of 250 nM and 450 nM, on NCl-H1975 and Calu-3 cells, respectively), as compared to cetuximab alone that, instead, was not effective (IC50>500 nM) **(Figures 11-12).**

**Example 3. Activity of ADCs on acetylation of histone H3 and $\alpha$-Tubulin in tumor cells**

The effect of ADCs on acetylation of typical HDAC substrates, i.e. histone H3 and $\alpha$-tubulin protein, was assessed by means of HCS fluorescence imaging on different tumor cells. Several tumor cell lines expressing various levels of EGFR and HER2 receptors, including lung (A549, H1975), breast (SKBR3), colon (LS174T), ovarian (SKOV3), pancreas (CAPAN1 and MIAPACA-2) and stomach (N87) carcinoma cell lines, were tested. Cells were seeded in 96-well microtiter plates (0.5-1x$10^4$ /well) in complete culture medium and, the day after, incubated with 5 $\mu$g/mL antibodies or ADCs, for 3 or 24 hours at 37°C. Following two washings with PBS, cells were fixed with 4% formaldehyde in PBS, permeabilized with PBS 0.2%Tween-20 (PBS-T) and blocked with 2% BSA in PBS-T. Mouse anti-acetylated-$\alpha$-tubulin IgG (clone 6-11B-1, from Sigma Aldrich) or rabbit anti-acetylated-histone H3 IgG (from Active Motif) were then added in PBS-T, and cells were incubated 1 hour at room temperature. After two washings with PBS, cells were ultimately stained for 1 additional hour with FITC-conjugated goat anti-mouse or anti-rabbit IgG (BD Pharmingen), according to the primary antibody used. Fluorescence was acquired and analyzed by means of the High Content Screening (HCS) system Operetta (Perkin Elmer). Cells were counterstained with Draq5 dye (Cell Signaling).

All tested ADCs induced in all tested tumor cell lines a relevant increase in the acetylation level of both $\alpha$-tubulin and histone H3, as a result of direct enzymatic inhibition of HDAC6 and class I HDACs, respectively (Figures **13-16**).

This result was coherent with the ability of ADCs to be internalized and degraded to release the ST7464AA1 moiety.

Increased acetylation of α-tubulin and histone H4 was observed by means of Western Blot analysis on protein lysates of different tumor cells. Briefly, the day before the experiment tumor cells were seeded into 6-well dishes in complete culture medium. Cells were then treated, for 3 hours at 37°C, with 20 μg/mL test antibodies. After treatment, cells were washed twice with ice-cold PBS and then whole cell lysate was prepared by incubation, 10 min on ice, with 1X Lysis Buffer (Cell Signaling) supplemented with protease inhibitors. Cell lysates were subjected to sonication prior to centrifugation at 14.000 x g, for 10 min at 4°C, to remove cell debris. The protein content was determined by the classical colorimetric Bradford method (Coomassie Bradford protein assay kit; Pierce), according to the manufacturer's instruction. To assess the extent of acetylation, equal amounts of proteins for each sample were resolved by SDS-PAGE and transferred onto a nitrocellulose membrane (Hybond-C extra, Amersham-GE Healthcare). Molecular weights were estimated based upon the relative migration with molecular weight protein markers (Prestained Kaleidoscope Standards; Bio-Rad). Nonspecific binding sites were then blocked by incubation of the membranes with 5% nonfat dry milk in TBS, overnight at 4 °C. Specific primary antibodies (rabbit anti-acetyl-histone H4 antibody, from Santa Cruz; mouse anti-histone H4 monoclonal antibody, from Cell Signaling; mouse anti-acetylated α-tubulin monoclonal antibody, from Sigma Aldrich, rabbit anti-α-tubulin antibody, from abcam), were added to the membranes at the optimal dilution in 5% nonfat dry milk/TBST overnight at 4 °C. Following four washes in TBST, membranes were incubated for 1 h with HRP-conjugated secondary antibodies in 5% nonfat dry milk/TBST. Immunoreactive bands were finally visualized by enhanced chemiluminescence with the ECLplus Western blotting detection reagent (GE Healthcare) and analyzed by a phosphoimaging system (STORM, Molecular Dynamics). Representative blots are shown in **Figure 17.**

**Example 4. Cet-lys-kad-HDACi (ST8154AA1) and Cet-ad-kad-HDACi (ST8155AA1) inhibit in vivo tumor growth of NCI-H1975 non-small cell lung cancer**

Nude Nu/Nu female mice, from Charles River, Italy, were given a single subcutaneous injection of $3x10^6$ NCI-H1975 non-small cell lung carcinoma cells suspended in 100 μL cell culture medium RPMI supplemented with 10% FBS. Cetuximab and cetuximab-lys-kad-HDACi or cetuximab-lys-ad-HDACi were given intraperitoneally every 4 days for 4 days (q4dx4) at a dose of 50 mg/10 mL/kg. Tumor growth was measured with a caliper and tumor volume was calculated using the formula length (mm) x width2 (mm)/2. The tumor inhibition was calculated according to the equation: %TVI=100-(mean tumor volume of treated group / mean tumor volume of control group) x 100. Toxicity was evaluated on the basis of the body weight reduction.

Animals were euthanized by CO2 inhalation when the tumors reached a volume that hampered them.

All the procedures adopted for housing and handling of animals were in strict compliance with Italian and European guidelines for Laboratory Animal Welfare.

Results showed that the ADCs were significantly more efficacious than cetuximab (P<0.001) (**Figures 18** and **19**).

**Example 5. Cet-lys-kad-HDACi (ST8154AA1) inhibits in vivo tumor growth of A549 non-small cell lung cancer**

Nude mice were given a subcutaneous injection of $5x10^6$ A549 non-small cell lung carcinoma suspended in 100 μL cell culture medium RPMI supplemented with 10% FBS. Cetuximab and cetuximab-lys-kad-HDACi were administered intraperitoneally every 4 days for 4 days (q4dx4) at a dose of 50 mg/10 mL/kg. Tumor measurements and data as in Example 3.

Treatment showed that the cetuximab-lys-kad-HDACi was significantly more efficacious than cetuximab (P<0.05) **(Figure 20).**

**Example 6. Cet-lys-kad-HDACi (ST8154AA1) delivered by aerosol inhibits in vivo tumor growth of metastatic A549 non-small cell lung cancer**

Metastatic lung cancer was established by injecting $5x10^6$ A549-luc-C8 (A549luc) cells into the tail vein of immunodeficient SCID/beige mice. After 1 week the mice were randomized in groups of 12 and treated by whole body aerosol (by means of the AirFamily system, Pic indolor) with ADC or Cetuximab (3.5 mL of 100 μg/mL solution). Treatments were repeated according to the schedule q7dx4. Tumor bioluminescence imaging (BLI) was recorded at different time points by Xenogen IVIS Imaging System 200 (Perkin Elmer), 15 min after i.p. injection of luciferin (150 μg/mouse). The evaluation of bioluminescence showed that ADC was able to significantly inhibit tumor metastases with a higher potency in comparison with Cetuximab (p<0.01) **(Figure 21).**

**Example 7. Tras-lys-kad-HDACi (ST8178AA1) and Tras-cys-Kad-HDACi (ST8176AA1) inhibit in vivo tumor growth of SKOV-3 ovarian cancer**

Nude Nu/Nu female mice, from Charles River, Italy, were given a single subcutaneous injection of $5x10^6$ SKOV-3 ovarian carcinoma cells suspended in 100 μL cell culture medium RPMI supplemented with 10% FBS.

Mice for each experimental group were treated ip every four days for 4 treatments (q4dx4) at a dose of 15 mg/10 mL/kg, showed that the trastuzumab-lys-kad-HDACi and tras-cys-kad-HDACi were significantly more efficacious than trastuzumab (P<0.05) **(Figures 22-23).** Tumor measurements and data as in Example 3. These data suggest a double effect of the ADC in comparison with Trastuzumab on the tumor growth.

**Example 8. Anti-tumor efficacy of intraperitoneal ADCs against peritoneal cancer**

The ADCs were also evaluated against an intraperitoneal tumor such as colon carcinoma, an aggressive tumor xenograft model.

The aim of this investigation was to demonstrate that ADCs may be also used by a local administration to treat diseases like peritoneal carcinomatosis. To demonstrate such activity LS-174T colon cancer cells were injected ip. The cells were collected and washed two times with PBS. Ten million cells were suspended in 0.2 mL of EMEM medium containing 20% of MatrigelTM and injected in the peritoneum of each mouse.

All the treatments with the ADC were performed by i.p. injection at a volume of 200 μL 3 days post tumor injection according to the schedule q4dx4.

Mice were monitored for mortality daily, while weight was recorded two times per week. Animals showing signs of discomfort, distress or in moribund condition were examined by the staff veterinarian or authorized personnel and, when necessary, humanely sacrificed to minimize undue pain or suffering.

Mortality data were processed according to the most appropriate statistical analysis to determine increase life span among the treatments and to produce a Kaplan-Mayer plot. All the statistical analysis was performed using the software *GraphPad-Prism6.*

The results showed in both vehicle- and Trastuzumab-treated groups a median survival time (MST) of 37 days. By contrast the ADC trastuzumab(Cys)-Kad-HDAC revealed to significantly increase the median survival time to 50 days (P<0.05) **(Figure 24).**

Currently, the traditional treatment of peritoneal cancer consisting of systemic chemotherapy, with or without palliative surgery, shows poor effects in terms of outcome and presents pharmacological limitations in terms of poor drug distribution in the peritoneal cavity and penetration into peritoneal nodules *(*Oyais A 2014 Zentralbl Chir. 2014 141: 421-4*).*

By contrast, a recent innovative method named PIPAC (pressurized intraperitoneal aerosol chemotherapy) of local delivery has showed to enhance the efficacy of intraperitoneal chemotherapy *(*Solass W 2014 Ann Surg Oncol 21:553-9*)* and this procedure resulted to be safe, with no post-treatment renal or hepatic toxicity *(*Blanco 2014 20:2311-6*;* Solass 2013 Ann Surg Oncol 20: 3504-11*).*

Because the novel ADCs show an antitumor activity by aerosol and intraperitoneally on a local tumor, these data encourage to PIPAC use of the ADCs herein described in addition to the standard parenteral administration.

PIPAC (pressurized intraperitoneal aerosol chemotherapy) is an innovative method of local delivery that enhances the efficacy of intraperitoneal chemotherapy *(*Solass W 2014 Ann Surg Oncol 21:553-9*).*

This procedure resulted to be safe, with no post-treatment renal or hepatic toxicity *(*Blanco 2014 20:2311-6*;* Solass et al 2013 Ann Surg Oncol 20: 3504-11*).*

Differently, traditional treatment of peritoneal cancer consists of systemic chemotherapy, with or without palliative surgery, with poor effects in terms of outcome. Another problem is the pharmacological limitation in terms of poor drug distribution in the peritoneal cavity and penetration into peritoneal nodules *(*Oyais A et al 2014 Zentralbl Chir. 2014 141: 421-4*).*

Based on biochemical analysis confirming stability of ADCs herein described upon nebulization and efficacy data on intraperitoneal delivery, the PIPAC use of ADCs is also justified.

## Claims

**1.** An antibody-drug-conjugate of formula (I)

$$D\text{-}(CU)_m\text{-}(S1)_n\text{-}L\text{-}(S2)_o\text{-}(CG)_p\text{-}Ab \qquad \text{(Formula I)}$$

or a pharmaceutically acceptable salt thereof, wherein

D is a histone deacetylase inhibitor drug,
CU is a connecting unit,
S1 and S2 are spacers, which may be the same or different,
L is a linker, which can be cleavable or not cleavable,
CG is a connecting group,
Ab is an antibody or an antigen binding fragment thereof, and
m, n, o and p represent integers of 0 or 1.

**2.** The antibody-drug-conjugate according to claim 1, wherein the histone deacetylase inhibitor is selected from thiol-based histone deacetylase inhibitors, hydroxamic acid-based histone deacetylase inhibitors or benzamid-based histone deacetylase inhibitors.

**3.** The antibody-drug-conjugate according to claim 1, wherein the histone deacetylase inhibitor is ST7464AA1 having the following formula

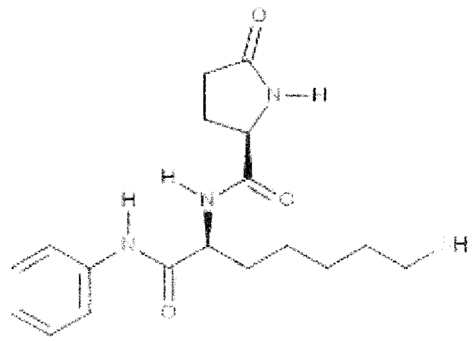

ST7464AA1
and the payload comprising D-$(CU)_m$-$(S1)_n$-L-$(S2)_o$-$(CG)_p$- is a compound selected from:

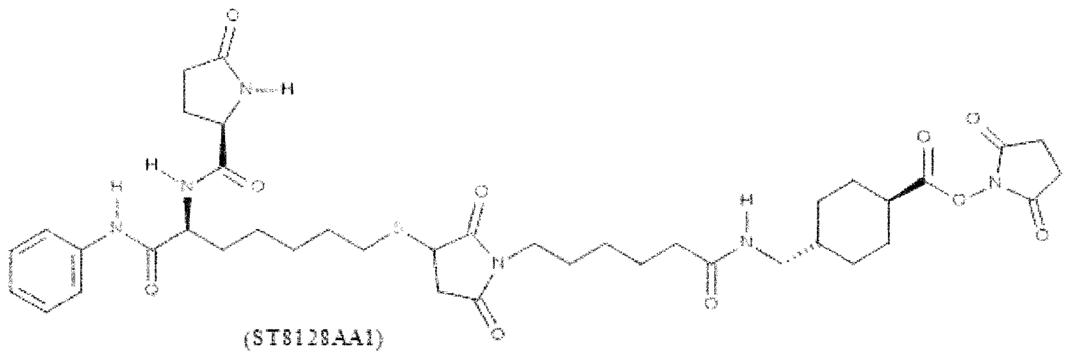

(ST8128AA1)

ST8128AA1

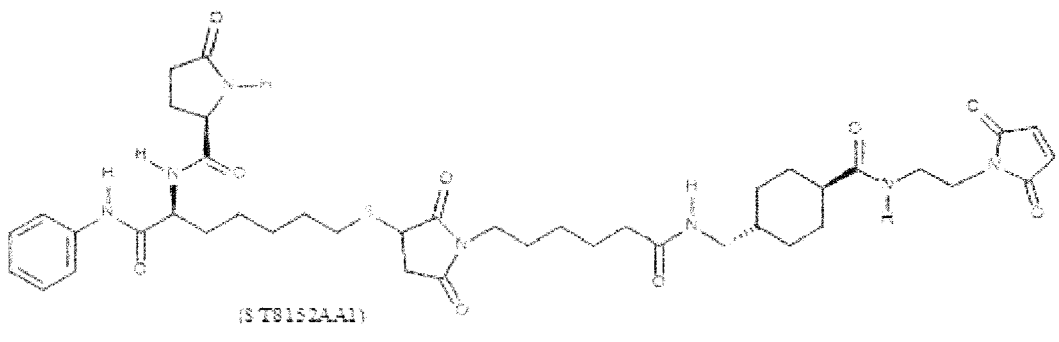

(ST8152AA1)

ST8152AA1

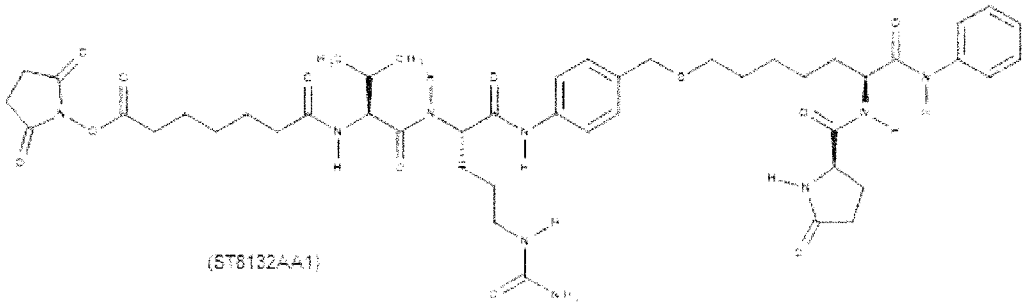

(ST8132AA1)

ST8132AA1

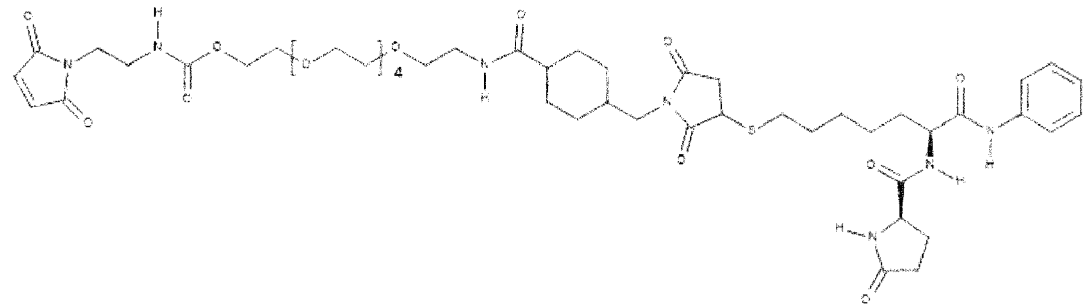

or

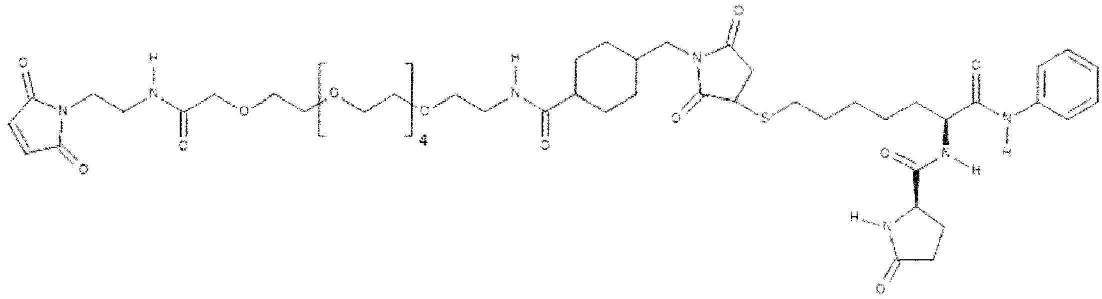

**4.** The antibody-drug-conjugate according to one of claims 1 to 3, wherein the connecting unit is succinimide or a 3,4-disubstituted succinimide or succinamic acid of the following formulae

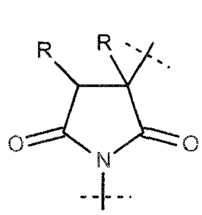

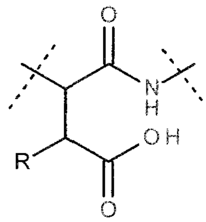

wherein R can be H or a $C_1$-$C_3$ alkyl group.

**5.** The antibody-drug-conjugate according to one of claims 1 to 4, wherein the spacer 1 is selected from alkylcarbonyl, cycloalkylcarbonyl, ω-aminoalkylcarbonyl, aminoarylcarbonyl. alkylaminocarbonyl, cycloalkylaminocarbonyl.

**6.** The antibody-drug-conjugate according to one of claims 1 to 5, wherein the linker is a cleavable linker having the formula

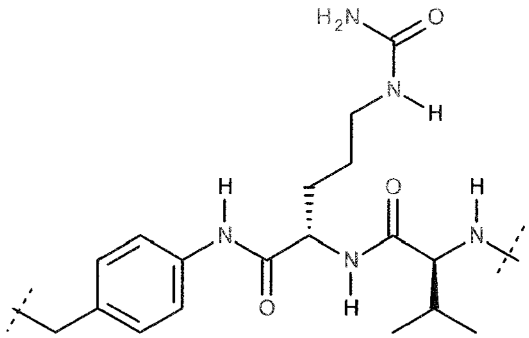

.

7. The antibody-drug-conjugate according to one of claims 1 to 5, wherein the linker is a non-cleavable linker having the formula

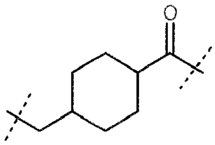
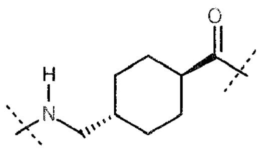
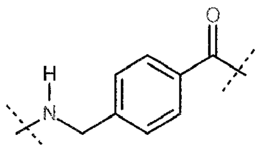
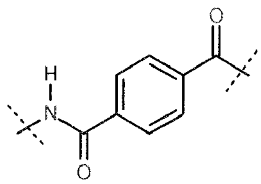

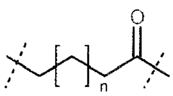
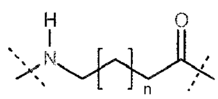
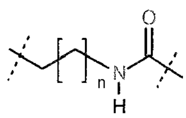
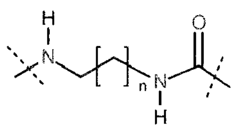

n= 0 to 4.

8. The antibody-drug-conjugate according to one of claims 1 to 7, wherein the spacer 2 is selected from

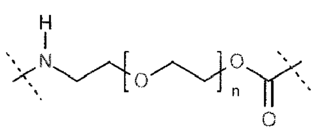 ; 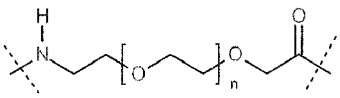
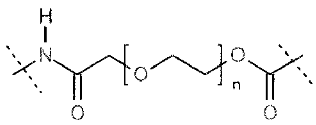

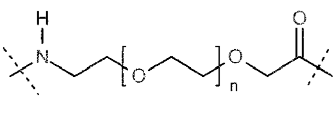 ; 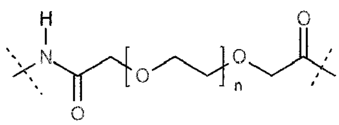 ; 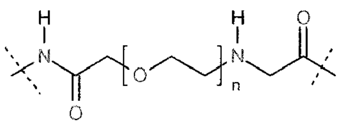 ;

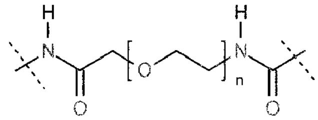
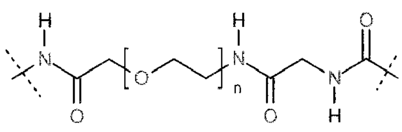

n=2 to 5.

9. The antibody-drug-conjugate according to one of claims 1 to 8, wherein the connecting group is selected from N-hydrosuccinimide (NHS) or an activated acylderivative or maleimide- or 3-methylenesuccinimide, 3,4-dibromo maleimide or {amino-carbonyl}-3-butenoic acid of the following formulae:

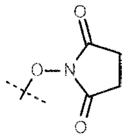 ; 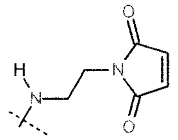 ; 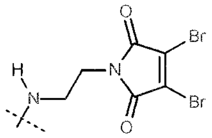 ; 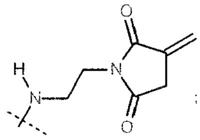 ; 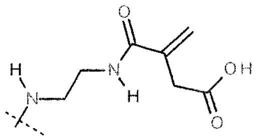 .

**10.** The antibody-drug conjugate according to one of claims 1 to 9, wherein the antibody is directed against a histone deacetylase inhibitor or against a receptor internalized by tumor cells to release a histone deacetylase inhibitor such as c-met or integrin receptors.

**11.** The antibody-drug-conjugate according to one of claims 1 to 10, wherein the antibody is an anti-EGFR family protein antibody.

**12.** The antibody-drug-conjugate according to one of claims 1 to 11, wherein the antibody is selected from Trastuzumab, Cetuximab, Bevacizumab, Panitumumab and anti-ErbB3 Fab Sigma Tau proprietary or related biosimilars.

**13.** An antibody-drug-conjugate according to one of claims 1 to 12 having a formula selected from

ST8154AA1

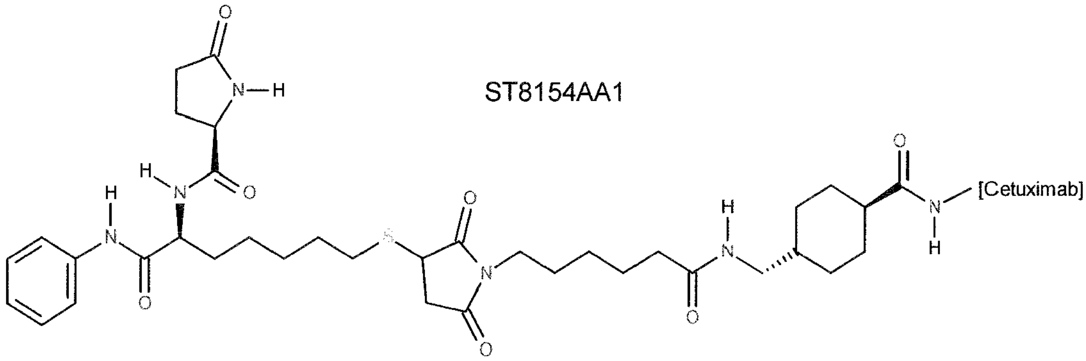

Formula (Ia)

ST8155AA1

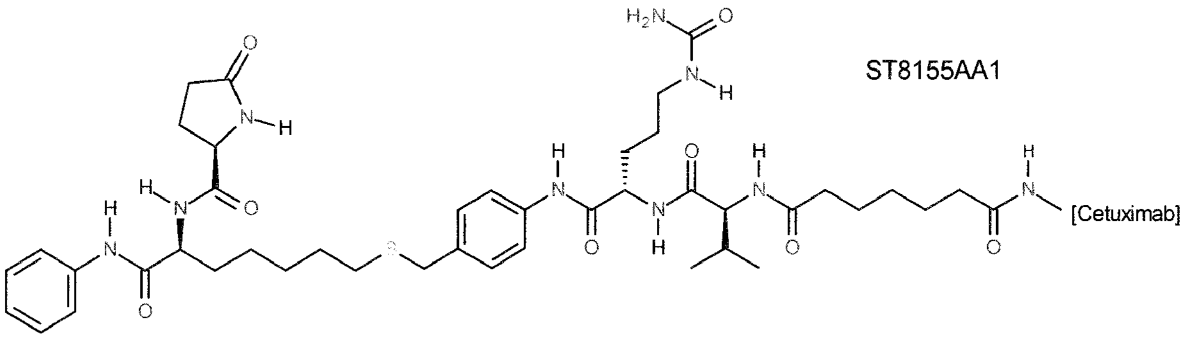

Formula (Ib)

ST8177AA1

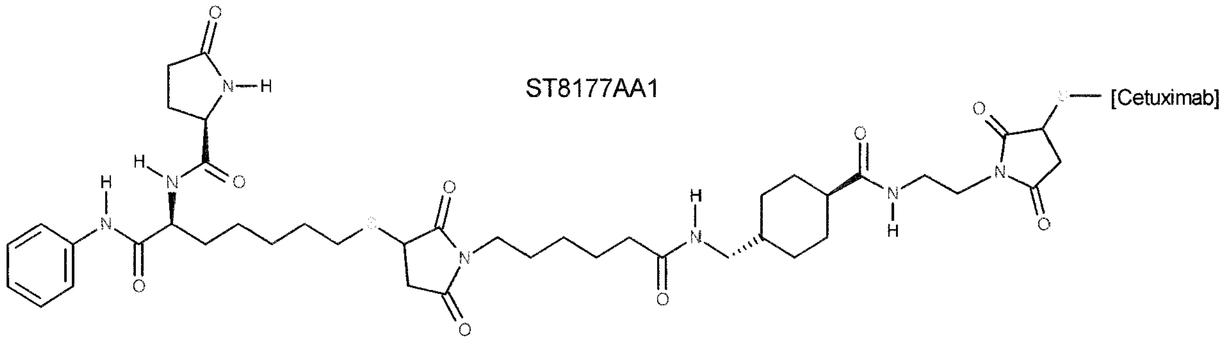

Formula (Ic)

ST8178AA1

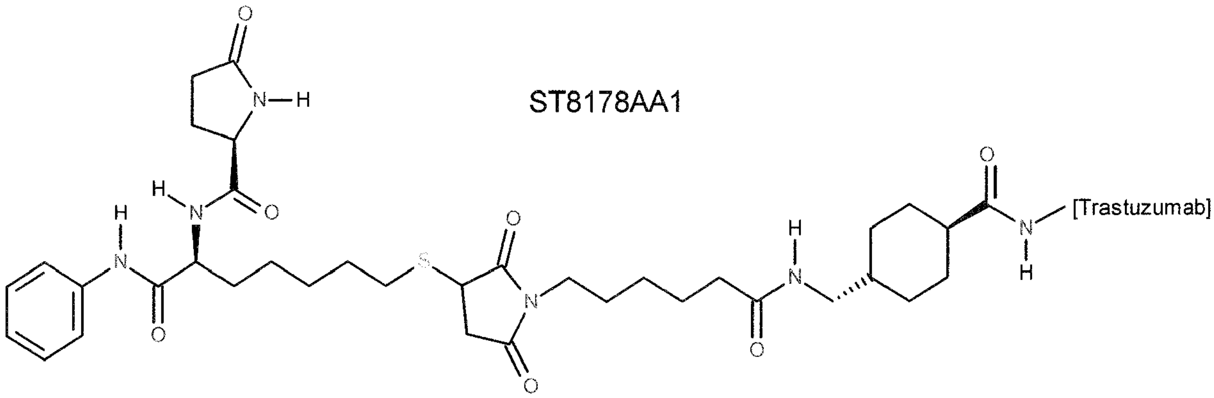

Formula (Id)

ST8178AA1

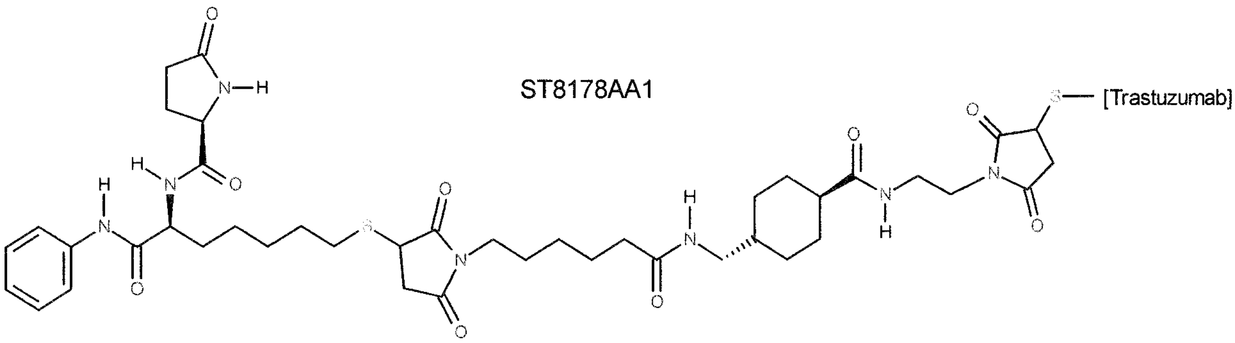

Formula (Ie)

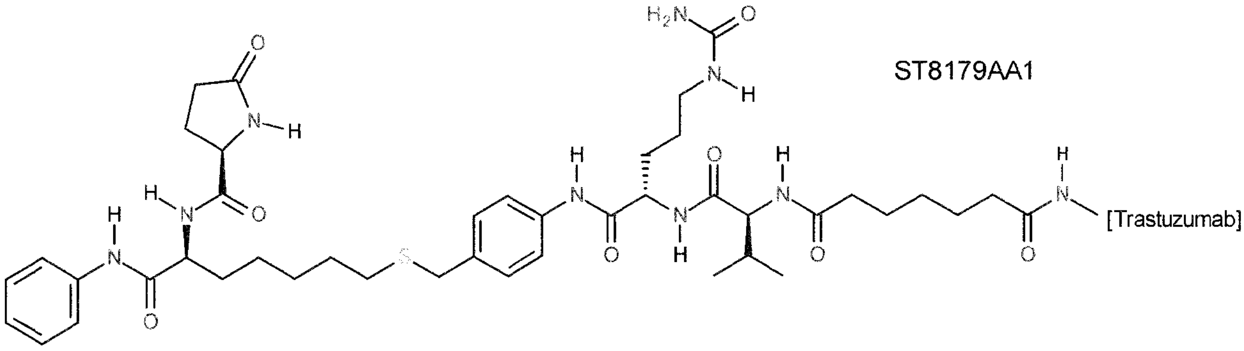

ST8179AA1

Formula (If).

**14.** The antibody-drug-conjugate according to one of claims 1 to 13 for use as a medicament.

**15.** A pharmaceutical composition comprising the antibody-drug-conjugate according to one of claims 1 to 13 together with a pharmaceutically acceptable vehicle.

**16.** An antibody-drug-conjugate according to one of claims 1 to 13 or a pharmaceutical composition according to claim 15 for use in the treatment of a cancer or a tumor expressing a receptor selected from ErbB1, ErbB2 or ErbB3.

**17.** The antibody-drug-conjugate according to one of claims 1 to 13 or the pharmaceutical composition according to claim 15 for the use of claim 16, wherein the cancer is selected from cancer of lung, peritoneum, breast, colon, brain, head and neck, endometrial cancer, cervix-endometrium cancer, renal cancer, pancreatic cancer, gastric cancer, colon cancer, appendiceal cancer, oesophageal cancer, ovarian and prostate cancer, leukemia, pseudomyxoma peritonei, liver metastases and abdominal sarcoma of non-gut tissues.

**18.** The antibody-drug-conjugate according to one of claims 1 to 13 or the pharmaceutical composition according to claim 15 for use as an adjuvant therapeutic in the treatment of HIV.

**19.** The antibody-drug-conjugate according to one of claims 1 to 13 or the pharmaceutical composition of claim 15 in a formulation suitable for local delivery by nebulization.

**20.** The antibody-drug-conjugate or the pharmaceutical composition according to claim 19 for use in the treatment of a disease associated with the lung or the peritoneum, preferably lung or peritoneal cancer, or for the treatment of cancer from ovarian, cervix-endometrium, gastric, colon, appendiceal, pseudomyxoma peritonei, pancreas, liver metastases or rare neoplasia such as abdominal sarcoma of non-gut tissues.

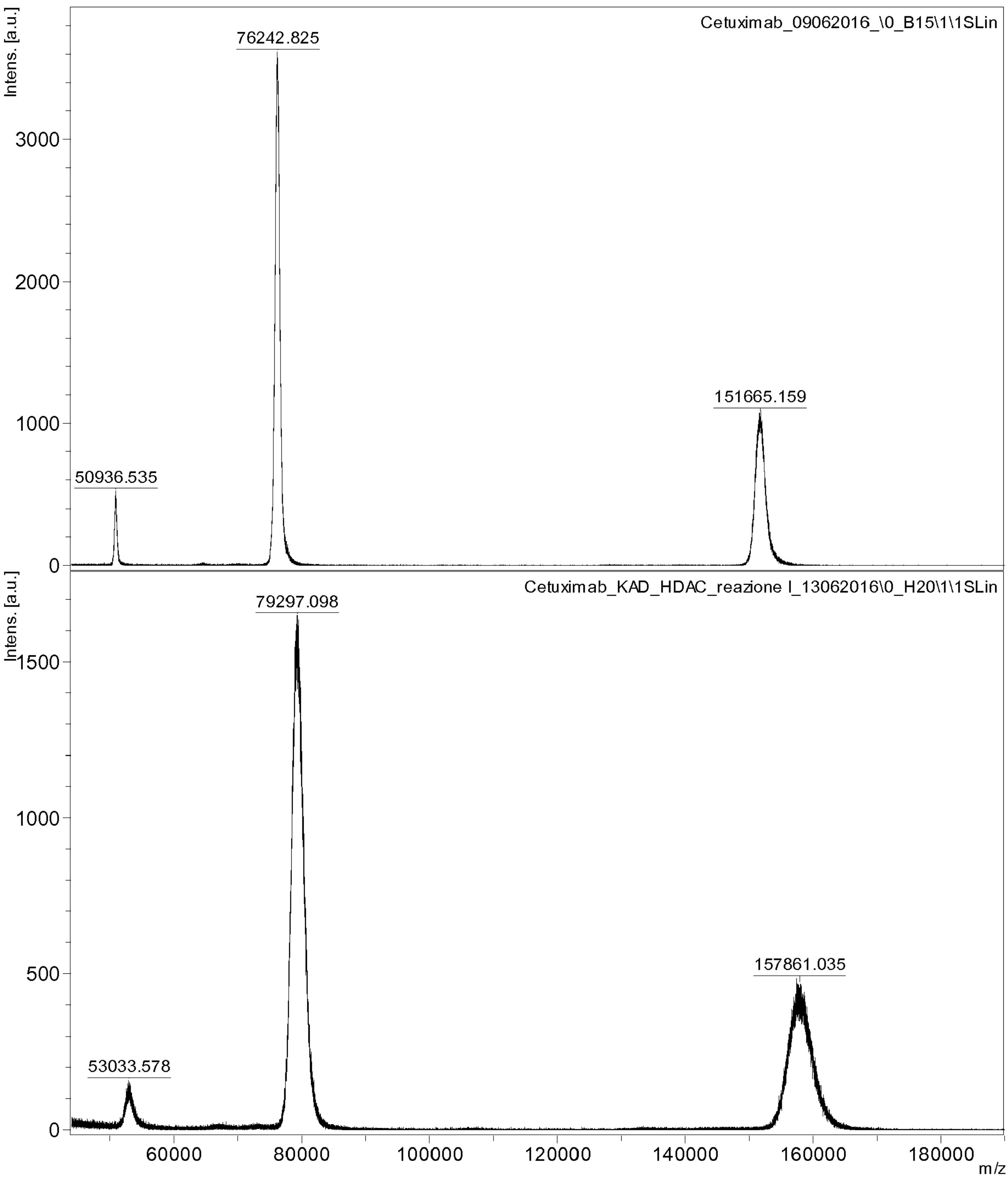
Cetuximab_09062016_\0_B15\1\1SLin
Intens. [a.u.]
76242.825
151665.159
50936.535
3000
2000
1000
0
Cetuximab_KAD_HDAC_reazione I_13062016\0_H20\1\1SLin
Intens. [a.u.]
79297.098
157861.035
53033.578
1500
1000
500
0
60000
80000
100000
120000
140000
160000
180000
m/z

Figure 1

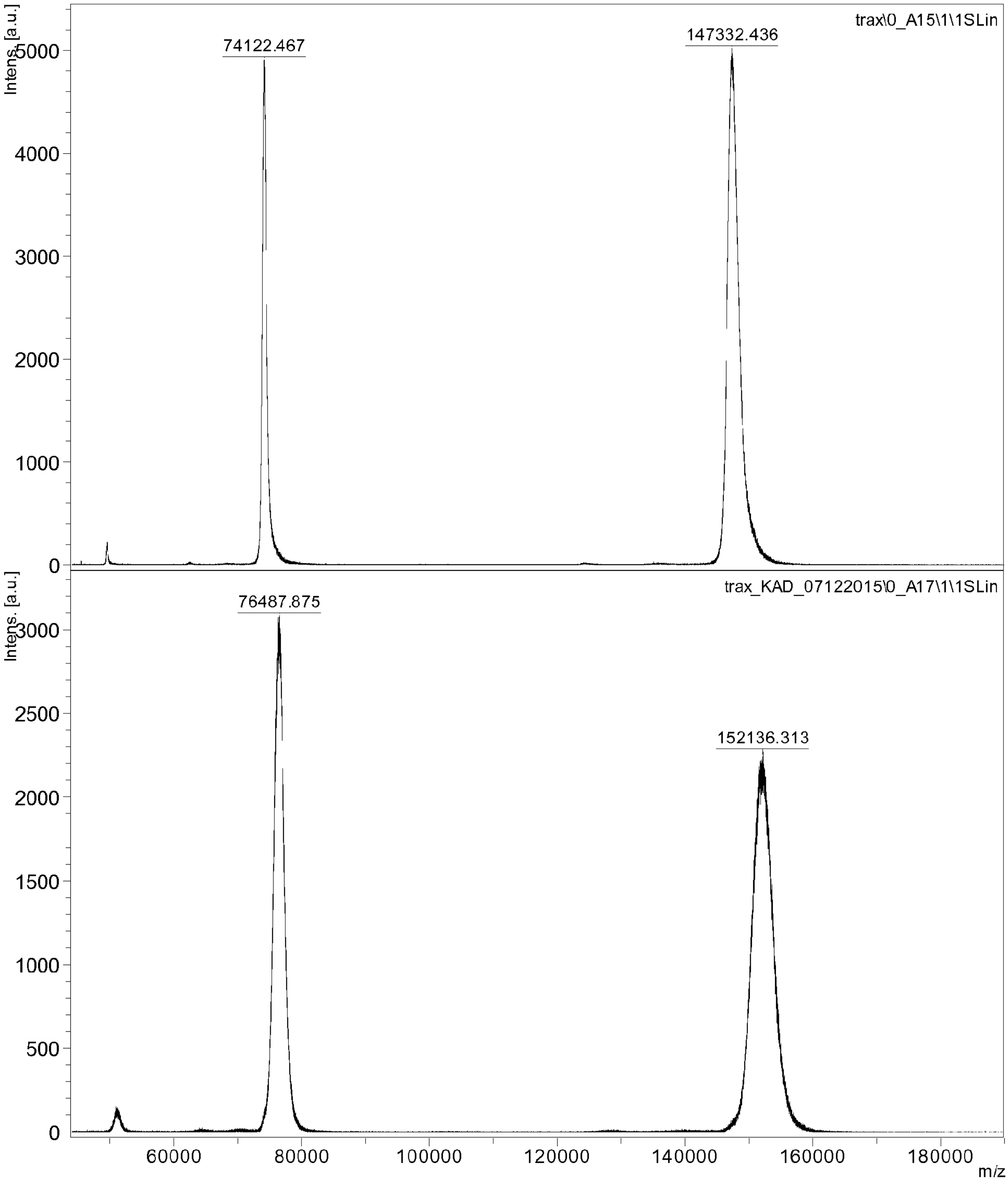
Intens. [a.u.]
trax\0_A15\1\1SLin
74122.467
147332.436
5000
4000
3000
2000
1000
0
Intens. [a.u.]
trax_KAD_07122015\0_A17\1\1SLin
76487.875
152136.313
3000
2500
2000
1500
1000
500
0
60000
80000
100000
120000
140000
160000
180000
m/z

**Figure 2**

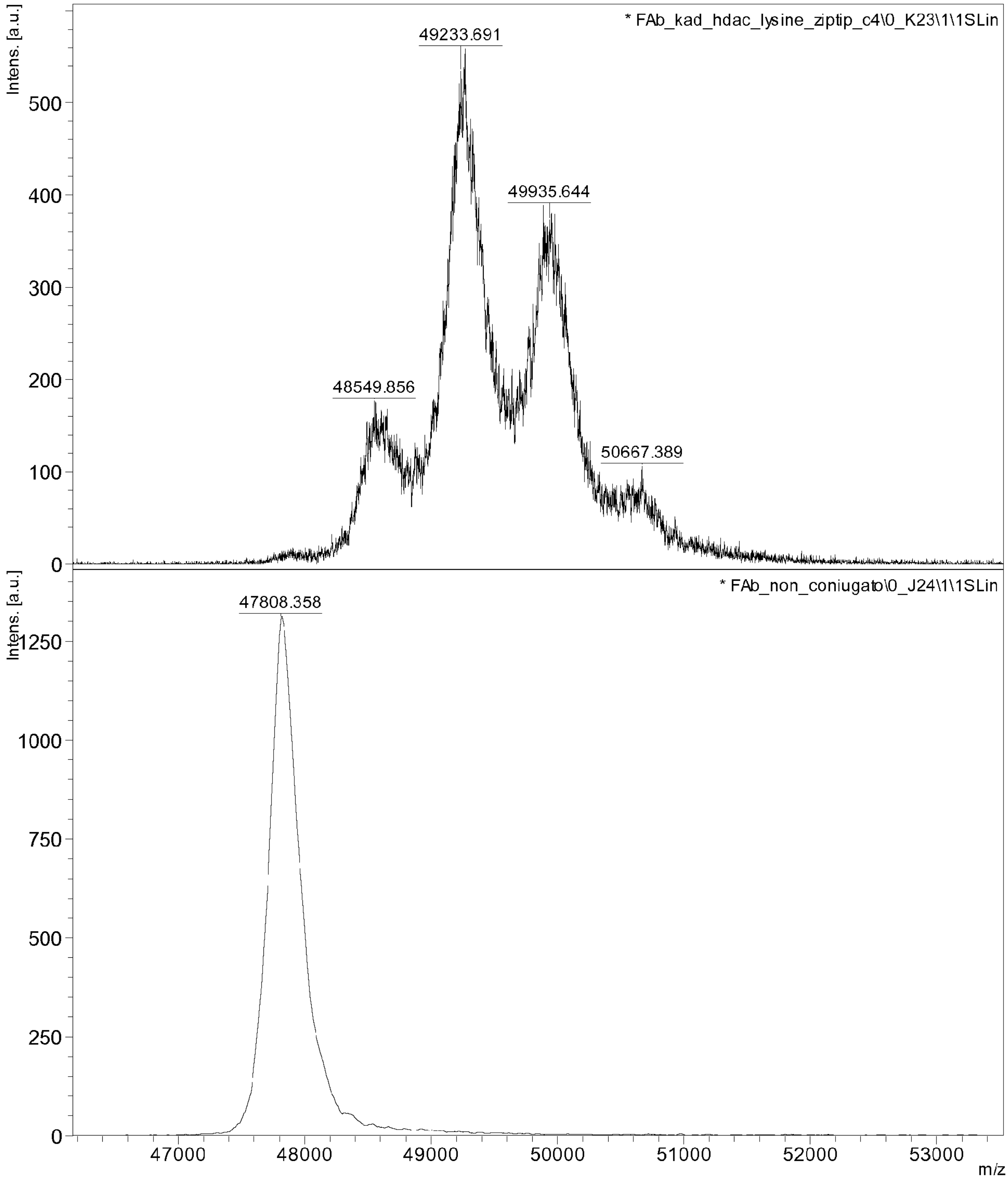
* FAb_kad_hdac_lysine_ziptip_c4\0_K23\1\1SLin
Intens. [a.u.]
49233.691
49935.644
48549.856
50667.389
500
400
300
200
100
0
* FAb_non_coniugato\0_J24\1\1SLin
Intens. [a.u.]
47808.358
1250
1000
750
500
250
0
47000
48000
49000
50000
51000
52000
53000
m/z

**Figure 3**

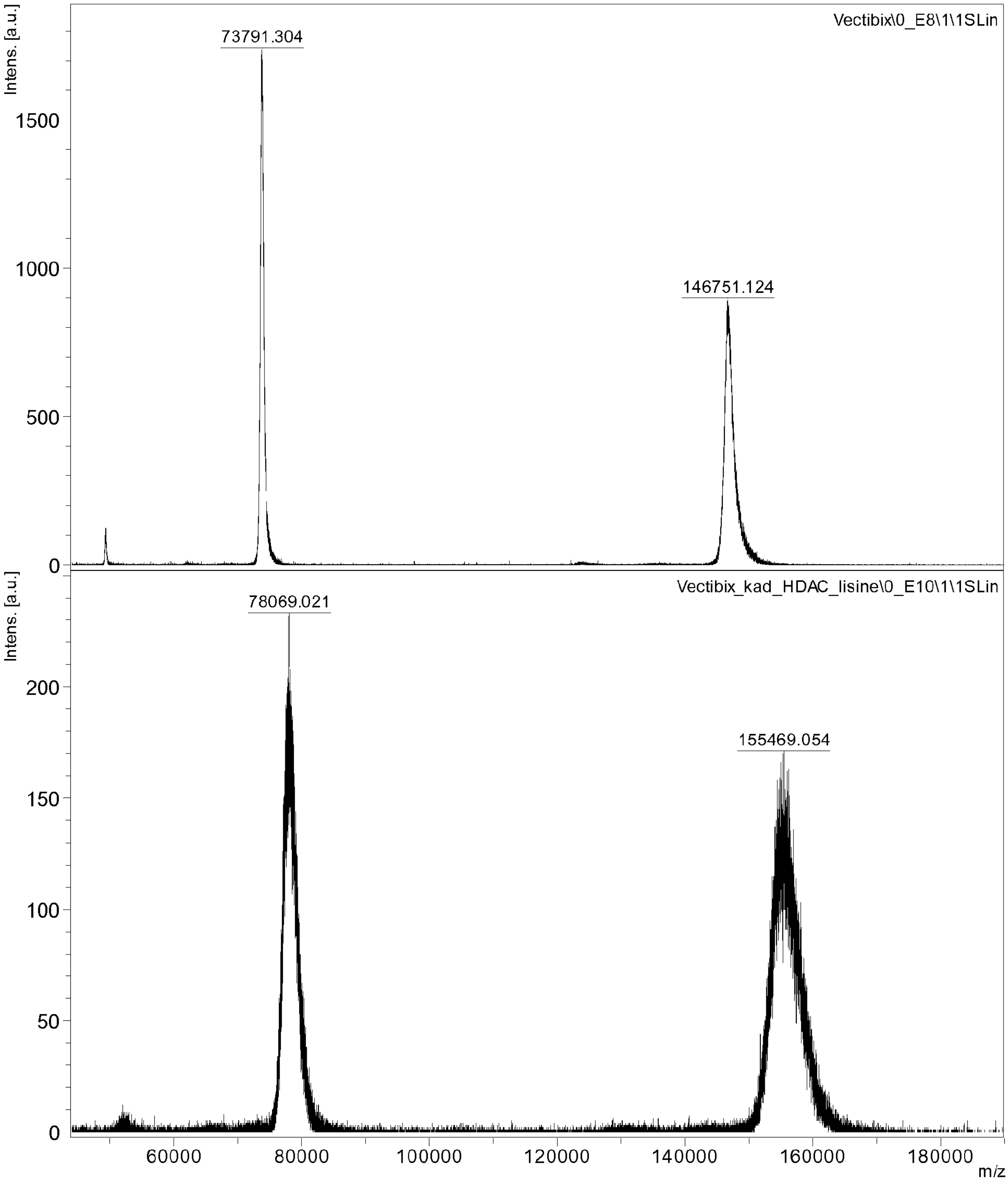
Vectibix\0_E8\1\1SLin
Intens. [a.u.]
73791.304
146751.124
1500
1000
500
0
Vectibix_kad_HDAC_lisine\0_E10\1\1SLin
Intens. [a.u.]
78069.021
155469.054
200
150
100
50
0
60000
80000
100000
120000
140000
160000
180000
m/z

Figure 3a

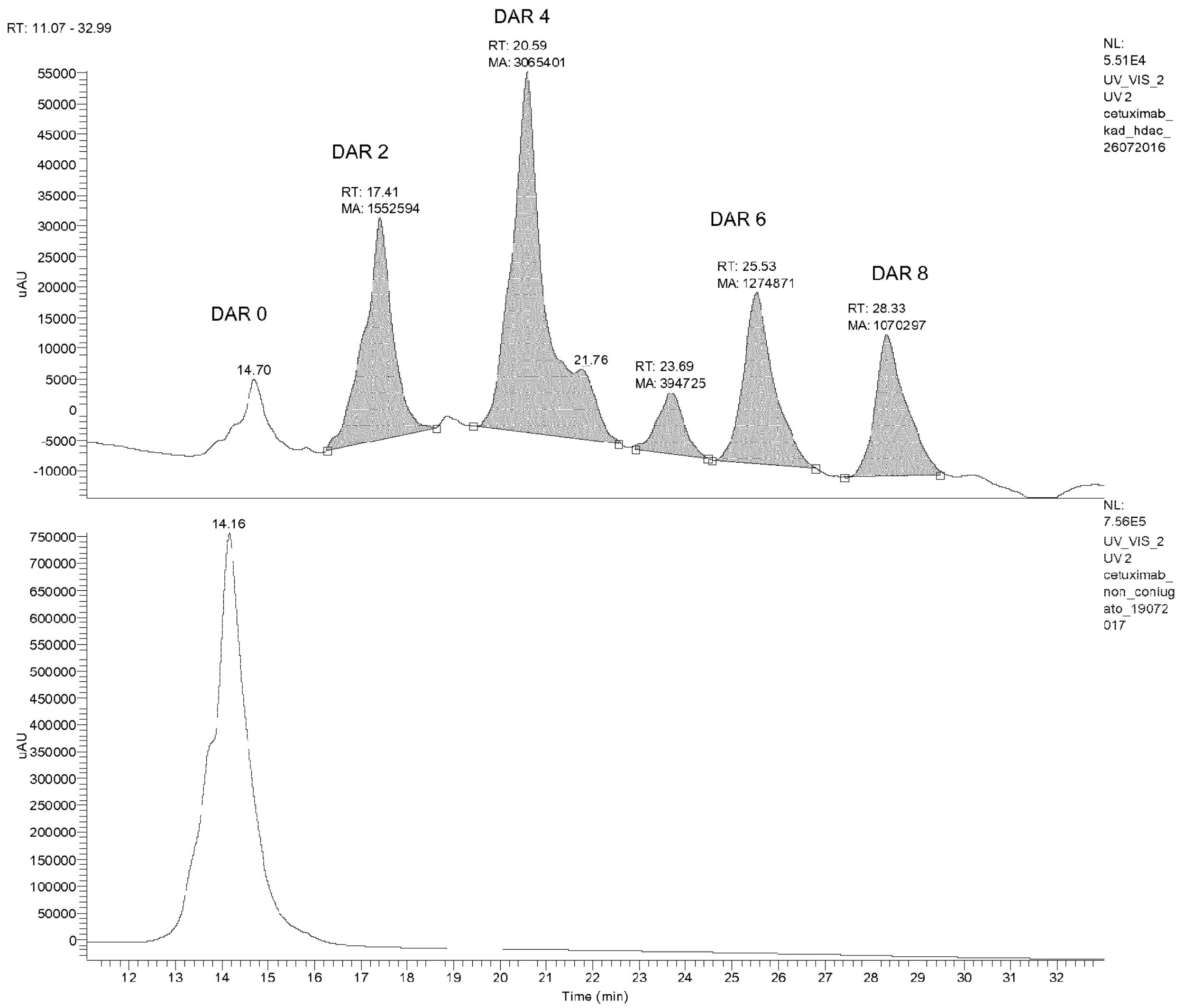
RT: 11.07 - 32.99
DAR 4
RT: 20.59
MA: 3065401
NL:
5.51E4
UV_VIS_2
UV2
cetuximab_
kad_hdac_
26072016
DAR 2
RT: 17.41
MA: 1552594
DAR 6
RT: 25.53
MA: 1274871
DAR 8
RT: 28.33
MA: 1070297
DAR 0
14.70
21.76
RT: 23.69
MA: 394725
uAU
NL:
7.56E5
UV_VIS_2
UV2
cetuximab_
non_coniug
ato_19072
017
14.16
Time (min)

Figure 4

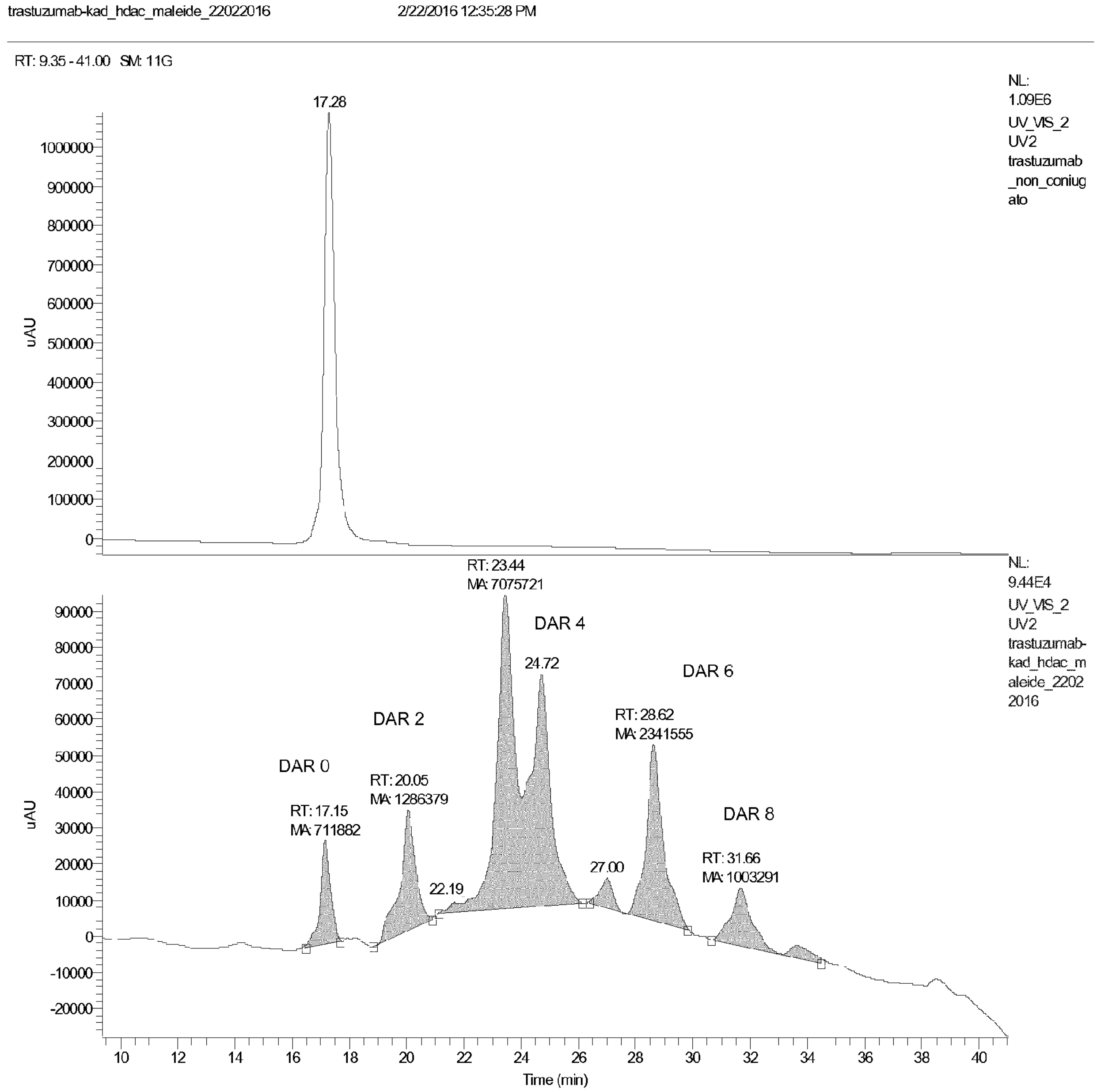
trastuzumab-kad_hdac_maleide_22022016
2/22/2016 12:35:28 PM
RT: 9.35 - 41.00 SM: 11G
NL: 1.09E6 UV_VIS_2 UV2 trastuzumab _non_coniug ato
17.28
NL: 9.44E4 UV_VIS_2 UV2 trastuzumab-kad_hdac_m aleide_2202 2016
RT: 23.44 MA: 7075721
DAR 4
24.72
DAR 6
DAR 2
RT: 28.62 MA: 2341555
DAR 0
RT: 20.05 MA: 1286379
RT: 17.15 MA: 711882
DAR 8
RT: 31.66 MA: 1003291
27.00
22.19
uAU
Time (min)

Figure 5

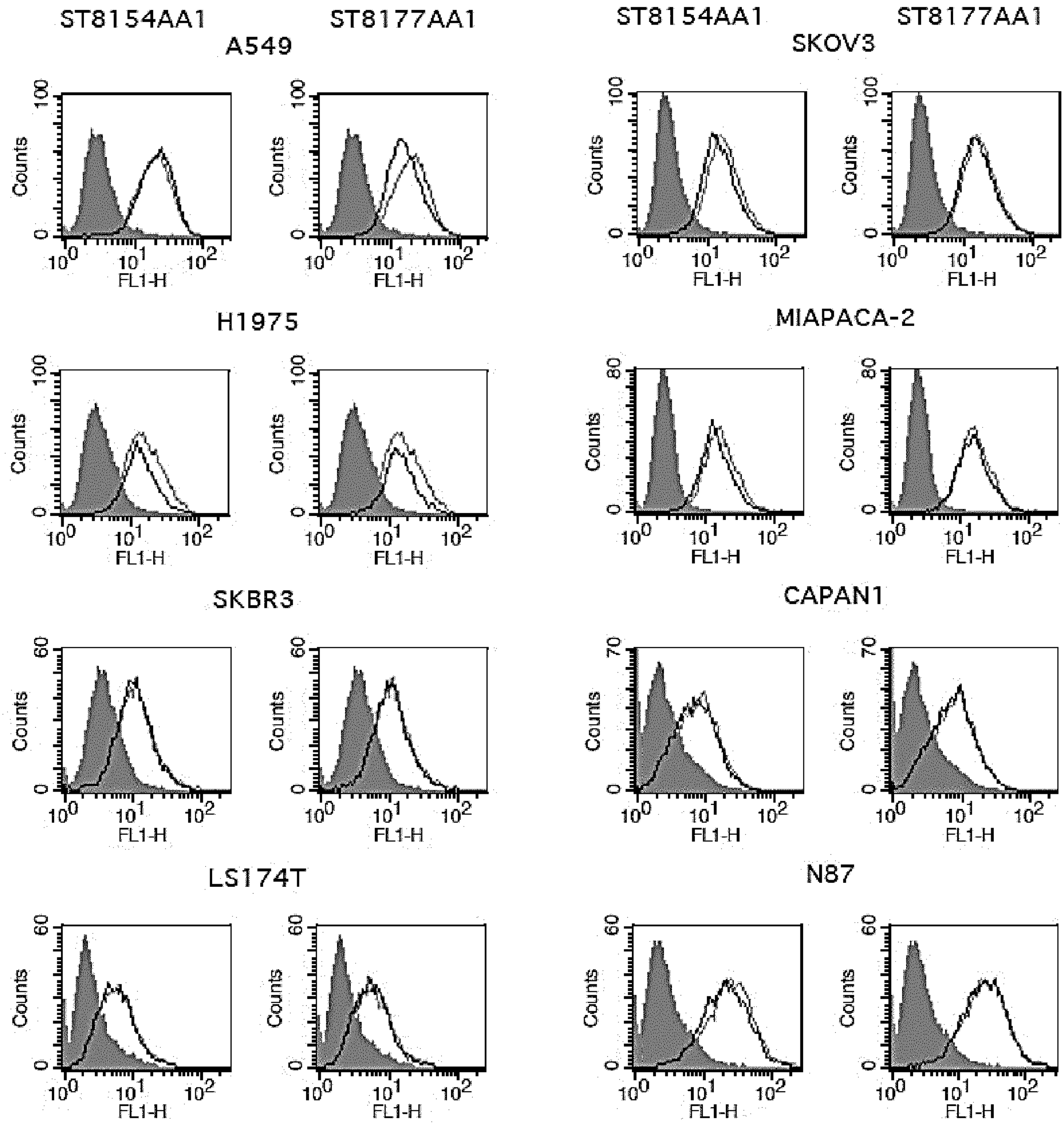
ST8154AA1
ST8177AA1
A549
SKOV3
H1975
MIAPACA-2
SKBR3
CAPAN1
LS174T
N87
Counts
FL1-H

Figure 6

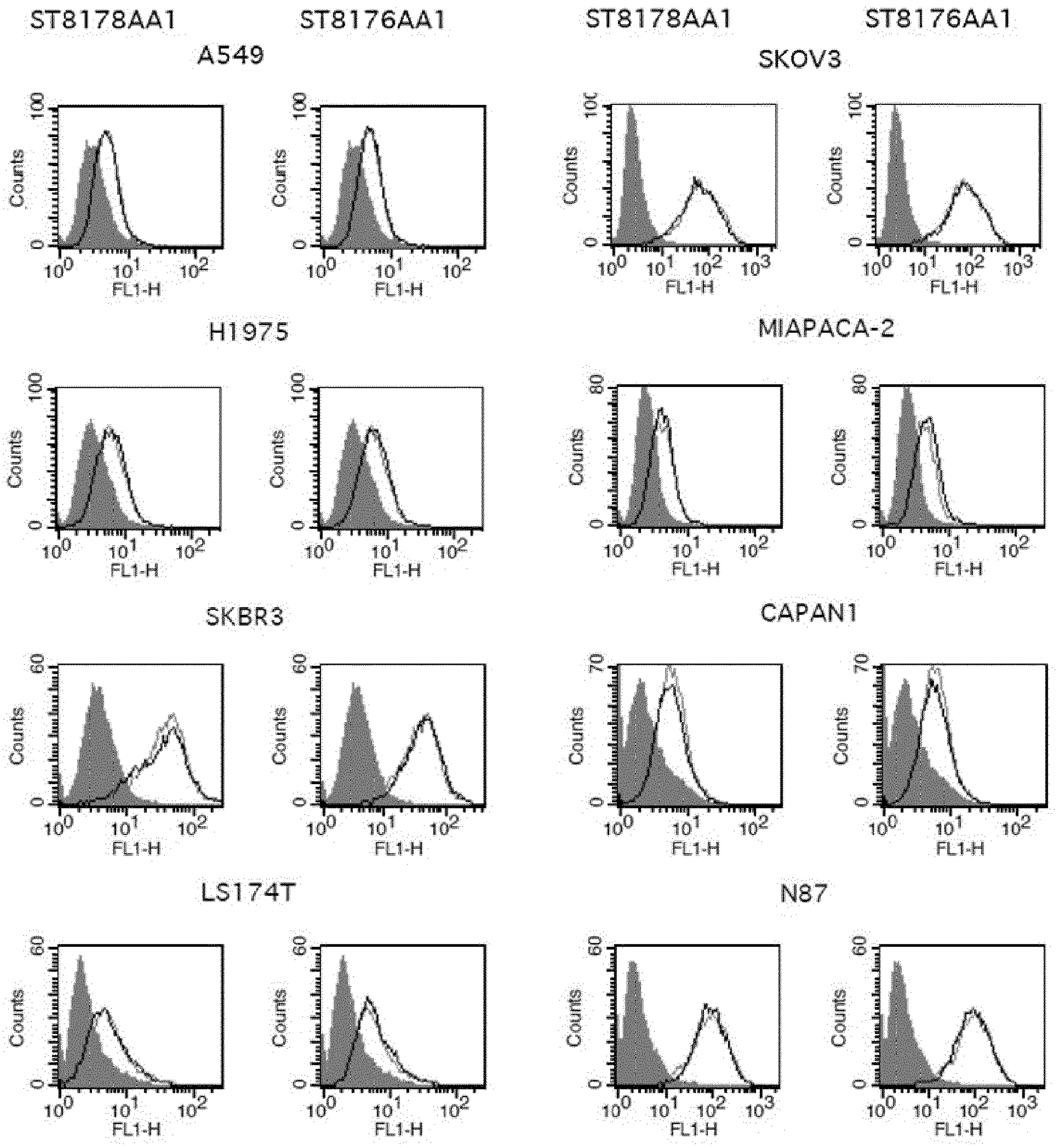
ST8178AA1
ST8176AA1
ST8178AA1
ST8176AA1
A549
SKOV3
H1975
MIAPACA-2
SKBR3
CAPAN1
LS174T
N87
Counts
FL1-H

Figure 7

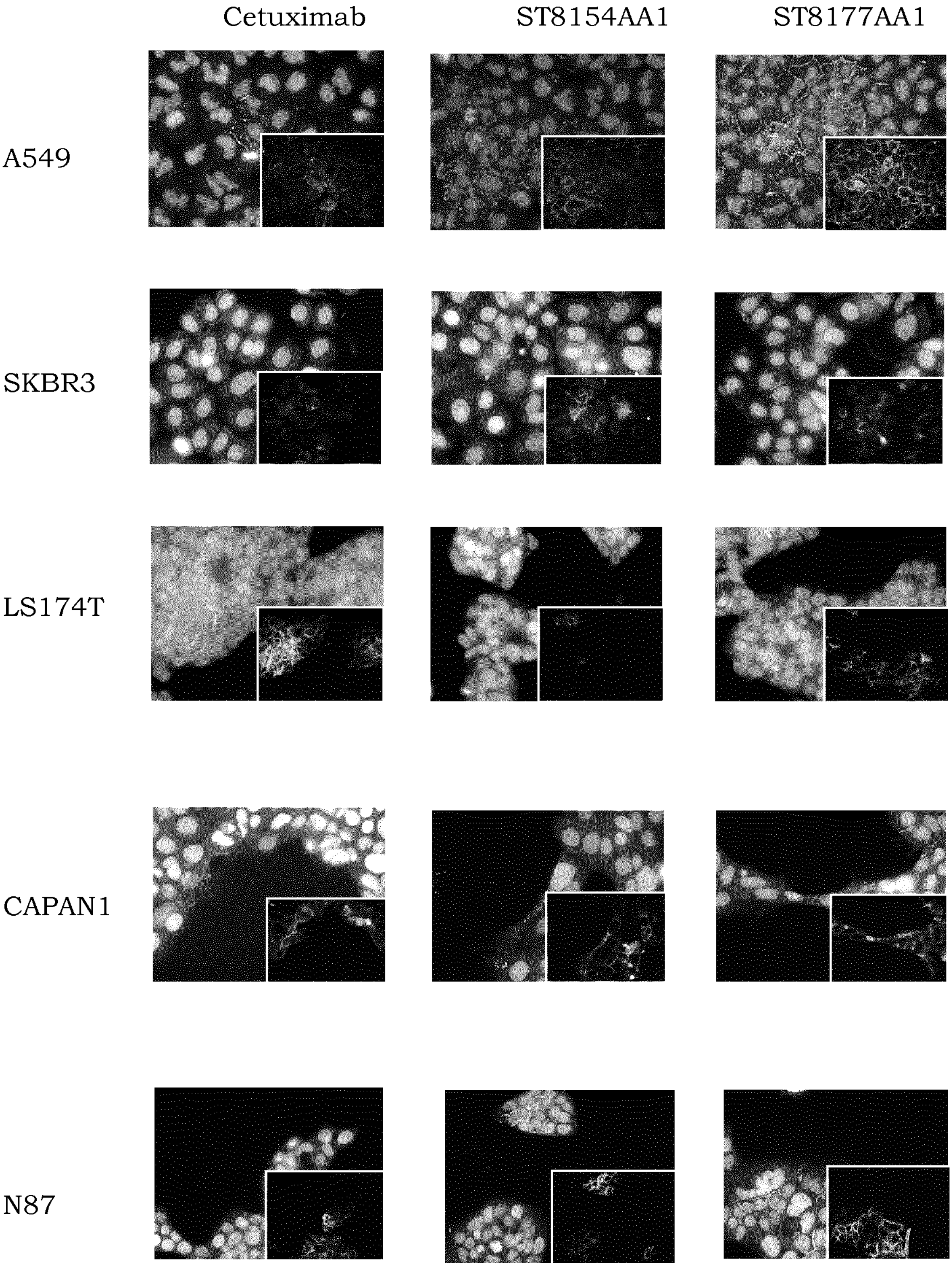
Cetuximab
ST8154AA1
ST8177AA1
A549
SKBR3
LS174T
CAPAN1
N87

Figure 8

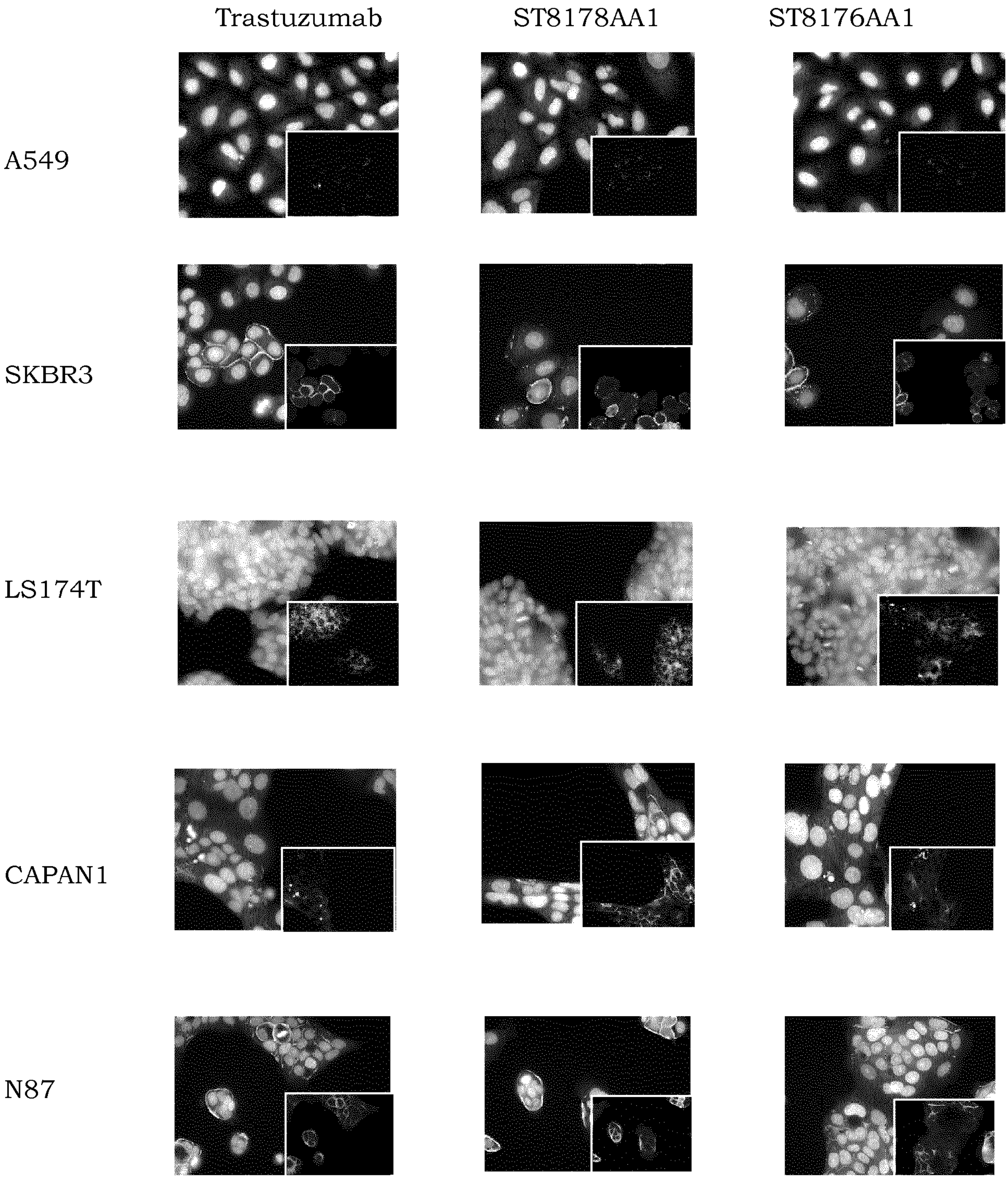
Trastuzumab
ST8178AA1
ST8176AA1
A549
SKBR3
LS174T
CAPAN1
N87

**Figure 9**

A)

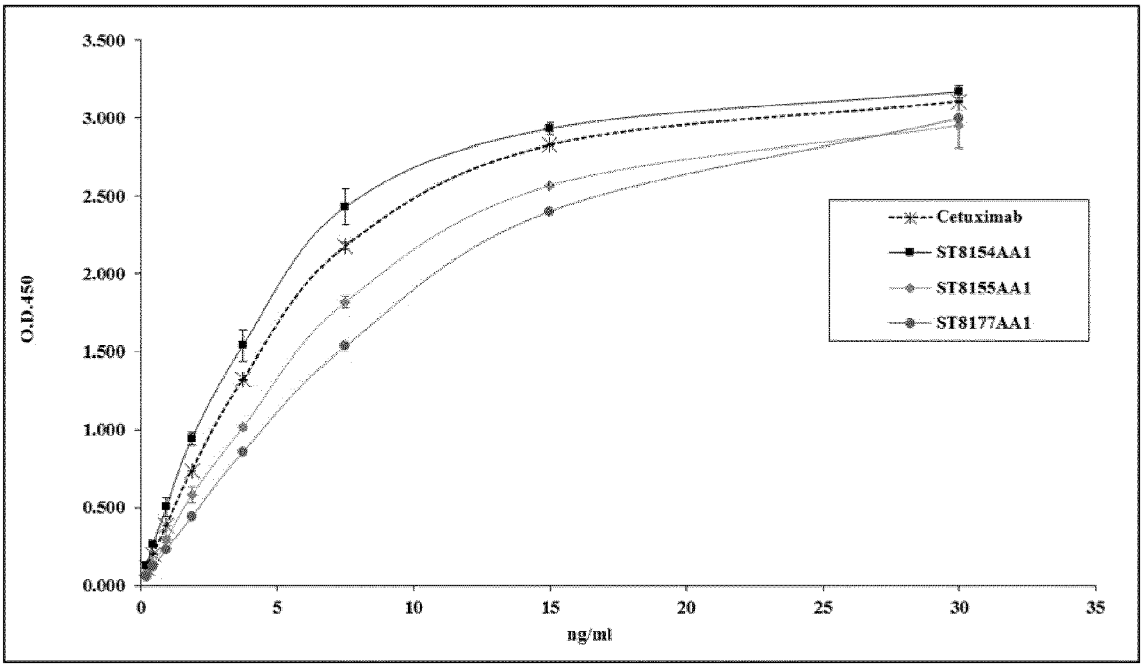
3.500
3.000
2.500
2.000
1.500
1.000
0.500
0.000
O.D.450
0
5
10
15
20
25
30
35
ng/ml
Cetuximab
ST8154AA1
ST8155AA1
ST8177AA1

B)

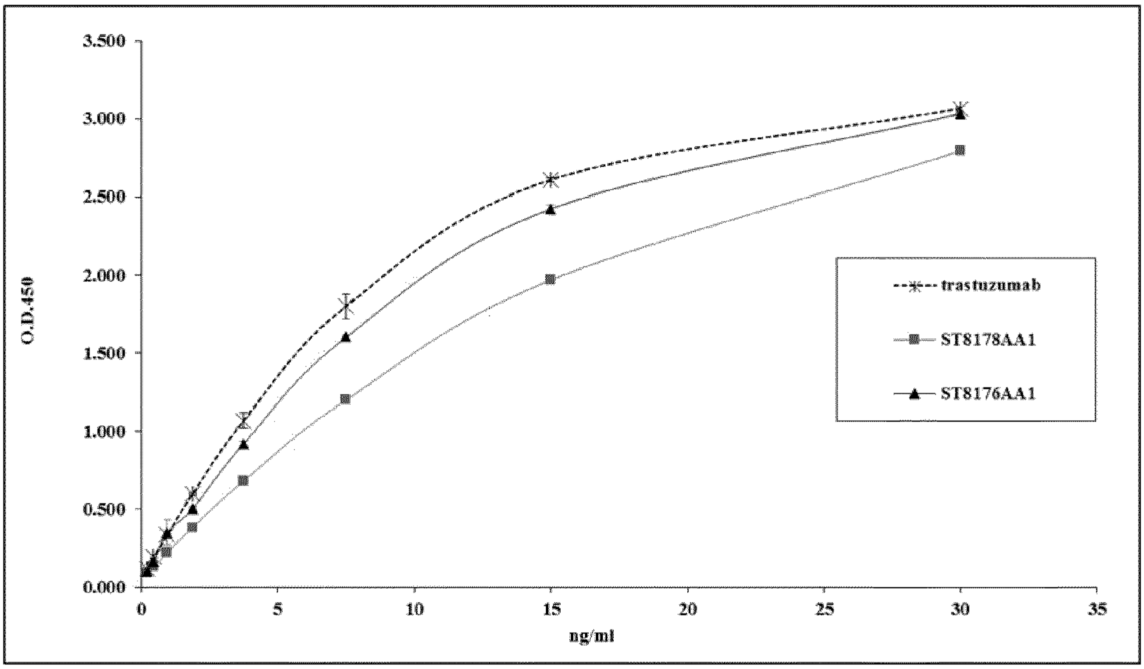
3.500
3.000
2.500
2.000
1.500
1.000
0.500
0.000
O.D.450
0
5
10
15
20
25
30
35
ng/ml
trastuzumab
ST8178AA1
ST8176AA1

**Figure 10**

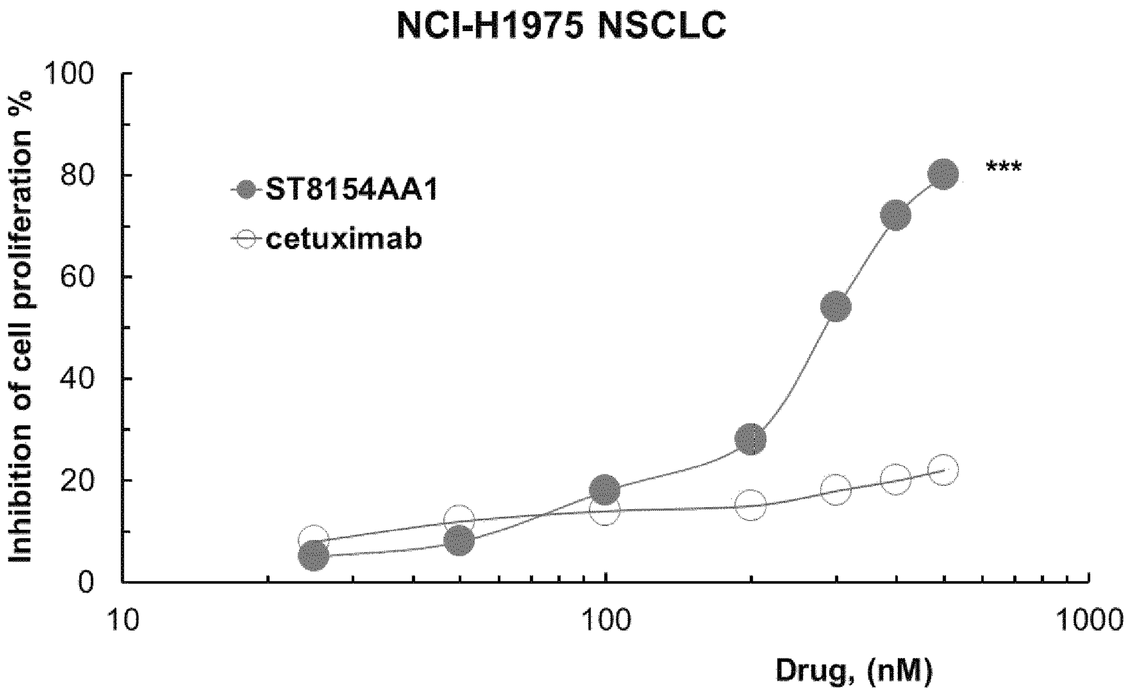
NCI-H1975 NSCLC
Inhibition of cell proliferation %
100
80
60
40
20
0
10
100
1000
Drug, (nM)
ST8154AA1
cetuximab
***

Figure 11

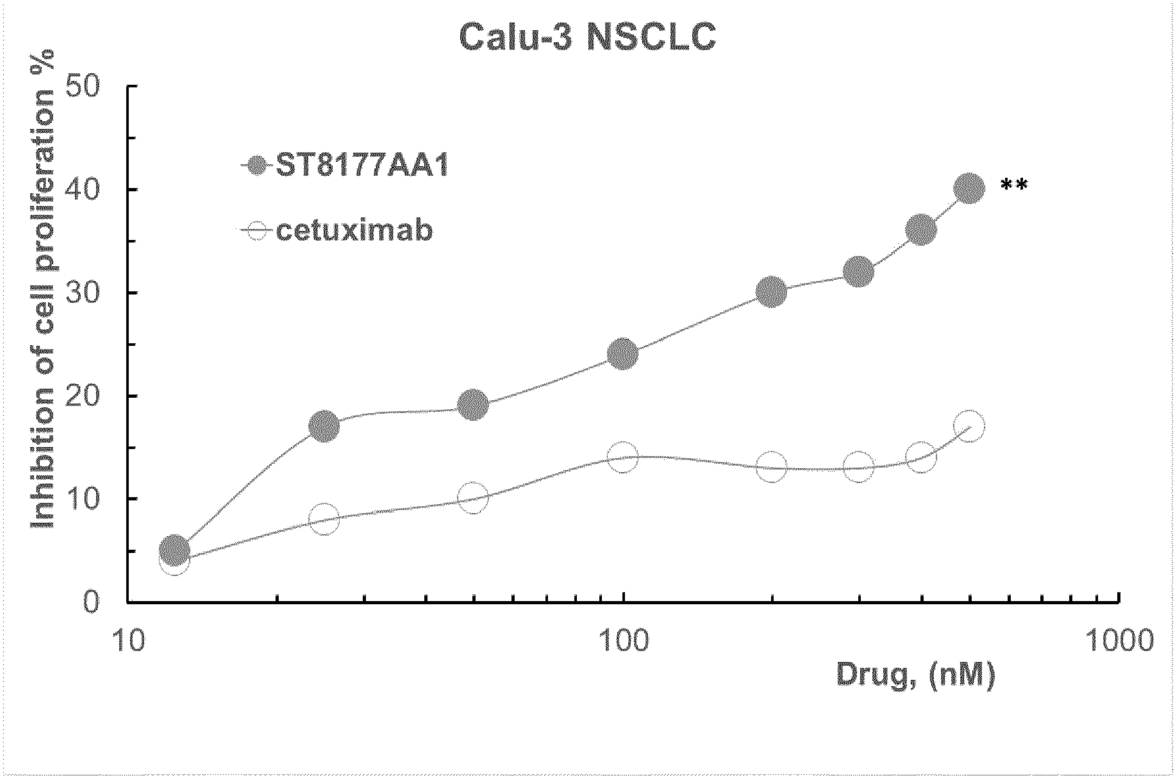
Calu-3 NSCLC
Inhibition of cell proliferation %
50
40
30
20
10
0
ST8177AA1
cetuximab
**
10
100
1000
Drug, (nM)

Figure 12

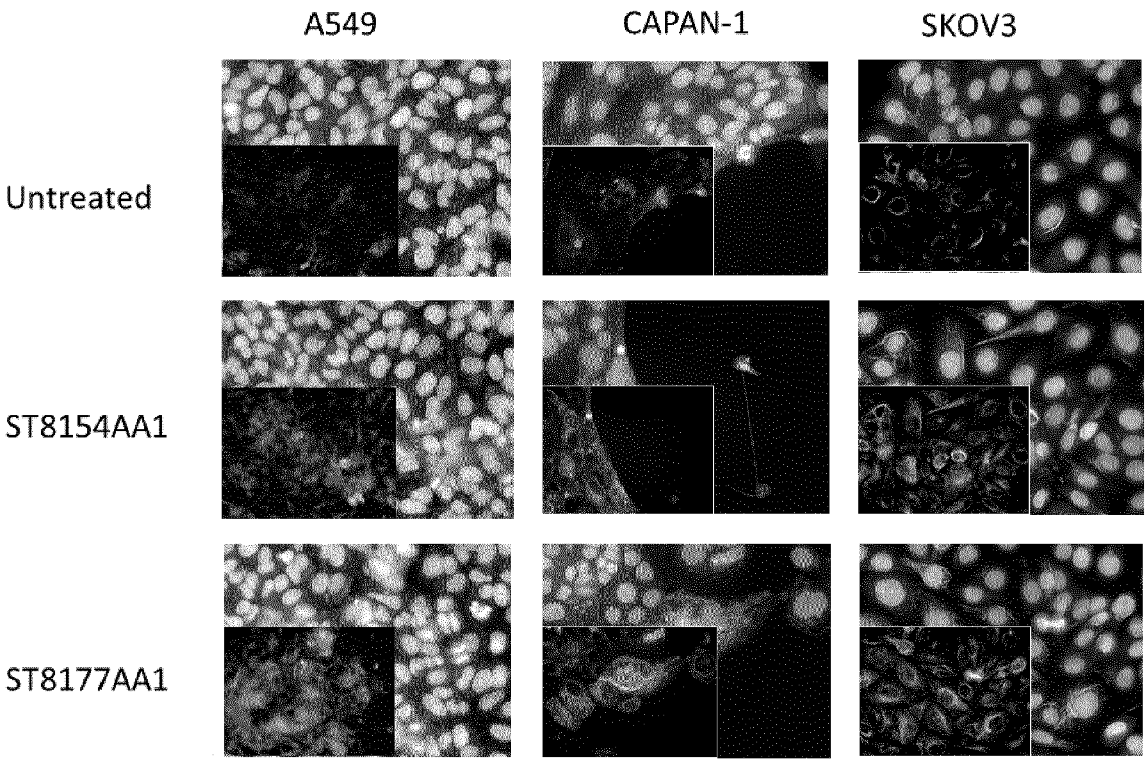


**Figure 13**

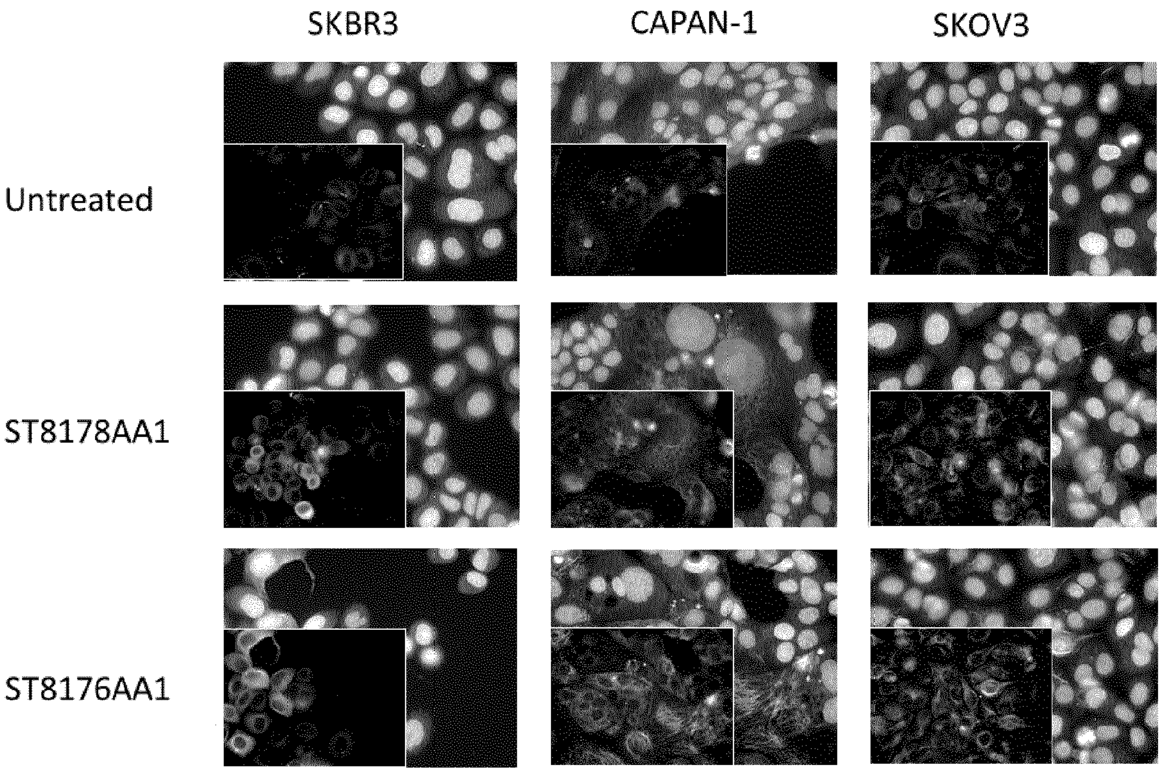


Figure 14

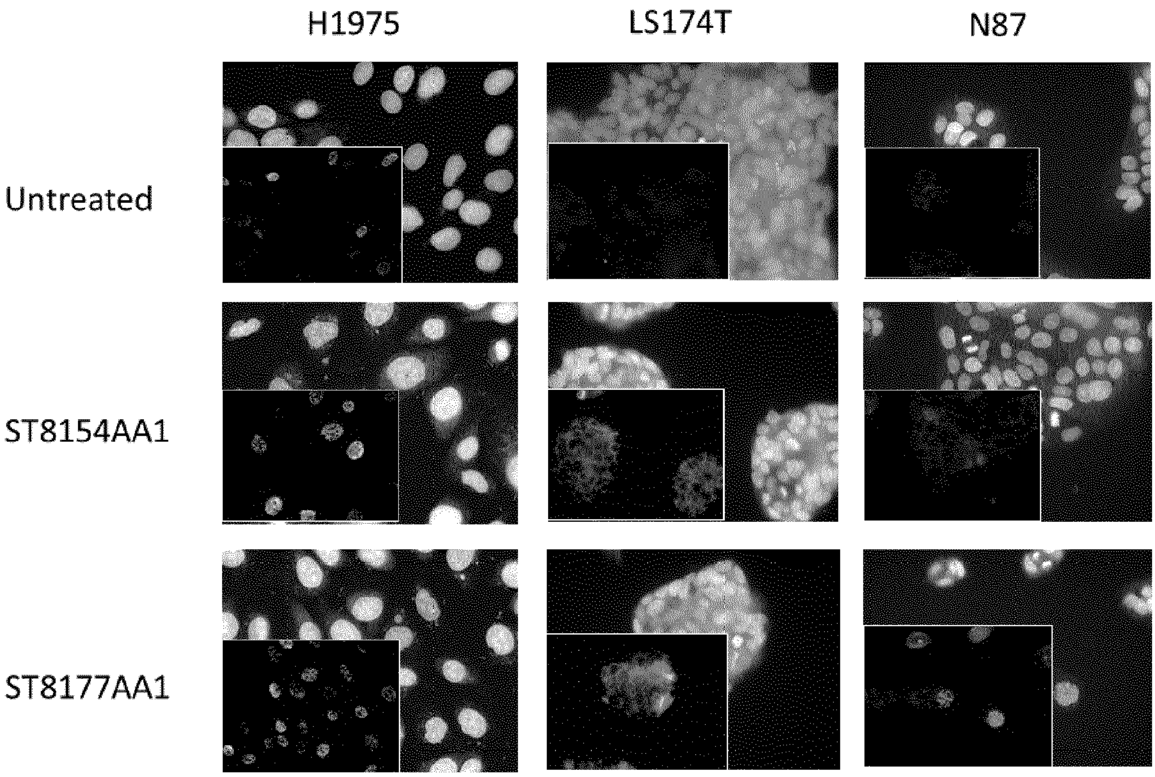


Figure 15

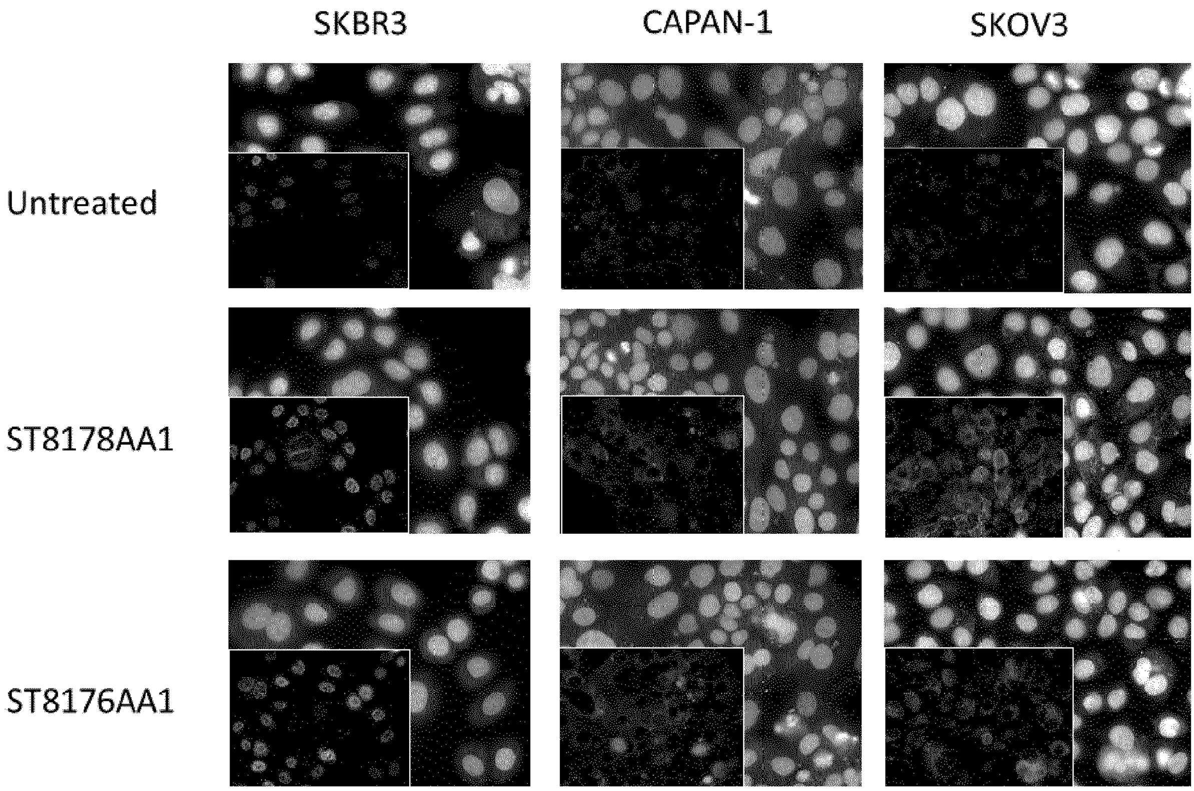


Figure 16

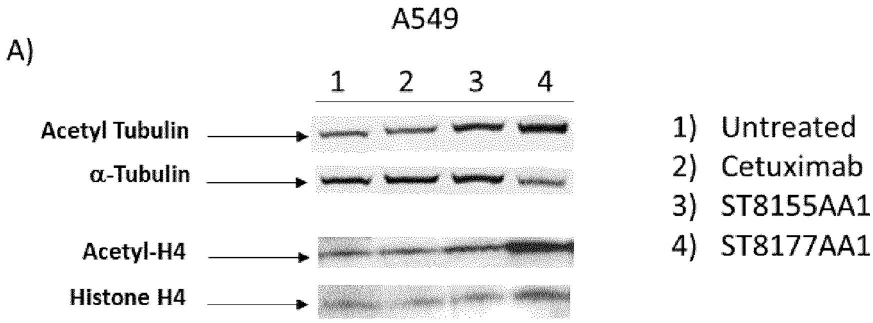
A549
A)
1 2 3 4
Acetyl Tubulin
α-Tubulin
Acetyl-H4
Histone H4
1) Untreated
2) Cetuximab
3) ST8155AA1
4) ST8177AA1

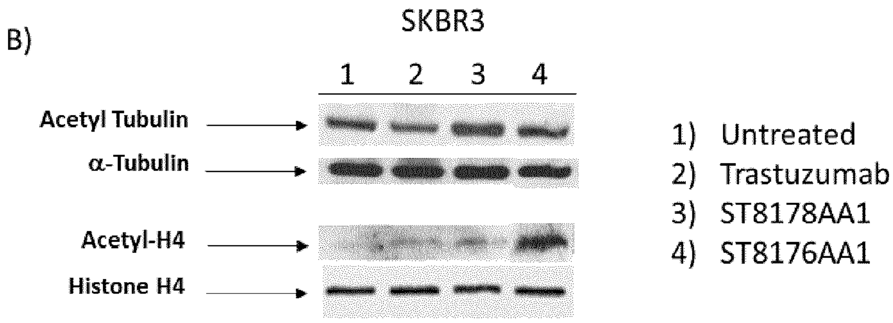
SKBR3
B)
1 2 3 4
Acetyl Tubulin
α-Tubulin
Acetyl-H4
Histone H4
1) Untreated
2) Trastuzumab
3) ST8178AA1
4) ST8176AA1

**Figure 17**

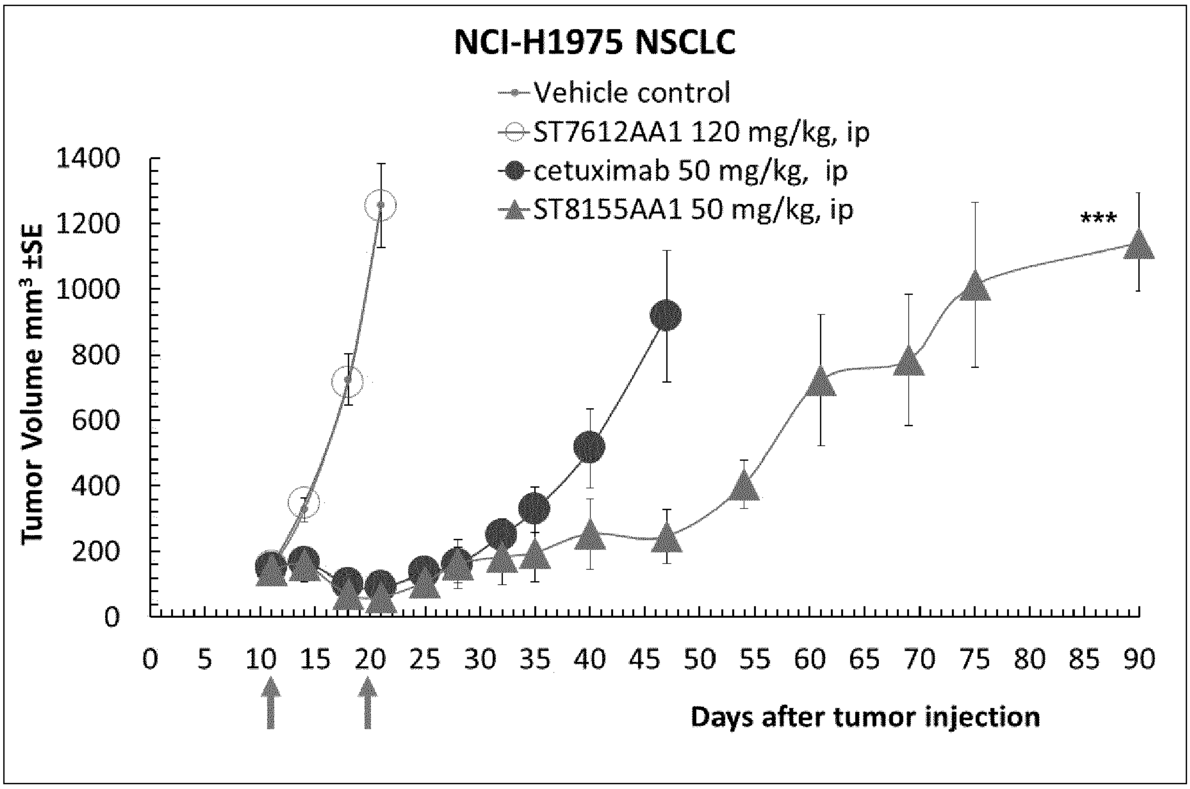
NCI-H1975 NSCLC
Vehicle control
ST7612AA1 120 mg/kg, ip
cetuximab 50 mg/kg, ip
ST8155AA1 50 mg/kg, ip
***
Tumor Volume mm³ ±SE
1400
1200
1000
800
600
400
200
0
0 5 10 15 20 25 30 35 40 45 50 55 60 65 70 75 80 85 90
Days after tumor injection

Figure 18

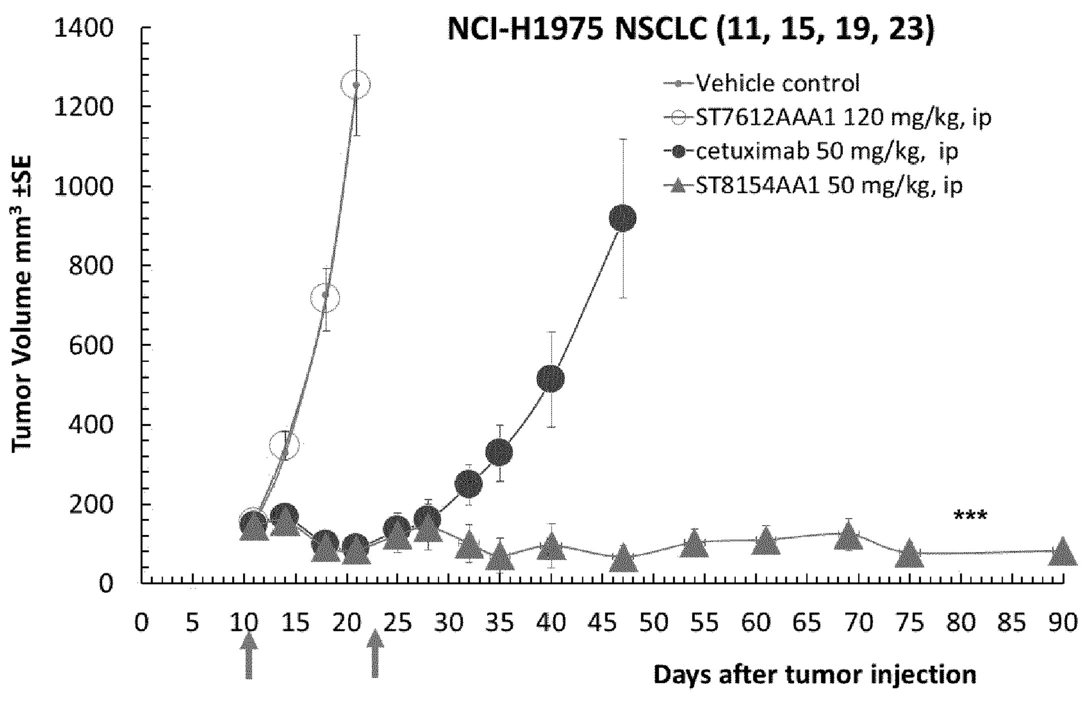
NCI-H1975 NSCLC (11, 15, 19, 23)
Vehicle control
ST7612AAA1 120 mg/kg, ip
cetuximab 50 mg/kg, ip
ST8154AA1 50 mg/kg, ip
Tumor Volume mm³ ±SE
1400
1200
1000
800
600
400
200
0
***
0 5 10 15 20 25 30 35 40 45 50 55 60 65 70 75 80 85 90
Days after tumor injection

Figure 19

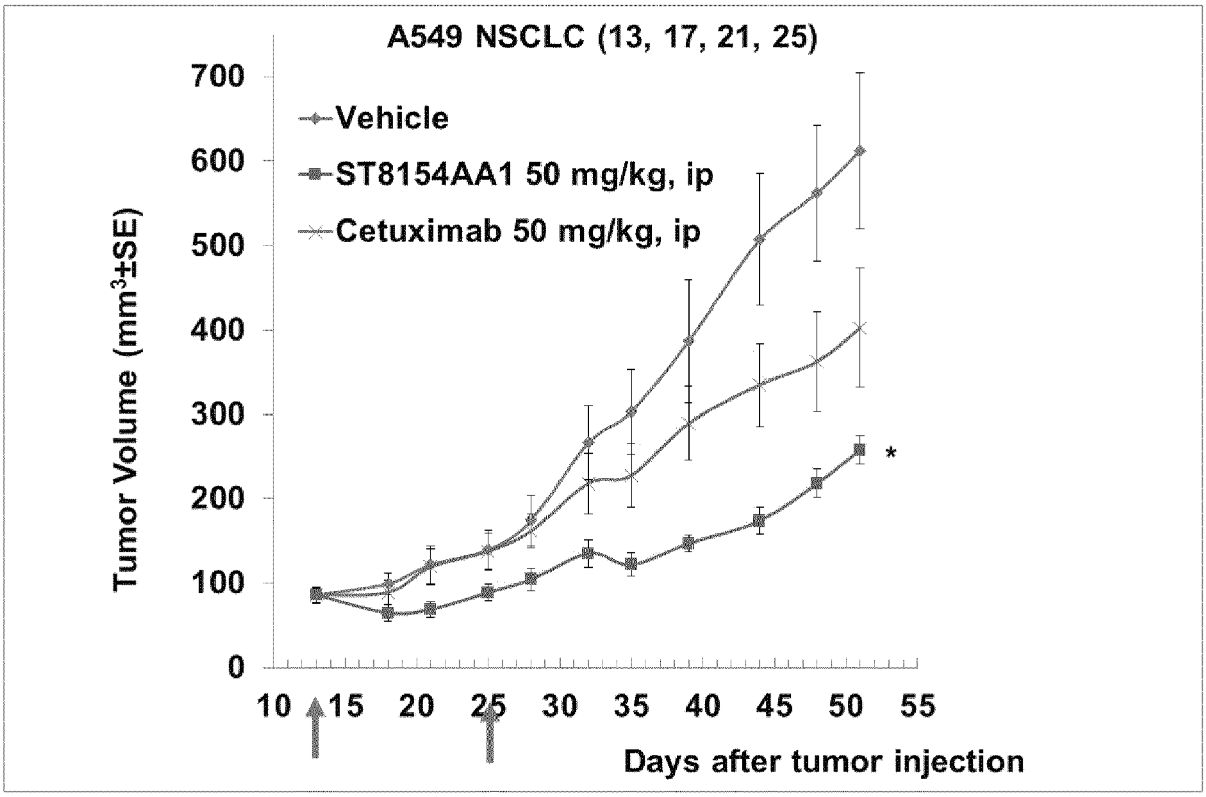
A549 NSCLC (13, 17, 21, 25)
Vehicle
ST8154AA1 50 mg/kg, ip
Cetuximab 50 mg/kg, ip
Tumor Volume (mm3±SE)
700
600
500
400
300
200
100
0
10
15
20
25
30
35
40
45
50
55
Days after tumor injection
*

Figure 20

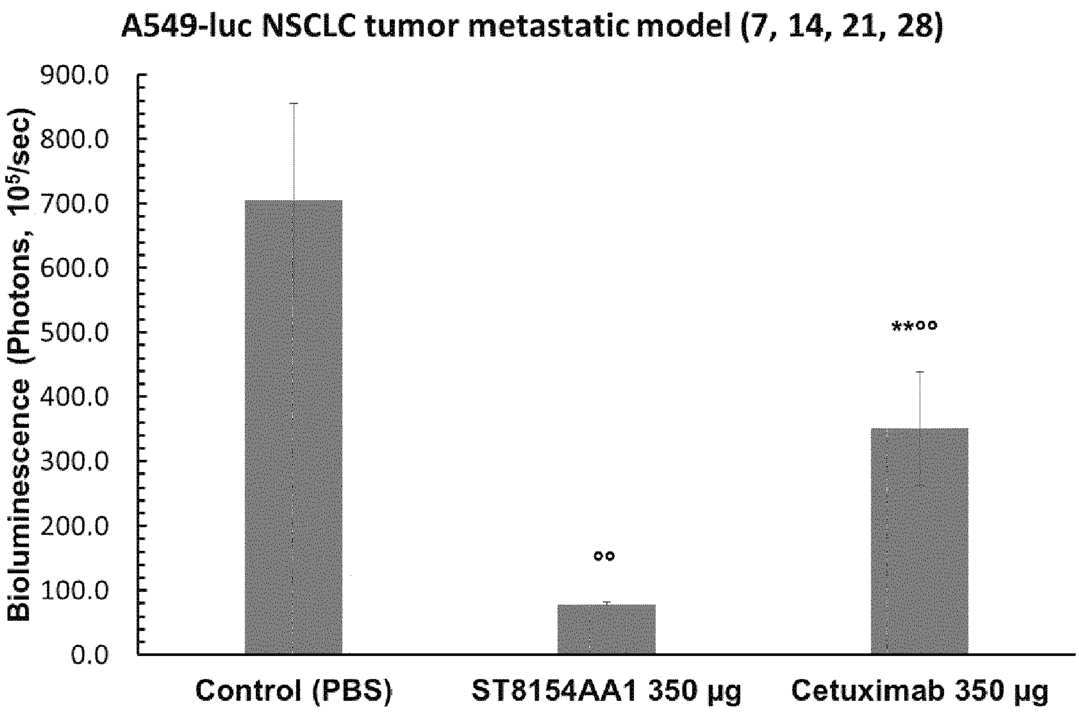
A549-luc NSCLC tumor metastatic model (7, 14, 21, 28)
Bioluminescence (Photons, 10^5/sec)
900.0
800.0
700.0
600.0
500.0
400.0
300.0
200.0
100.0
0.0
°°
**°°
Control (PBS)
ST8154AA1 350 µg
Cetuximab 350 µg

Figure 21

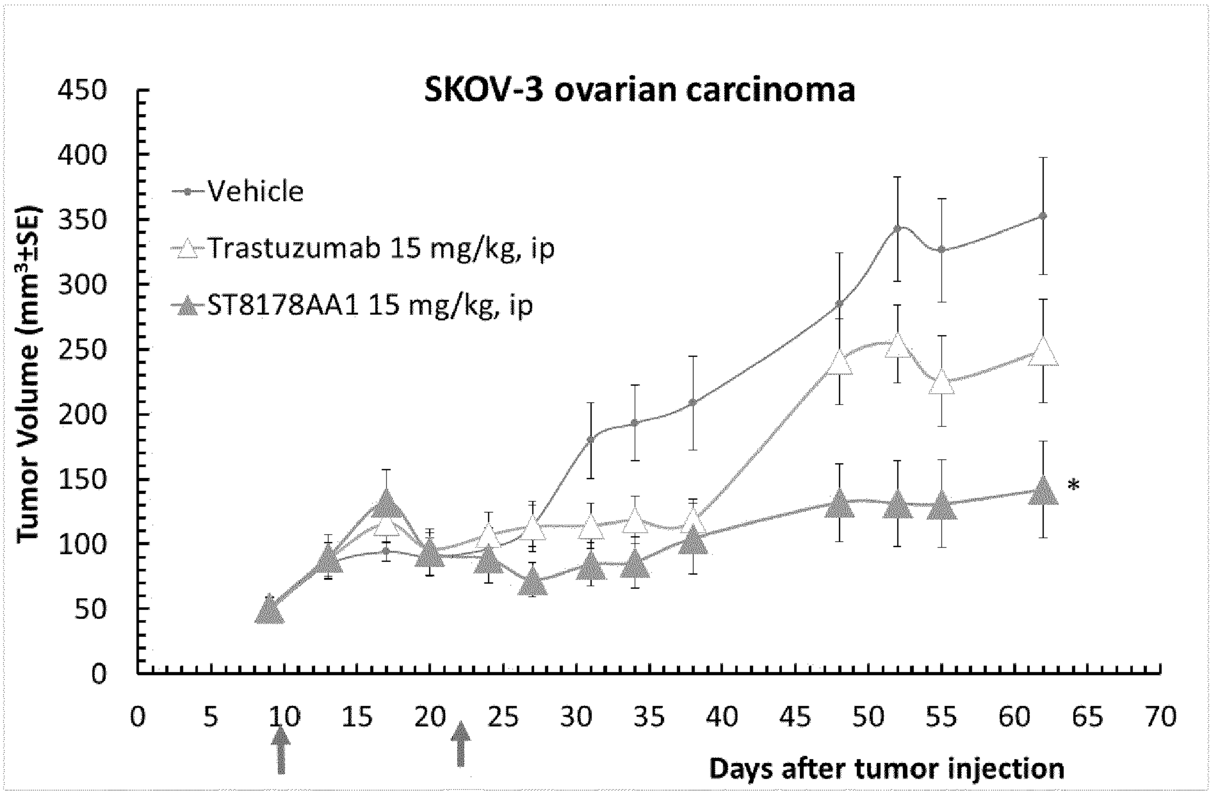
SKOV-3 ovarian carcinoma
Tumor Volume (mm³±SE)
450
400
350
300
250
200
150
100
50
0
Vehicle
Trastuzumab 15 mg/kg, ip
ST8178AA1 15 mg/kg, ip
*
0
5
10
15
20
25
30
35
40
45
50
55
60
65
70
Days after tumor injection

Figure 22

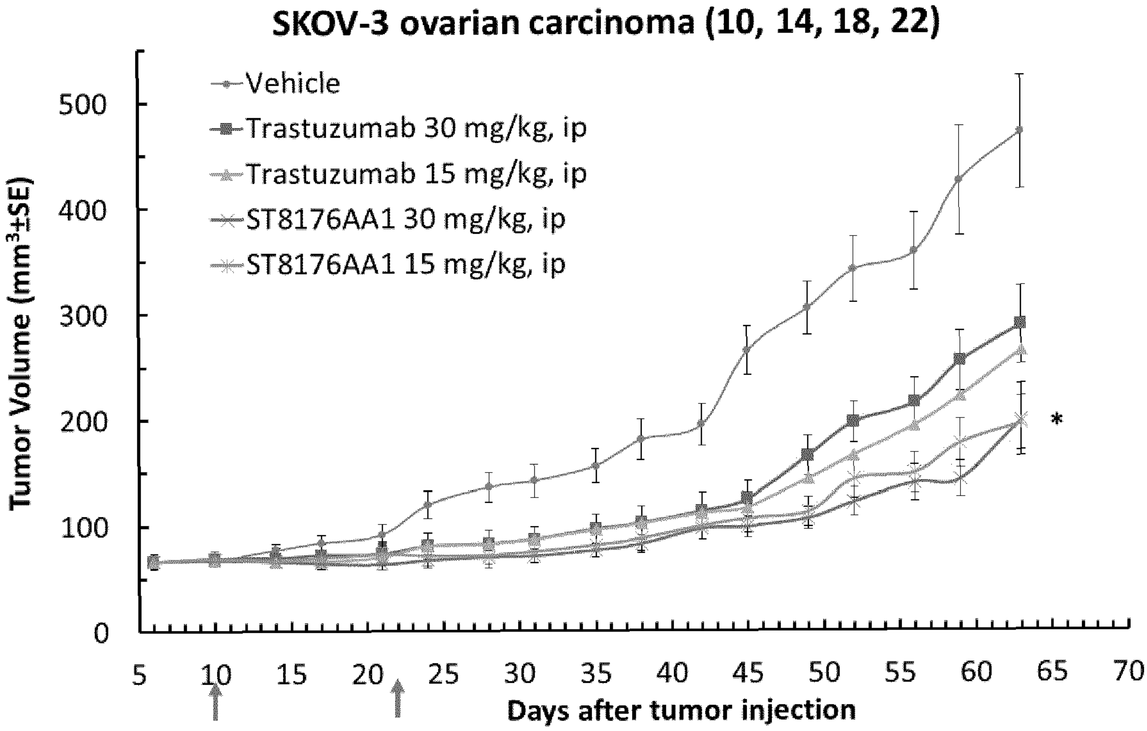
SKOV-3 ovarian carcinoma (10, 14, 18, 22)
Vehicle
Trastuzumab 30 mg/kg, ip
Trastuzumab 15 mg/kg, ip
ST8176AA1 30 mg/kg, ip
ST8176AA1 15 mg/kg, ip
Tumor Volume (mm$^3$±SE)
Days after tumor injection
0
100
200
300
400
500
5
10
15
20
25
30
35
40
45
50
55
60
65
70
*

Figure 23

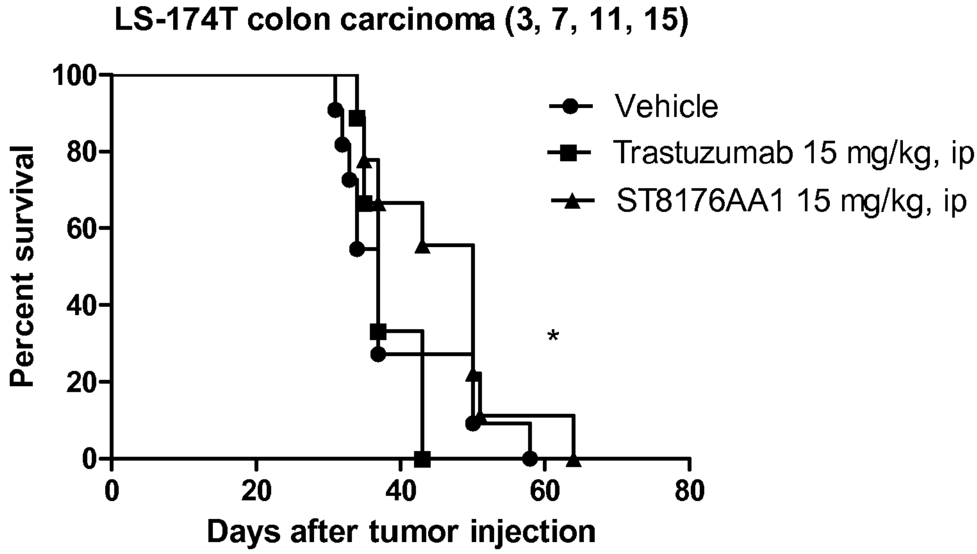
LS-174T colon carcinoma (3, 7, 11, 15)
Percent survival
100
80
60
40
20
0
0
20
40
60
80
Days after tumor injection
Vehicle
Trastuzumab 15 mg/kg, ip
ST8176AA1 15 mg/kg, ip
*

Figure 24

Europäisches Patentamt
European Patent Office
Office européen des brevets

# EUROPEAN SEARCH REPORT

Application Number
EP 17 16 3065

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/157595 A1 (MEDIMMUNE LLC [US]) 15 October 2015 (2015-10-15) * claims 24, 25, 44-51 * ----- | 1,14,15, 17,19,20 | INV. A61K47/68 A61P35/00 A61P31/18 |
| X | CHOI S ET AL: "Single chain variable fragment CD7 antibody conjugated PLGA/HDAC inhibitor immuno-nanoparticles: developing human T cell-specific nano-technology for delivery of therapeutic drugs targeting latent HIV", JOURNAL OF CONTROLLED RELEASE, ELSEVIER, AMSTERDAM, NL, vol. 152, no. suppl. 1, 30 November 2011 (2011-11-30), pages e9-e10, XP002720609, ISSN: 0168-3659, DOI: 10.1016/J.JCONREL.2011.08.089 | 1,14,15, 18 | |
| Y | * the whole document * ----- | 1,2, 4-12, 14-20 | |
| A | Gianfranco Battistuzzi ET AL: "Current Bioactive Compounds", , 1 December 2016 (2016-12-01), XP055405704, DOI: 10.2174/15734072126661605041605 Retrieved from the Internet: URL:https://www.ncbi.nlm.nih.gov/pmc/articles/PMC5101637/pdf/CBC-12-282.pdf [retrieved on 2017-09-12] ----- -/-- | 3,13 | TECHNICAL FIELDS SEARCHED (IPC) A61K |

The present search report has been drawn up for all claims

| Place of search | Date of completion of the search | Examiner |
|---|---|---|
| The Hague | 19 September 2017 | Langer, Miren |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 17 16 3065

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LOREDANA VESCI ET AL: "Preclinical antitumor activity of ST7612AA1: a new oral thiol-based histone deacetylase (HDAC) inhibitor", ONCOTARGET, vol. 6, no. 8, 20 March 2015 (2015-03-20), pages 5735-5748, XP055405708, DOI: 10.18632/oncotarget.3240 ----- | 3,13 | |
| Y | T. AI ET AL: "Multi-Targeted Histone Deacetylase Inhibitors in Cancer Therapy", CURRENT MEDICINAL CHEMISTRY : THE NEW INTERNATIONAL JOURNAL FOR TIMELY IN-DEPTH REVIEWS IN MEDICINAL CHEMISTRY, vol. 19, no. 4, 1 February 2012 (2012-02-01), pages 475-487, XP055406772, NL ISSN: 0929-8673, DOI: 10.2174/092986712798918842 * abstract * ----- | 1,2, 4-12, 14-20 | |
| | | | TECHNICAL FIELDS SEARCHED (IPC) |
| Y | ALISON C. WEST ET AL: "The combination of histone deacetylase inhibitors with immune-stimulating antibodies has potent anti-cancer effects", ONCOIMMUNOLOGY, vol. 1, no. 3, 1 May 2012 (2012-05-01), pages 377-379, XP055406778, DOI: 10.4161/onci.18804 * abstract * ----- | 1,2, 4-12, 14-20 | |

The present search report has been drawn up for all claims

3

| Place of search | Date of completion of the search | Examiner |
|---|---|---|
| The Hague | 19 September 2017 | Langer, Miren |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 16 3065

This annex lists the patent family members relating to the patent documents cited in the above-mentioned European search report. The members are as contained in the European Patent Office EDP file on
The European Patent Office is in no way liable for these particulars which are merely given for the purpose of information.

19-09-2017

| Patent document cited in search report | Publication date | Patent family member(s) | Publication date |
|---|---|---|---|
| WO 2015157595 A1 | 15-10-2015 | AU 2015243380 A1 | 10-11-2016 |
| | | CA 2944784 A1 | 15-10-2015 |
| | | CN 106459205 A | 22-02-2017 |
| | | EP 3129406 A1 | 15-02-2017 |
| | | JP 2017512486 A | 25-05-2017 |
| | | WO 2015157595 A1 | 15-10-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BERDASCO.** *Hum Genet,* 2013, vol. 132, 359-83
- **CHOUDHARY.** *Science,* 2009, vol. 325, 834-40
- **MINUCCI.** *Nature Rev Cancer,* 2006, vol. 6, 38-51
- **GLOZAK.** *Oncogene,* 2007, vol. 26, 5420-32
- **ZHANG.** *Med Res Rev,* 2015, vol. 35, 63-84
- **DINARELLO.** *Mol Med,* 2010, vol. 17, 333-52
- **DUVIC.** *Blood,* 2007, vol. 109, 31-39
- **VANDERMOLEN.** *J Antibiot (Tokyo),* 2011, vol. 64, 525-531
- **WEST.** *J Clin Invest,* 2014, vol. 124, 30-39
- **GARNOCK-JONES KP.** *Drugs,* 2015, vol. 75, 695-704
- **DINARELLO.** *Cell,* 2010, vol. 140, 935-950
- **GRAY.** *Epigenomics,* 2011, vol. 3, 431-450
- **GIANNINI.** *J Med Chem,* 2014, vol. 57, 8358-77
- **VESCI.** *OncoTarget,* 2015, vol. 20, 5735-48
- **BADIA.** *Antiviral Res,* 2015, vol. 123, 62-9
- **LEAL M.** *Ann NY Acad Sci,* 2014, vol. 1321, 41-54
- **OKELEY.** *Hematol Oncol Clin North Am,* 2014, vol. 28, 13-25
- **BARON.** *J Oncol Pharm Pract,* 2015, vol. 21, 132-42
- **BORNSTEIN.** *AAPS Journal,* 2015, vol. 17, 525-34
- **CASI ; NERI.** *J Med Chem,* 2015, vol. 58, 8751-61
- **SAMANTA.** *Biochim Biophys Acta,* 2017, vol. 1863, 518-28
- **AKHTAR.** *Plos One,* 2013, vol. 8, e67813
- **MEI.** *Neuron,* 2014, vol. 83, 27-49
- **BURGESS AW.** *Growth Factors,* 2008, vol. 26, 263-74
- **MENDELSOHN J.** *Semin Oncol,* 2006, vol. 33, 369-85
- **FEINER.** *Exp Rev Proteomics, Sep,* 2016, vol. 13, 817-32
- **ENRIQUE AA.** *Front Biosci,* 2012, vol. 4, 12-22
- **LANDI L.** *Expert Opin Pharmacol Ther,* 2014, vol. 15, 2293-305
- **CHONG CR.** *Nat Med,* 2013, vol. 19, 1389-400
- **LI T.** *Target Oncol,* 2009, vol. 4, 107-19
- **GRYDER.** *Future Med Chem,* 2012, vol. 4, 505-24
- **CLAWSON.** *Ann Transl Med,* 2016, vol. 4, 287
- **FELICE.** *Aliment Pharmacol Ther,* 2015, vol. 41, 26-38
- **COLLIN.** *Front Pharmacol,* 2015, vol. 6, 283
- **MELLMAN.** Perspect Biol. Cold Spring Harb, 2013, vol. 5, a016949
- **MOSESSON.** *Nat Rev Cancer,* 2008, vol. 8, 835-50
- *Chem. Soc. Rev.,* 2016, vol. 45, 1691-1719
- *Bioorganic & Medicinal Chemistry Letters,* 2016, vol. 26, 1542-1545
- **OYAIS A.** *Zentralbl Chir. 2014,* 2014, vol. 141, 421-4
- **SOLASS W.** *Ann Surg Oncol,* 2014, vol. 21, 553-9
- *Blanco,* 2014, vol. 20, 2311-6
- **SOLASS.** *Ann Surg Oncol,* 2013, vol. 20, 3504-11
- **SOLASS et al.** *Ann Surg Oncol,* 2013, vol. 20, 3504-11
- **OYAIS A et al.** *Zentralbl Chir. 2014,* 2014, vol. 141, 421-4